(19) Europäisches Patentamt
European Patent Office
Office européen des brevets

(11) **EP 4 258 984 B1**

(12) **EUROPEAN PATENT SPECIFICATION**

(45) Date of publication and mention
of the grant of the patent:
**03.12.2025  Bulletin 2025/49**

(21) Application number: **21836234.1**

(22) Date of filing: **10.12.2021**

(51) International Patent Classification (IPC):
*A61B 5/0245* (2006.01)   *A61B 5/346* (2021.01)
*A61B 5/361* (2021.01)   *A61B 5/363* (2021.01)
*G16H 10/60* (2018.01)   *G16H 15/00* (2018.01)
*A61B 5/333* (2021.01)   *A61B 5/339* (2021.01)
*A61B 5/11* (2006.01)

(52) Cooperative Patent Classification (CPC):
**A61B 5/0245; A61B 5/1116; A61B 5/333;
A61B 5/339; A61B 5/346; A61B 5/361;
A61B 5/363; G16H 10/60; G16H 15/00;
G16H 40/67; G16H 50/20**

(86) International application number:
**PCT/IB2021/061589**

(87) International publication number:
**WO 2022/130152 (23.06.2022 Gazette 2022/25)**

(54) **CONTEXTUAL BIOMETRIC INFORMATION FOR USE IN CARDIAC HEALTH MONITORING**

KONTEXTUELLE BIOMETRISCHE INFORMATIONEN ZUR VERWENDUNG BEI DER
ÜBERWACHUNG DER HERZGESUNDHEIT

INFORMATIONS BIOMÉTRIQUES CONTEXTUELLES DESTINÉES À ÊTRE UTILISÉES DANS LA
SURVEILLANCE DE LA SANTÉ CARDIAQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **14.12.2020   US 202063199208 P
18.10.2021   US 202163262657 P**

(43) Date of publication of application:
**18.10.2023   Bulletin 2023/42**

(73) Proprietor: **ZOLL Medical Israel Ltd.
4464323 Kfar Saba (IL)**

(72) Inventors:
• **VOLOSIN, Kent J**
**4464323 Kfar-Saba (IL)**
• **WEINSTEIN, Uriel**
**4464323 Kfar-Saba (IL)**
• **FLETCHER ATIENZA, Melissa Marie**
**4464323 Kfar-Saba (IL)**
• **PERUMAL, Ramu**
**4464323 Kfar-Saba (IL)**
• **RAVID, Rafi**
**4464323 Kfar-Saba (IL)**

(74) Representative: **Potter Clarkson
Chapel Quarter
Mount Street
Nottingham NG1 6HQ (GB)**

(56) References cited:
WO-A1-2019/030746   US-A1- 2011 245 629
US-A1- 2017 300 653   US-A1- 2019 069 794

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

The present disclosure relates to monitoring cardiac and biometric signals of a patient and preparing cardiac health reports based on the monitored cardiac and biometric signals.

There are a wide variety of electronic and mechanical devices for monitoring and treating patients' medical conditions. In some examples, depending on the underlying medical condition being monitored or treated, medical devices such as cardiac monitors or defibrillators may be surgically implanted or externally connected to the patient. In some cases, physicians may use medical devices alone or in combination with drug therapies to treat conditions such as cardiac arrhythmias.

A patient with a known or suspected cardiac arrhythmia may be provided with a device that monitors the patient's cardiac activity. The device may transmit data on the patient's cardiac activity to a remote server, where the data is analyzed by a technician. The technician prepares a report on the patient's cardiac activity from the received data and transmits the report to the patient's physician for review. Based on the report of the patient's cardiac activity, the physician may make one or more recommendations for the patient's cardiac health.

US 2017/300653 A1 discloses a device for graphically reconstructing information received from a medical device. US 2011/245629 A1 discloses a device that displays the temporal correlation between a bioelectrical brain signal of a patient and patient motion data, such as a signal indicative of patient motion or a patient posture indicator. WO 2019/030746 A1 discloses a physiological patient monitoring system which includes a physiological monitoring device for physiological monitoring of patients with a continuous or near-continuous transmission and analysis of monitored physiological data during the monitoring process. US 2019/069794 A1 discloses a system and method for facilitating a cardiac rhythm disorder diagnosis based on subcutaneous cardiac monitoring data with the aid of a digital computer.

SUMMARY

The invention is as set out in the appended claims.

BRIEF DESCRIPTION OF THE DRAWINGS

Various aspects of at least one example are discussed below with reference to the accompanying figures, which are not intended to be drawn to scale. The figures are included to provide an illustration and a further understanding of the various aspects and examples, and are incorporated in and constitute a part of this specification, but are not intended to limit the scope of the disclosure. The drawings, together with the remainder of the specification, serve to explain principles and operations of the described and claimed aspects and examples. In the figures, each identical or nearly identical component that is illustrated in various figures is represented by a like numeral. For purposes of clarity, not every component may be labeled in every figure.

FIG. 1 depicts a cardiac monitoring system for remotely monitoring the cardiac status of patients with known or suspected heart conditions, in accordance with an example of the present disclosure.

FIG. 2A depicts a sample process flow for displaying contextual biometric information for arrhythmia events occurring in a patient, in accordance with an example of the present disclosure.

FIG. 2B depicts a sample process flow for determining the onset of an arrhythmia event, in accordance with an example of the present disclosure.

FIG. 2C depicts a sample process flow for determining an arrhythmia contextual time period for an arrhythmia event, in accordance with an example of the present disclosure.

FIG. 2D depicts a sample process flow for determining the contextual biometric information for an arrhythmia contextual time period, in accordance with an example of the present disclosure.

FIG. 2E depicts a sample process flow for motion classification, in accordance with an example of the present disclosure.

FIG. 2F depicts a sample process flow for preparing the display of an arrhythmia report, in accordance with an example of the present disclosure.

FIG. 2G depicts a sample process flow for determining the arrhythmia or symptom contextual time period, in accordance with an example of the present disclosure.

FIG. 3 depicts another sample process flow for displaying contextual biometric information for arrhythmia events occurring in a patient, in accordance with an example of the present disclosure.

FIG. 4A depicts an arrhythmia event report, in accordance with an example of the present disclosure.

FIG. 4B depicts a continuation of the arrhythmia event report, in accordance with an example of the present disclosure.

FIG. 4C depicts a daily report, in accordance with an example of the present disclosure.

FIG. 4D depicts a continuation of the daily report, in accordance with an example of the present disclosure.

FIG. 5 depicts a sample process flow for displaying contextual biometric information for patient-provided symptom inputs, in accordance with an example of the present disclosure.

FIG. 6A depicts a screen that may be displayed on a visual display of a portable gateway of a cardiac monitoring system, in accordance with an example of the present disclosure.

FIG. 6B depicts a screen that may be displayed on a visual display of a portable gateway of a cardiac monitoring system, in accordance with an example of the present disclosure.

FIG. 6C depicts a screen that may be displayed on a visual display of a portable gateway of a cardiac monitoring system, in accordance with an example of the present disclosure.

FIG. 7 depicts another sample process flow for displaying contextual biometric information for patient-provided symptom inputs, in accordance with an example of the present disclosure.

FIG. 8A depicts a symptom report, in accordance with an example of the present disclosure.

FIG. 8B depicts a continuation of the symptom report, in accordance with an example of the present disclosure.

FIG. 8C depicts a symptom report, in accordance with an example of the present disclosure.

FIG. 8D depicts a continuation of the symptom report, in accordance with an example of the present disclosure.

FIG. 9A depicts a sample process flow for displaying graphical summary views of arrhythmia events occurring in a patient and/or patient-provided symptom inputs over predetermined use periods, in accordance with an example of the present disclosure.

FIG. 9B depicts a sample process flow for determining a biometric context summary for a predetermined use period, in accordance with an example of the present disclosure.

FIG. 9C depicts a sample process flow for presenting a summary view of arrhythmia events and/or symptom inputs for a predetermined use period, in accordance with an example of the present disclosure.

FIG. 10 depicts another sample process flow for displaying graphical summary views of arrhythmia events occurring in a patient and/or patient-provided symptom inputs over predetermined use periods, in accordance with an example of the present disclosure.

FIG. 11A depicts a use-period report, in accordance with an example of the present disclosure.

FIG. 11B depicts a continuation of the use-period report, in accordance with an example of the present disclosure.

FIG. 11C depicts a use-period report, in accordance with an example of the present disclosure.

FIG. 11D depicts a continuation of the use-period report, in accordance with an example of the present disclosure.

FIG. 11E depicts a use-period report, in accordance with an example of the present disclosure.

FIG. 11F depicts a use-period report, in accordance with an example of the present disclosure.

FIG. 12A depicts an adhesive patch, in accordance with an example of the present disclosure.

FIG. 12B depicts a sensor unit, in accordance with an example of the present disclosure.

FIG. 12C depicts attaching a sensor unit to an adhesive patch, in accordance with an example of the present disclosure.

FIG. 12D depicts attaching a cardiac sensor to a patient's body, in accordance with an example of the present disclosure.

FIG. 12E depicts attaching a cardiac sensor to a patient's body, in accordance with an example of the present disclosure.

FIG. 13A depicts a back view of a sensor unit, in accordance with an example of the present disclosure.

FIG. 13B depicts a front view of a sensor unit, in accordance with an example of the present disclosure.

FIG. 13C depicts an exploded view of a sensor unit, in accordance with an example of the present disclosure.

FIG. 14 depicts device electronic architecture for a sensor unit, in accordance with an example of the present disclosure.

FIG. 15 depicts another cardiac sensor adhered to a patient, in accordance with an example of the present disclosure.

FIG. 16A depicts a charger for a cardiac monitoring device, in accordance with an example of the present disclosure.

FIG. 16B depicts a charger being used with a sensor unit, in accordance with an example of the present disclosure.

FIG. 16C depicts a charger being used with a sensor unit and a portable gateway, in accordance with an example of the present disclosure.

FIG. 16D depicts a charger being used with a sensor unit and a portable gateway, in accordance with an example of the present disclosure.

FIG. 17A depicts plots for accelerometer data, in accordance with examples of the present disclosure.

FIG. 17B depicts plots for accelerometer data, in accordance with examples of the present disclosure.

FIG. 18A depicts an event report, in accordance with examples of the present disclosure.

FIG. 18B depicts a continuation of the event report, in accordance with examples of the present disclosure.

FIG. 19A depicts a use-period report, in accordance with examples of the present disclosure.

FIG. 19B depicts a continuation of the use-period report, in accordance with examples of the present disclosure.

DETAILED DESCRIPTION

In today's busy cardiology practice, the assessment and management of patients involves an integrated approach of reviewing several parameters such as clinical observations/assessment, ECG changes, thoracic fluid changes, weight loss/gain, urine output, radiographic signs, among others. Medical professionals assess their patients' clinical status by monitoring multiple vital signs such as ECG-based heart rate, respiration rate, temperature, blood pressure, and oxygen saturation. This approach is superior to one where only individual vital signs (such as only heart rate changes) are obtained and assessed. For example, normal respiration rate ranges from 10 to 20 breaths per minute and, in accordance with current standards of care, changes are part of the patient clinical assessment. Consequently, the disclosed improved cardiac monitoring systems, devices and techniques for health care professionals to remotely monitor a plurality of these parameters will play an important role.

This disclosure relates to an improved cardiac monitoring system for remotely monitoring the cardiac health of patients with known or suspected heart conditions, such as arrhythmias. Such patients may be prescribed a cardiac sensor configured to be worn continuously for an extended period of time and transmit (e.g., via internal communications circuitry or a separate portable gateway), information about the patient's suspected cardiac symptoms, cardiac and physical activity to a remote server. For example, such information can be ECG signals and motion signals (including both motion and posture information) associated with the patient. At the remote server, this information can be processed to determine arrhythmias and biometric information. The biometric information provides context for the determined arrhythmia, and can include activity and posture information such as whether the patient is standing, sitting, walking, climbing stairs, running, or lying down; respiration information; pulse oximetry information; blood pressure information; body temperature information; and sleep data. In some examples, the cardiac sensor and/or portable gateway may itself process such ECG and motion signals and determine the arrhythmias and the biometric information, and then transmit such determined arrhythmias and biometric information to the remote server.

In some implementations, the biometric information may also include environmental conditions of the patient, cardiac sensor, and/or portable gateway. For example, the biometric information may include the temperature outside the cardiac sensor and/or portable gateway, the humidity level, pressure, elevation, windchill, air quality, auditory noise level, and so on. These environmental conditions may be measured by the cardiac sensor and/or portable gateway and transmitted to the remote server, and/or these environmental conditions may be determined by the remote server. For example, the remote server may receive location data from the cardiac sensor and/or portable gateway determined by a global positioning system (GPS) sensor of the cardiac sensor and/or portable gateway. Using the location data of the cardiac sensor, the remoter server may look up the environmental conditions for the patient.

In this disclosure, techniques and systems are described for presenting, to caregivers and technicians, information about the arrhythmias and biometric information such that the arrhythmias are presented within the context of the biometric information. The improved cardiac monitoring system as described herein provides several advantages over prior art systems. For example, the additional contextual biometric information enhances the diagnostic yield or diagnostic quality of the reporting infrastructure within the cardiac care continuum. In this regard, the improved cardiac monitoring system enhances reporting of data from prescribed cardiac sensors to provide medically relevant information needed to establish a diagnosis for the patient without a need for additional diagnostic tests, clinical visits, or extending a time period of cardiac monitoring. Example use scenarios are provided below that further demonstrate the value afforded by the improved cardiac monitoring systems described herein. Accordingly, in implementations herein, the cardiac monitoring system comprises a cardiac sensor that includes ECG and biometric data monitoring. The improved system overcomes limitations in existing technologies where heart rate and rhythm data have limited clinical utility by an inability to place cardiac events into the context of what the patient may be doing during such events. Traditionally, patients are asked to maintain a diary of their activities to allow for later analysis of these events. The improved systems and techniques herein provide missing information so the interpreting physician will know if the patient is sleeping, exercising, hyperventilating, etc. at the time of an arrhythmic event or patient reported symptom. By placing the circumstances in the correct context, the appropriate clinical action can be taken. With situations where there is not a significant arrhythmia, the improved system can still provide valuable wellness information with biometric information, such as activity, respiration rate, and body position.

For example, in one implementation, the remote server may prepare reports, or graphically present via a user interface, including detailed information based on an arrhythmia event along with an associated biometric context for the arrhythmia event. The arrhythmia information and the contextual biometric information is graphically presented in a predetermined relationship illustrating visually the context for the detected arrhythmias. For example, the report illustrates the arrhythmia event overlaid on the contextual biometric information that corresponds to the arrhythmia event.

For example, overlaying the arrhythmia event on the contextual biometric information includes displaying the arrhythmia event information on a foreground of a display area, while the contextual biometric information is graphically presented on a background of the display area.

In an example, overlaying the arrhythmia event on the contextual biometric information includes graphical animation to first present the contextual biometric information in a display area and then, upon user prompt or after a short configurable delay, displaying the arrhythmia event information on top of the presented biometric information on the display area. In an example, overlaying the arrhythmia event on the contextual biometric information includes graphical animation to first present the arrhythmia event information on the display area and then presenting contextual biometric information in a display area and then, upon user prompt or after a short configurable delay, displaying contextual biometric information in the display area. In an example, overlaying the arrhythmia event on the contextual biometric information includes visually presenting graphical animation to first present the arrhythmia event information on the display area and then presenting contextual biometric information in a display area and then, upon user prompt or after a short configurable delay, displaying contextual biometric information in the display area.

The remote server may be in communication with a user interface, such as a computer monitor, laptop, tablet, or a portable handheld device associated with a technician or a physician. As such, the remote server may cause the report to be displayed on the user interface such that it is viewable by the technician or caregiver in a way that increases diagnostic yield or diagnostic quality.

In another implementation, the cardiac sensor and/or portable gateway may be further configured to receive an input from a patient indicating that the patient is experiencing a symptom suspected of being related to a cardiac issue in the patient. For example, a suspected cardiac-related symptom may include light-headedness, a racing heart, fatigue, fainting, a fall, chest discomfort, a skipped heartbeat, and/or shortness of breath. The cardiac sensor may transmit this patient-provided input to the remote server, which may prepare reports based on the patient-provided symptom input and a biometric context for the symptom. The patient-provided symptom input and the contextual biometric information is graphically presented in a predetermined relationship illustrating visually the context for the symptom. For example, the report illustrates the patient-provided symptom input overlaid on the contextual biometric information that corresponds to the symptom input. The overlying of the symptom input on the contextual biometric information includes similar systems and techniques described for the arrhythmia event report above. Similar to the arrhythmia event report discussed above, the remote server may cause the report to be displayed on the user interface such that it is viewable by a technician or caregiver.

In another implementation, the remote server may prepare reports for a predetermined use period of the cardiac sensor. The predetermined use period may be, for example, 24 hours, 48 hours, 72 hours, a week, two weeks, a month, 2 months, 3 months, 5 months, or 6 months. Alternatively, the use period may be specified by a user under authorization from a licensed prescriber. The remote server may identify arrhythmias that occurred in the patient over the predetermined use period. The remote server may also determine a biometric context summary for the predetermined use period, where the biometric context summary includes a summary of one or more biometric parameters of the patient over the predetermined use period. For instance, the biometric context summary may include how many hours per day of the use period that the patient was asleep, how many hours per day of the use period that the patient was active, what percentage of arrhythmias occurred while the patient was asleep over the use period, and so on. Additionally or alternatively, the identification of the arrhythmias over the use period and the biometric context summary for the use period may be determined by the cardiac sensor and/or portable gateway device and transmitted to the remote server. Once the determination of the arrhythmia events and biometric context summary for the predetermined use period is made, the remote server may prepare a report for the use period. If the report is an interim report, a user under authorization from a licensed prescriber may specify the length of the report period as the predetermined use period described above. For instance, the user may seek interim reports once a day, once every two days, once every three days, once a week, once every two weeks, once every month, or any other configurable interim duration. In some examples, the use-period report can cover a custom period of time. For example, the user under authorization from a licensed prescriber can input a custom period range as the predetermined use period. The use-period report may include a graphical summary of the arrhythmia events over the predetermined use period. The arrhythmia information and the biometric context summary information may be graphically presented in a predetermined relationship illustrating visually the context for the detected arrhythmias over the predetermined use period. As an illustration, the predetermined relationship includes presenting via a first axis of the graphical summary, information about each of the arrhythmia events and, via a second axis of the graphical summary, information about the biometric context summary. As with the arrhythmia and symptom reports discussed above, the remote server may be in communication with a user interface and cause the use-period report to be displayed on the user interface of a computer monitor, laptop, tablet, or a portable handheld device such that it is viewable, for example, by a technician or caregiver.

Described below is a cardiac monitoring system for monitoring the status of patients with known or suspected heart conditions, such as known or suspected cardiac arrhythmias. The cardiac monitoring system includes a cardiac monitoring device, where the cardiac monitoring device includes a cardiac sensor that is continuously worn by the patient for an extended period of time (e.g., depending on how much data a caregiver needs to make a diagnosis). The cardiac sensor includes ECG electrodes that senses ECG signals from the patient, as well as one or more motion sensors that acquire motion signals (including posture information) associated with the patient. As an illustration, in some cases, the cardiac sensor may be a patch-based device configured to be adhesively coupled to the patient for the extended period of time. In some cases, the cardiac sensor may include a sensor unit connected via cables to multiple ECG electrodes, where each of the ECG electrodes is individually adhered to the patient. The cardiac sensor may be in communication with a portable gateway device configured for data transmission. Thus, through the portable gateway device, the cardiac sensor may transmit data for the patient to a remote server. Alternatively, in some cases, the cardiac monitoring device may not include a portable gateway device, and the cardiac sensor may include communications circuitry be configured to transmit data from the patient to the remote server. For example, the communications circuitry may implement broadband cellular technology (e.g., 2.5G, 2.75G, 3G, 4G, 5G cellular standards) and/or Long-Term Evolution (LTE) technology or GSM/EDGE and UMTS/HSPA technologies for high-speed wireless communication. In some implementations, the communications circuitry in the cardiac sensor and/or the portable gateway may communicate with the remote server over a Wi-Fi™ communications link based on the IEEE 802.11 standard. In some implementations, the cardiac sensor and/or portable gateway device may be part of an Internet of Things (IoT) and communicate with each other and/or the remote server 102 via IoT protocols (e.g., Constrained Application Protocol (CoAP), Message Queuing Telemetry Transport (MQTT), Wi-Fi, Zigbee, Bluetooth®, Extensible Messaging and Presence Protocol (XMPP), Data-Distribution Service (DDS), Advanced Messaging Queuing Protocol (AMQP), and/or Lightweight M2M (LwM2M)).

In some embodiments, the cardiac sensor transmits the sensed ECG signals and motion signals to a remote server via the portable gateway device. The remote server stores the ECG signals and the motion signals in a database (e.g., implemented in a non-transitory memory and in communication with a processor of the remote server). The remote server then uses the stored ECG signals to identify an arrhythmia event that occurred, or in some cases is occurring, in the patient. In some implementations, the cardiac sensor may locally store at least some of the data that is transmitted to the remote server, such as several days of data at a time. Locally storing the data may, for example, help prevent data losses when there is a connectivity issue between the cardiac monitoring device and the remote server. Additionally, the remote server uses the motion and/or other biometric signals to identify a biometric context for the arrhythmia event. For example, the biometric context may include the posture of the patient, the patient's respiratory rate, whether the patient was active, and/or whether the patient was asleep when the arrhythmia event occurred. Alternatively, in some embodiments, the identification of the arrhythmia event and/or the biometric context for the arrhythmia event may occur at the cardiac sensor and/or the portable gateway. In such embodiments, the portable gateway device may transmit information on the arrhythmia event and/or information on the biometric context for the arrhythmia event to the remote server instead of, or in addition to, the ECG and motion signals.

The remote server may prepare one or more reports based on the arrhythmia event and the biometric context for the arrhythmia event. In various embodiments, the report illustrates the arrhythmia event overlaid on the contextual biometric information that corresponds to the arrhythmia event. The remote server may be in communication with a user interface, such as an interface associated with a technician or a physician. As such, the remote server may cause the report to be displayed on the user interface such that it is viewable by the technician or physician. Additionally, in some implementations, the remote server may prepare the one or more reports based on interactive feedback with a technician. For example, a technician may provide time intervals to the remote server to include in a report or desired biometric information to include in the report, and the remote server may prepare or modify the report based on the technician's input. In some implementations, the remote server may prepare the one or more reports in concert with other report generation tools, such as programs used to take existing data sets and output the data into a particular format.

Alternatively, instead of or in addition to identifying that a patient has experienced an arrhythmia event, the cardiac monitoring system may receive an input from a patient indicating that the patient has experienced a symptom suspected to be related to a cardiac condition. For example, a suspected cardiac-related symptom may include light-headedness, a racing heart, fatigue, fainting, a fall, chest discomfort, a skipped heartbeat, and/or shortness of breath. Accordingly, in such cases, a cardiac monitoring device (e.g., including a cardiac sensor and portable gateway) is configured such that the patient can provide the input to the cardiac monitoring system (e.g., via a cardiac sensor and/or a portable gateway of the cardiac monitoring device). The portable gateway (or, in some implementations, the cardiac sensor) transmits the patient-provided symptom input to the remote server, along with ECG signals and motion signals for the patient. The remote server then identifies a biometric context for the symptom input. Alternatively, in some embodiments, the cardiac sensor and/or the portable gateway may identify the biometric context for the symptom input and transmit the biometric context to the remote server.

The remote server may then prepare one or more reports based on the patient-provided symptom input and the biometric context for the input. In various embodiments, similar to the reports on arrhythmia events discussed above, the report illustrates the patient-provided symptom input overlaid on the contextual biometric information corresponding to the patient-provided symptom input. The remote server may also be in communication with a user interface and cause the report to be displayed on the user interface such that it is viewable, for example, by a technician or physician.

In some embodiments, the cardiac monitoring system may additionally or alternatively prepare use-period reports for a patient wearing a cardiac sensor. A use period is predetermined for a patient wearing a cardiac sensor. This predetermined use period may be as described above. Alternatively, the use period may be a custom period specified by a user under authorization from a licensed prescriber. In some cases, the use period may span the entire period that the patient wears the cardiac sensor. In other cases, the use period may be a subset of the entire period that the patient wears the cardiac sensor. Similar to the embodiments discussed above, the cardiac sensor transmits ECG signals and motion signals for the patient to the remote server via the portable gateway device. The remote server stores the ECG signals and motion signals and then determines, based on the ECG signals, arrhythmia events that occurred in the patient over the predetermined use period for the cardiac sensor.

The remote server also determines, based on the motion signals, a biometric context summary for the predetermined use period. This biometric context summary may include a summary of one or more biometric parameters of the patient over the use period. For instance, the biometric context summary may include how many hours per day of the use period that the patient was asleep, how many hours per day of the use period that the patient was active, what percentage of arrhythmias occurred while the patient was asleep over the use period, what percentage of arrhythmias occurred while the patient was active over the use period, and so on. Alternatively, in some cases, the determination of the arrhythmia events over the predetermined use period and/or the determination of the biometric context summary for the predetermined use period may be performed by the cardiac sensor and/or the portable gateway device. In such embodiments, the portable gateway device may instead transmit information on the arrhythmia event and/or information on the biometric context for the arrhythmia event to the remote server.

Once the determination of the arrhythmia events and biometric context summary for the predetermined use period is made, the remote server may prepare a report for the use period. The use-period report may include a graphical summary of the arrhythmia events over the predetermined used period, where the use-period report includes information about each of the arrhythmia events, presented via a first axis of the graphical summary, and information about the biometric context summary, presented via a second axis of the graphical summary. As an illustration, the use-period report may include a graph that shows types of arrhythmias on one axis and a timeline of the predetermined use period on another axis. The graph may chart each day that an arrhythmia occurred in the patient and which type(s) of arrhythmias occurred on that day. The graph may also show biometric information for each day of the predetermined use period along the timeline, such as the number of hours the patient slept per day of the use period or the number of hours the patient was active per day of the use period. As with the arrhythmia and symptom reports discussed above, the remote server may be in communication with a user interface and cause the use-period report to be displayed on the user interface such that it is viewable, for example, by a technician or physician.

Example use case scenarios are presented below to demonstrate advantages/benefits of the improved cardiac monitoring system and techniques described herein.

In one example use case, a 76-year-old male patient wearing a cardiac monitoring device may experience a four-second pause due to sinus arrest at 4 pm, which the cardiac monitoring system detects. Yet, there is no corresponding entry in the patient's diary, meaning that the patient's caregiver does not have patient-reported biometric context information to rely on in interpreting the four-second pause. Appropriate clinical action could depend on whether the patient was awake or asleep at the time of these events. However, using the motion signals from the cardiac monitoring device, the cardiac monitoring system is able to determine the patient's activity level and body posture at the time of the pause event. Evaluation of the patient's activity level and body posture reveals that this episode, as well as previous pause episodes, occurred while the patient was asleep. This information may help the patient's caregiver determine the best treatment plan for the patient's pause episodes.

In another example use case, a 56-year-old female patient may complain to her caregiver that she is suffering from a shortness of breath with daily activities. Her caregiver may prescribe that she wear a cardiac monitoring device. Using motion signals from the cardiac monitoring device, the cardiac monitoring system may determine that the patient has a normal respiratory rate during the day and at night. However, the cardiac monitoring system may further determine that while the patient was engaged in significant activity, her sinus rate never increased above 85 beats per minute ("bpm"), suggesting a diagnosis of chronotropic incompetence.

In another example use case, a 62-year-old male patient may complain to her caregiver of heart palpitations and syncope. His caregiver may prescribe that he wear a cardiac monitoring device for 30 days. After the 30 days, the cardiac monitoring system may analyze the ECG signals and motion signals acquired by the cardiac monitoring device and determine that no significant arrhythmias occurred over the 30-day use period. The patient was able to exercise with no arrhythmias and able to sleep flat with a nighttime resting heart rate of 72 bpm. As such, the patient's caregiver may determine that the patient does not need a treatment plan at this time.

In another example use case, a patient may experience a syncopal episode or a fainting spell. The patient may report the fainting spell as a symptom input to the cardiac monitoring device. The remote server may prepare a contextual biometric report for the fainting spell, which the patient's caregiver reviews. This contextual biometric report may include, for example, the activity level and the posture of the patient when the fainting spell occurred. Based on the contextual biometric report, the patient's caregiver may correctly identify that the fainting spell was a syncope.

In another example use case, a recently adult patient (e.g., around 18-21 years of age) may report to their caregiver that they have been feeling heart palpitations. The caregiver may prescribe that the patient wear a cardiac monitoring device for 30 days to assure the patient that the palpitations are not part of an underlying cardiac condition. The remote server may prepare a use-period report for the 30 days, which includes biometric information as described herein, and the caregiver may use the report to verify to the patient that the patient does not have an underlying cardiac condition.

In another example use case, a technician may receive a preliminary report or data set from the remote server that includes a biometric context for a certain period of time around an arrhythmia. The technician may modify the period of time and submit the modified period of time to the remote server. For instance, the technician may expand the period of time to show a broader context for the arrhythmia, or may decrease the period of time to cut out less informative context. The remote server may then prepare an updated report that includes a biometric context for the modified period of time. The technician may also draft a summary for the arrhythmia, which the remote server further includes in the report.

In traditional cardiac monitoring regimes, cardiac monitors are used to monitor the heart conditions of various patients. A cardiac monitor being worn by a given patient may provide ECG signals to a remote server, which analyzes the ECG signals to identify that an arrhythmia has occurred or is occurring in the patient. The remote server may also prepare a report on the arrhythmia for a physician of the patient to review. This report may include information on the ECG of the patient and other heart information, such as an average heart rate of the patient, over the period of the arrhythmia. A cardiac monitor being worn by a given patient may also be able to provide an indication to the remote server that the patient has experienced a suspected cardiac-related symptom. Thus, the remote server may also prepare a report on the suspected cardiac-related symptom that similarly includes, for example, information on the ECG of the patient and other heart information from the time of the symptom. The remote server may further prepare a similarly configured use-period report at the end of a period of use for the device.

A physician may use these reports as part of treating the patient. For example, the physician may use these reports to diagnose a cardiac condition in the patient and determine a treatment plan for the cardiac condition. However, the physician's decisions regarding diagnosing or treating the patient may be affected by a biometric context in which the patient's arrhythmias and/or patient-reported symptom occurred, as well as by a context in which arrhythmias did not occur. As an illustration, a physician's treatment plan may be affected by whether the arrhythmias occurred while the patient was asleep as opposed to while the patient was awake or whether the arrhythmias occurred while the patient was active versus while the patient was inactive. A physician's treatment plan may also be affected by whether the patient experienced any arrhythmias while exercising. The biometric context for an arrhythmia and/or patient-reported symptom is generally not included in reports prepared from cardiac monitors. In some cases, a patient may keep a physical or virtual (e.g., stored on a remote server via an online portal or locally on non-transitory storage media) diary of their symptoms, including how and when their symptoms occurred. However, the patient may forget to record symptoms or may incompletely record symptoms, making the diary's reliability variable based on the patient. Physical diaries may also be lost, while both physical or virtual diaries may be of insufficient accuracy to be useful. For example, without a highly accurate patient diary, it may not be possible to know if a person's episodes of atrial fibrillation only occur during exercise, or whether a person's episodes of atrial fibrillation only occur during sleep, which could include daytime sleep. The ability to provide objective physiologic data could reduce or eliminate the reliance on diary information for rhythm and symptom interpretation.

As such, it would be beneficial for reports prepared by a cardiac monitoring system to include biometric context information for arrhythmias and patient-reported symptoms. Advantageously, a report including biometric context information for arrhythmias and patient-reported symptoms would help the patient's physician understand the context of the patient's arrhythmias and symptoms and therefore make a better diagnosis or treatment plan. Additionally, this information would be objectively recorded and analyzed by the cardiac monitoring system, and therefore would not rely on the reliability of the patient like the patient diary does. Such biometric context information provides clinicians with a global evaluation of arrhythmic or other cardiovascular risks in individual patients and insights into the patient's overall wellness and health status.

Additionally, depending on the reason for ambulatory arrhythmia monitoring and the duration of monitoring, in some scenarios, around 12-50% of patients may experience no arrhythmias or symptoms while undergoing arrhythmia monitoring. In such cases, longitudinal tracking of biometric parameters (e.g., tracking biometric parameters over time) is useful to clinicians. Such tracking may reveal information including discovery of a disordered sleep pattern in a patient complaining of fatigue or significant decreases in activity during periods of paroxysmal atrial fibrillation.

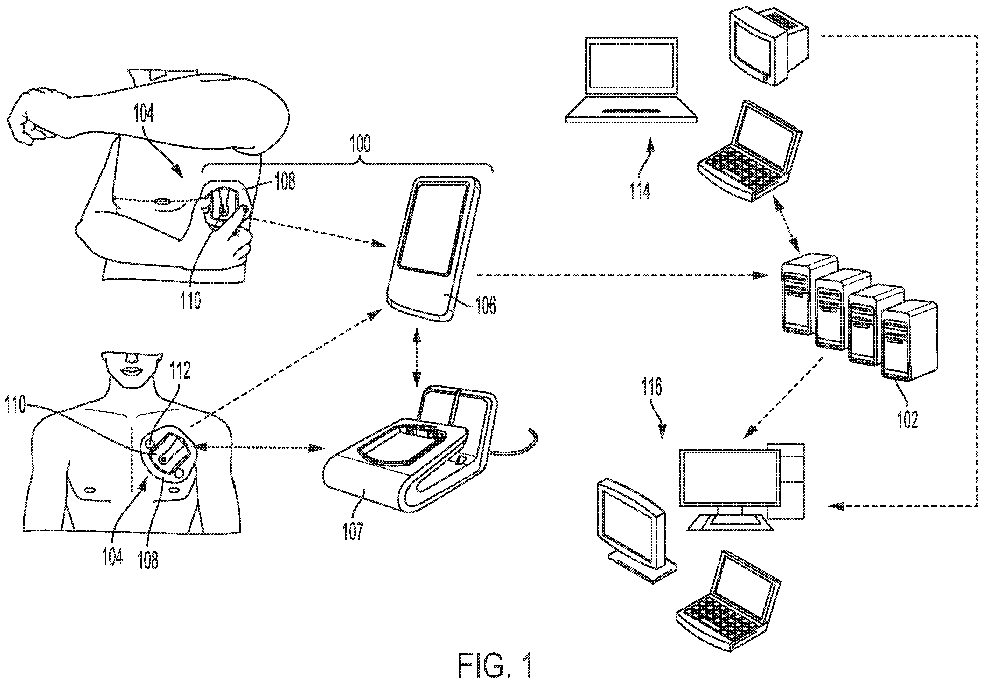

FIG. 1 shows a cardiac monitoring system that includes a cardiac monitoring device 100 in communication with a remote server 102. The cardiac monitoring device 100 is configured to gather signals from the patient and, as shown, transmit those signals to the remote server 102. More specifically, in some embodiments, the cardiac monitoring device 100 includes a cardiac sensor 104, a portable gateway 106, and a charger 107. The cardiac sensor 104 is configured to sense signals from the patient (e.g., ECG signals) and acquire motion signals associated with the patient (e.g., 1-axis channel, 2-axis channel, 3-axis channel, or multi-axis channel accelerometer signals, gyroscope signals, magnetometer signals, ballistocardiograph signals, and the like) and transmit the ECG and motion signals to the portable gateway 106. In turn, the portable gateway 106 transmits the sensed and acquired signals to the remote server 102. Alternatively, in some embodiments, the cardiac monitoring device 100 does not include a portable gateway 106 and instead includes the cardiac sensor 104 (with internal communications circuitry, as noted above) and the charger 107.

In some embodiments, such as the embodiment shown in FIG. 1, the cardiac sensor 104 includes an adhesive patch 108 configured to be adhesively coupled to the skin of a patient and a sensor unit 110 configured to be removably attached to the adhesive patch 108. The adhesive patch 108 includes two or more ECG sensors 112 that sense ECG signals from the patient and are in electronic communication with the sensor unit 110. For example, the ECG sensors may be ECG electrodes that are integrated in the adhesive patch 108. Similarly, the sensor unit 110 additionally includes at least one sensor configured to sense motion signals and/or other biometric signals of the patient. To illustrate, in some embodiments, the sensor unit 110 may include at least one accelerometer that senses accelerometer signals associated with the patient. The accelerometer may be, for instance, a 3D accelerometer with three axes and a span of $\pm$ 2g such that the accelerometer can be used to measure patient posture, respiration rate, and activity level.

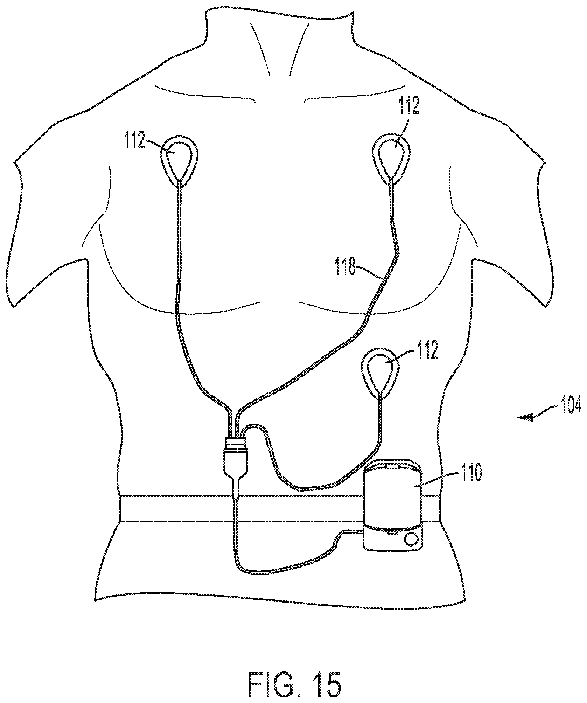

Alternatively or additionally, in some embodiments, the portable gateway 106 includes at least one sensor configured to sense motion signals of the patient, such as a 3D accelerometer. Further, another embodiment of the cardiac sensor 104 may include a sensor unit 110 connected via cables to electrodes 112 that are individually adhered to the patient, as shown in FIG. 15 and described below. In such embodiments, the sensor unit 110 may include at least one sensor configured to sense motion signals and/or other biometric signals of the patient. The sensor may additionally or alternatively coupled to one or more of the electrodes 112 and/or the cables connecting the sensor unit 110 to the electrodes 112.

Alternatively, or additionally, the sensor unit 110 may include another type of motion sensor, such as a gyroscope configured to detect patient posture and movement. Further, in some embodiments, the cardiac sensor 104 may include additional physiological sensors or functionalities. As an example, the cardiac sensor 104 includes a radiofrequency (RF) sensor comprising an RF antenna and RF transmitter and receiver such that the cardiac sensor 104 can take bio-impedance measurements of the patient's thorax. RF technology as described herein is available from ZOLL Medical Corporation (Chelmsford, MA), and makes use of a non-ionizing techniques by illuminating the body with low-power electromagnetic pulses at microwave range frequencies (approximately 0.5-30 GHz, more particularly approximately 0.5-5 GHz, more particularly approximately 0.5-2.5 GHz) and measuring the returned RF signals. The reflected and refracted RF signals enable the system to discern between different tissues based on their electromagnetic properties. Such RF technologies are biologically safe, having electromagnetic power much lower than those from a cellular phone. The RF sensor can be used to determine physiological information such as heart wall motion, respiration, thoracic fluid context (e.g., lung water), certain arrhythmias, heart valve abnormalities, arterial pulse information (e.g. pulse transit time (PTT), pulse arrival times (PAT)), blood pressure readings based on the arterial pulse information. Additionally or alternatively, the cardiac sensor 104 includes a temperature sensing device configured to sense a body temperature of the patient. For example, the temperature sensing device can include an infrared device for determining the body temperature. Additionally or alternatively, the cardiac sensor 104 includes a pulse oximeter configured to sense oxygen saturation information for the patient. Additionally or alternatively, the cardiac sensor 104 includes a respiration accelerometer that is separate from a motion sensor used to measure, for instance, the patient's posture and activity level. To illustrate, the respiratory sensor may be based on the operation of a tri-axis microelectromechanical system (MEMS) accelerometer within the cardiac sensor 104 when mounted on the patient's torso.

Further, the cardiac sensor 104 is configured for long-term and/or extended use or wear by, or attachment or connection to, a patient. For example, devices as described herein may be capable of being continuously used or continuously worn by, or attached or connected to a patient, without substantial interruption (e.g., up to 24 hours or beyond, such as for weeks, months, or even years). In some implementations, such devices may be removed for a period of time before use, wear, attachment, or connection to the patient is resumed. As an illustration, devices may be removed to change batteries, carry out technical service, update the device software or firmware, and/or to take a shower or engage in other activities, without departing from the scope of the examples described herein. Such substantially or nearly continuous use or wear as described herein may nonetheless be considered continuous use or wear.

In some embodiments, the cardiac sensor 104 is configured to monitor, record, and transmit the signals to the portable gateway 106 continuously. In particular, the cardiac sensor 104 may not interrupt monitoring and/or recording additional data while transmitting already acquired data to the portable gateway 106. Put another way, in some embodiments, both the monitoring/recording and the transmission processes occur at the same time or at least nearly at the same time.

As another example, if the cardiac sensor 104 does suspend monitoring and/or recording additional data while it is transmitting already acquired data to the portable gateway 106, the cardiac sensor 104 may then resume monitoring and/or recording additional data prior to all the already acquired data being transmitted to the portable gateway 106. As an illustration, the interruption period for monitoring and/or recording may be less in comparison to the time it takes to transmit the already acquired data (e.g., between about 0% to about 80%, about 0% to about 60%, about 0% to about 40%, about 0% to about 20%, about 0% to about 10%, about 0% to about 5%, including values and subranges therebetween). This moderate interruption period facilitates the near-continuous monitoring and/or recording of additional data during transmission of already acquired physiological data. For example, in one scenario, when a measurement time is around two minutes, any period of suspension or interruption in the monitoring and/or recording of subsequent measurement data may range from a few milliseconds to about a minute. Example reasons for such suspension or interruption of data may include allowing for the completion of certain data integrity and/or other online test of previously acquired data. If the previous measurement data has problems, the cardiac sensor 104 can notify the patient and/or remote technician of the problems so that appropriate adjustments can be made.

In some embodiments, the cardiac sensor 104 may be configured to monitor, record, and transmit some data in a continuous or near-continuous manner as discussed above, while monitoring, recording, and transmitting some other data in a non-continuous manner (e.g., periodically, non-periodically, etc.). For example, the cardiac sensor 104 may be configured to record and transmit ECG data continuously or nearly continuously while biometric-based measurements and/or transmissions may be periodic. As an illustration, ECG data may be transmitted to the portable gateway 106 (and subsequently the remote server 102) continuously or near-continuously as additional ECG data is being recorded, while biometric measurements may be transmitted once the measuring process is completed. Monitoring and/or recording of signals by the cardiac sensor 104 may be periodic and, in some embodiments, may be accomplished as scheduled (e.g., periodically) without delay or latency during the transmission of already acquired data to the portable gateway 106. For example, the cardiac sensor 104 may sense signals or acquire signals from the patient in a periodic manner and transmit the data to the portable gateway 106 in a continuous manner as described above.

As discussed above, the portable gateway 106 is configured to receive the signals sensed or acquired by the cardiac sensor 104 and transmit the signals to the remote server 102. Accordingly, the portable gateway 106 may be in wired and/or wireless communication with the cardiac sensor 104 and the remote server 102. As an illustration, the portable gateway 106 may communicate with the cardiac sensor 104 via Bluetooth®, via Ethernet, via Wi-Fi, via RF, via near-field communication (NFC), and the like. The portable gateway 106 may further communicate with the remote server 102 via cellular networks, via Bluetooth®-to-TCP/IP access point communication, via Ethernet, via Wi-Fi, and the like.

In some embodiments, the portable gateway 106 may transmit the signals sensed or acquired by the cardiac sensor 104 continuously to the remote server 102. Thus, for example, the portable gateway 106 may transmit the signals from the cardiac sensor 104 to the remote server 102 with little or no delay or latency. To this end, in the context of data transmission between the cardiac monitoring device 100, including the cardiac sensor 104 and the portable gateway 106, and the remote server 102, continuously includes continuous (e.g., without interruption) or near continuous (e.g., within one minute after completion of a measurement and/or an occurrence of an event on the cardiac sensor 104). Continuity may also be achieved by repetitive successive bursts of transmission (e.g., high-speed transmission). Similarly, immediate includes occurring or done immediately or nearly immediately (e.g., within one minute after the completion of a measurement and/or an occurrence of an event on the cardiac sensor 104).

Further in the context of signal acquisition and transmission by the cardiac monitoring device 100, continuously also includes uninterrupted collection of sensor data, such as ECG data and/or motion data, with clinical continuity. In this case, short interruptions in data acquisition of up to one second several times an hour or longer interruptions of a few minutes several times a day may be tolerated and still seen as continuous. As to latency as a result of such a continuous scheme as described herein, this relates to the overall budget of response time that can amount to between about five to about fifteen minutes overall response time (e.g., time from when an event onset is detected to when a notification regarding the event is issued). As such, transmission/acquisition latency would therefore be in the order of minutes.

In some embodiments, the bandwidth of the link between the cardiac sensor 104 and the portable gateway 106 may be larger, and in some instances, significantly larger, than the bandwidth of the acquired data to be transmitted via the link (e.g., burst transmissions). Such embodiments may ameliorate issues that may arise during link interruptions, periods of reduce/absent reception, etc. In some embodiments, when transmission is resumed after interruption, the resumption may be in the form of last-in-first-out (LIFO). Additionally, in some embodiments, the portable gateway 106 may be configured to operate in a store and forward mode where the data received from the cardiac sensor 104 is first stored in an onboard memory of the portable gateway 106 and then forwarded to the remote server 102. In some embodiments, the portable gateway 106 may function as a pipeline and pass through data from the cardiac sensor 104 immediately to the remote server 102. Further, in some embodiments, the data from the cardiac sensor 104 may be compressed using data compression techniques to reduce memory requirements as well as transmission times and power consumption.

Alternatively, in some embodiments, the cardiac sensor 104 may be configured to transmit the sensed or acquired signals to the remote server 102 instead of, or in addition to, transmitting the signals to the portable gateway 106. Accordingly, the cardiac sensor 104 may be in wired or wireless communication with the remote server 102. As an illustration, the cardiac sensor 104 may communicate with the remote server 102 via cellular networks, via Ethernet, via Wi-Fi channels, and the like. Further, in some embodiments, the cardiac monitoring device 100 may not include the portable gateway 106. In such embodiments, the cardiac sensor 104 may perform the functions of the portable gateway 106 described above.

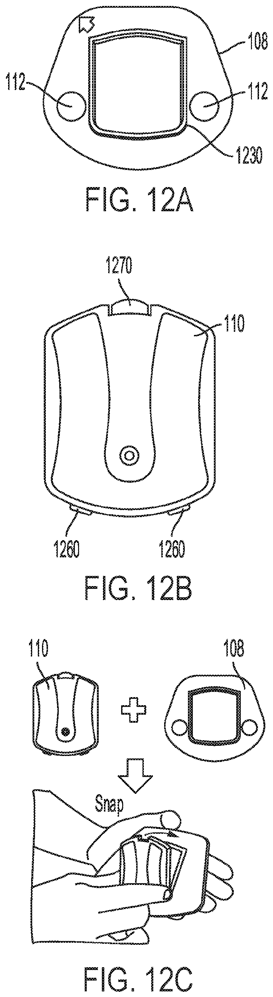

The charger 107 includes charging cradles configured to hold and recharge the sensor unit 110 and the portable gateway 106. Alternatively, in some embodiments, the cardiac monitoring device 100 may not include the portable gateway, and accordingly, the charger 107 may be configured to hold the sensor unit 110 alone. Embodiments of the cardiac monitoring device 100 are described in further detail below with reference to FIGS. 12A-16D.

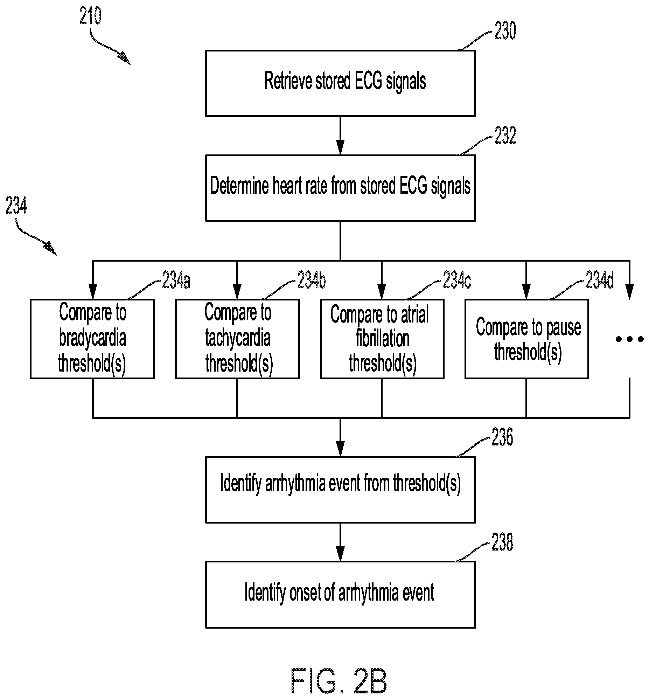

The remote server 102 is configured to receive and process the signals transmitted by the cardiac monitoring device 100. Accordingly, the remote server 102 may include a computing device, or a network of computing devices, including at least one database (e.g., implemented in a non-transitory memory) and at least one processor configured to execute instructions (e.g., stored in the database, with the at least one processor being in communication with the database) to receive and process the signals transmitted by the cardiac monitoring device 100. In various embodiments, the remote server 102 identifies an arrhythmia that has occurred in the patient from the ECG signals transmitted by the cardiac monitoring device 100, as described in further detail below (e.g., with reference to FIG. 2B). Types of arrhythmias detected by the remote server 102 may include ventricular ectopic beats (VEB), ventricular runs/ventricular tachycardia, bigeminy, supraventricular ectopic beats (SVEB), supraventricular tachycardia, atrial fibrillation, ventricular fibrillation, pauses, $2^{nd}$ AV blocks, $3^{rd}$ AV blocks, bradycardia, and/or other types of tachycardia. Additionally, the remote server 102 may perform other processing or analyses of the ECG signal, such as band pass filtering, detecting R-R intervals, detecting QRS intervals, and/or heart rate estimation.

Alternatively, in some embodiments, the identification of arrhythmias from the ECG signal and other ECG analysis may be performed by the cardiac monitoring device 100. As an example, the cardiac sensor 104 may transmit ECG signals to the portable gateway 106, which detects an arrhythmia from the ECG signals. The portable gateway 106 may then transmit an indication that an arrhythmia has occurred to the remote server 102, along with the relevant ECG signals. In another example, the portable gateway 106 may detect an arrhythmia from the ECG signals and transmit the indication that an arrhythmia has occurred and the complete ECG signals to the remote server 102. In another example, the cardiac sensor 104 may be configured to detect an arrhythmia from the ECG signals sensed by the cardiac sensor 104. The cardiac sensor 104 may transmit the indication that the arrhythmia has occurred, along with the relevant ECG signals and/or the complete ECG signals, to the portable gateway 106. The portable gateway 106 then passes the indication that the arrhythmia has occurred and the relevant and/or complete ECG signals to the remote server 102. As noted herein, arrhythmia detection and/or determination processes can be implemented on one or more of the cardiac sensor, portable gateway, and/or the remote server.

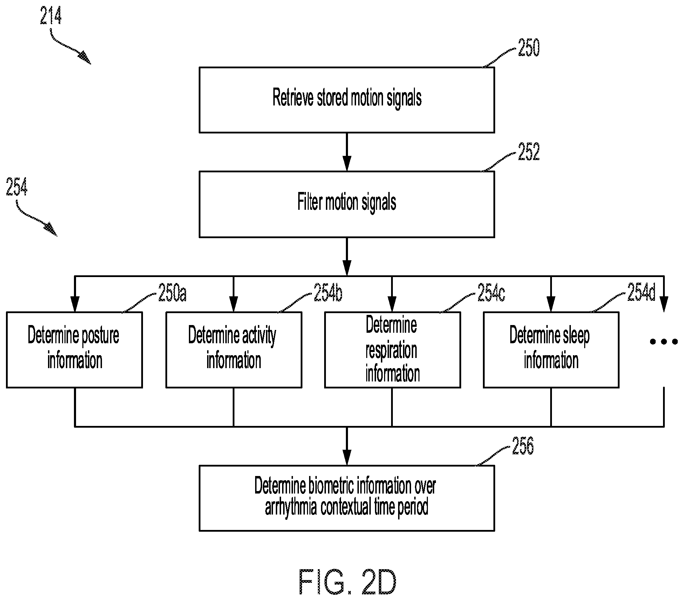

In various embodiments, the remote server 102 also processes the motion signals received from the cardiac monitoring device 100 to determine biometric parameters for the patient. For instance, an accelerometer of the cardiac sensor 104 may measure patient posture (e.g., 45° $\pm$ 20°) and detect movement indicative of respiration rate and activity level. Accordingly, the remote server 102 may process the accelerometer signals to identify patient posture, respiration rate, and activity level over periods of time. Other biometric information that may be determined for the patient may include, for example, sleep status, heart rate recovery, blood pressure, body temperature, and so on. In some implementations, biometric information may additionally or alternatively include environmental conditions for the patient and/or cardiac monitoring device 100, such as the temperature, humidity level, pressure, elevation, windchill, air quality, auditory noise level, and so on outside the cardiac monitoring device 100. As such, in some implementations, the cardiac monitoring device 100 may additionally or alternatively include other biometric sensors (e.g., non-motion biometric sensors) that transmit biometric signals to the remote server 102 for processing. The process of determining the biometric information for a patient is described in further detail below (e.g., with reference to FIG. 2D).

In some embodiments, the remote server 102 may also process other biometric signals transmitted by the cardiac monitoring device 100, such as RF signals used to determine the patient's thoracic impedance levels. These thoracic impedance levels may be further used to identify an amount of fluid in the patient's thorax. Additionally, the remote server 102 may perform secondary processing of the patient's biometrics determined from the accelerometer or other signals transmitted by the cardiac monitoring device 100 to determine further biometrics for the patient. As an example, in some embodiments, the remote server 102 may determine the patient's sleep status (e.g., whether the patient is awake or asleep) based on a combination of the patient's posture, activity level, and respiration rate. For instance, the remote server 102 may determine that the patient is asleep at any given minute where activity (e.g., as determined by accelerometer counts) is detected for less than 12 seconds and the patient's average posture is less than or equal to 35 degrees for that minute.

Alternatively, in some embodiments, the processing of the signals measuring biometric parameters of the patient that are acquired by the cardiac monitoring device 100 may occur at the cardiac monitoring device 100. As an example, the cardiac sensor 104 may transmit accelerometer signals to the portable gateway 106, which determines patient posture, activity level, respiration rate, and/or sleep status over time from the accelerometer signals. The portable gateway 106 may then transmit the patient posture, activity level, respiration rate, and/or sleep status over time to the remote server 102. In another example, the cardiac sensor 104 may be configured to determine the patient posture, activity level, respiration rate, and/or sleep status over time from the accelerometer signals acquired by the cardiac sensor 104. The cardiac sensor 104 may then transmit the patient posture, activity level, respiration rate, and/or sleep status over time to the portable gateway 106, which then passes these biometrics to the remote server 102.

As shown in FIG. 1, the cardiac monitoring system further includes one or more user interfaces, such as technician interfaces 114 and caregiver interfaces 116. The technician interfaces 114 and caregiver interfaces 116 are in electronic communication with the remote server 102 through a wired or wireless connection. For instance, the technician interfaces 114 and caregiver interfaces 116 may communicate with the remote server 102 via Wi-Fi, via Ethernet, via cellular networks, and the like. Additionally, as shown, at least some of the technician interfaces 114 may also be in electronic communication with at least some of the caregiver interfaces 116 through a wired or wireless connection, such as via Wi-Fi, via Ethernet, via cellular networks, and the like. The technician interfaces 114 and the caregiver interfaces 116 may include, for example, desktop computers, laptop computers, and/or portable personal digital assistants (e.g., smartphones, tablet computers, etc.).

The technician interfaces 114 are configured to electronically communicate with the remote server 102 for the purpose of preparing reports on patients wearing cardiac monitoring devices 100. Accordingly, a technician interface 114 may include a computing device having a processor communicably connected to a memory and a visual display. The technician interface 114 may display to a user of the technician interface 114 (e.g., a technician) one or more parameters relating to the patient and/or to preparation of a report. The user of the technician interface 114 can interact with the parameters via the technician interface 114 to guide the remote server 102 in preparing a report on a patient. As an example, a technician may select a time period to use for a report, and the remote server 102 prepare a report corresponding to the selected time period. As another example, a technician may view a report prepared by the remote server 102 and draft a summary of the report that is included in a summary section of the report. Alternatively, in some embodiments, the remote server 102 may prepare a patient report with minimal or no input or interaction with a technician via a technician interface 114. In this way, the remote server 102 may prepare a patient report through a completely or mostly automated process.

The caregiver interfaces 116 are configured to electronically communicate with the remote server 102 for the purpose of viewing reports on patients wearing cardiac monitoring devices 100. As such, a caregiver interface 116 may include a computing device having a processor communicably connected to a memory and a visual display. The caregiver interface 116 may display to a user of the caregiver interface 116 (e.g., a physician, a nurse, or other caregiver) a patient report prepared by the remote server 102. In some implementations, the user of the caregiver interface 116 may be able to interact with the patient report. For example, the user of the caregiver interface 116 may be able to select a portion of the patient report and, in response, be able to view additional information relating to the selected portion of the report. In implementations, the user of the caregiver interface 116 may view the patient report without interacting with the patient report.

In some implementations, a technician interface 114 and/or a caregiver interface 116 may be a specialized user interface configured to communicate with the remote server 102. As an example, the technician interface 114 may be a specialized user interface configured to receive preliminary patient reports from the remote server 102, receive input from a technician to adjust the preliminary patient report (e.g., adjust a timeline of the preliminary patient report), and transmit the input to the remote server 102. The remote server 102 then uses the input from the technician to prepare a finalized patient report, which the remote server 102 transmits to the technician interface 114. As another example, the caregiver interface 116 may be a user interface specialized to receive and display patient reports from the remote server 102. In such implementations, the specialized technician interface 114 and/or caregiver interface 116 may be limited to performing functions related to the cardiac monitoring system.

In implementations, a technician interface 114 and/or a caregiver interface 116 may be a generalized user interface that has been adapted to communicate with the remote server 102. To illustrate, the technician interface 114 may be user interface executing a technician application that configures the portable personal digital assistant to communicate with the remote server 102. For example, the technician application may be downloaded from an application store or otherwise installed on the user interface. Accordingly, when the user interface executes the technician application, the user interface is configured to receive preliminary patient reports from the remote server 102, receive input from a technician, transmit the input to the remote server 102, and receive finalized patient reports from the remote server 102. Similarly, the caregiver interface 116 may be a user interface executing a caregiver application that configures the user interface to communicate with the remote server 102. The caregiver application may be similarly downloaded from an application store or otherwise installed on the user interface and, when executed, may configure the user interface to receive and display patient reports from the remote server 102. The application store is typically included within an operating system of the device implementing the user interface. For example, in a device implementing an operating system provided by Apple Inc. (Cupertino, California), the application store can be the App Store, a digital distribution platform, developed and maintained by Apple Inc., for mobile apps on its iOS and iPadOS operating systems. The application store allows users to browse and download a technician and/or caregiver interface app developed with in accordance with Apple's iOS Software Development Kit. For example, such technician and/or caregiver interface apps can be downloaded on the iPhone smartphone, the iPod Touch handheld computer, or the iPad tablet computer, and some can be transferred to the Apple Watch smartwatch.

In some cases, the technician application and the caregiver application may be the same application, and the application may provide different functionalities to the device executing the application based on, for example, credentials provided by the user. For instance, the application may provide technician functionalities to a first user interface in response to authenticating technician credentials entered on the first user interface, and may provide caregiver functionalities to a second user interface in response to authenticating caregiver credentials entered on the second user interface. In other cases, the technician application and the caregiver application may be separate applications, each providing separate functionalities to a user device executing them.

In some implementations, the system shown in FIG. 1 may include other types of interfaces. To illustrate, in some examples, the system may include patient interfaces. In such examples, the remote server 102 and/or a technician interface 114 may provide a report on a patient wearing a cardiac monitoring device 100 to the patient via a patient interface. This report may be the same as the report provided to a caregiver via a caregiver interface 116, or this report may be different from the report provided to a caregiver via a caregiver interface 116. For instance, the report provided to a patient may be an abridged version of the patient report prepared for the caregiver, such as a report that does not include ECG information. The report for the patient may instead include a summary of the arrhythmia events experienced by the patient over a longer period of time (e.g., a week of wearing the cardiac monitoring device 100), whereas the caregiver may be provided with a daily report for the patient that includes ECG information for each arrhythmia experienced by the patient. In various implementations, the patient interface may be configured similarly to and function similarly to the caregiver interface 116 discussed above (e.g., with some additional restrictions on what is included in a report and/or functionalities the patient can access).

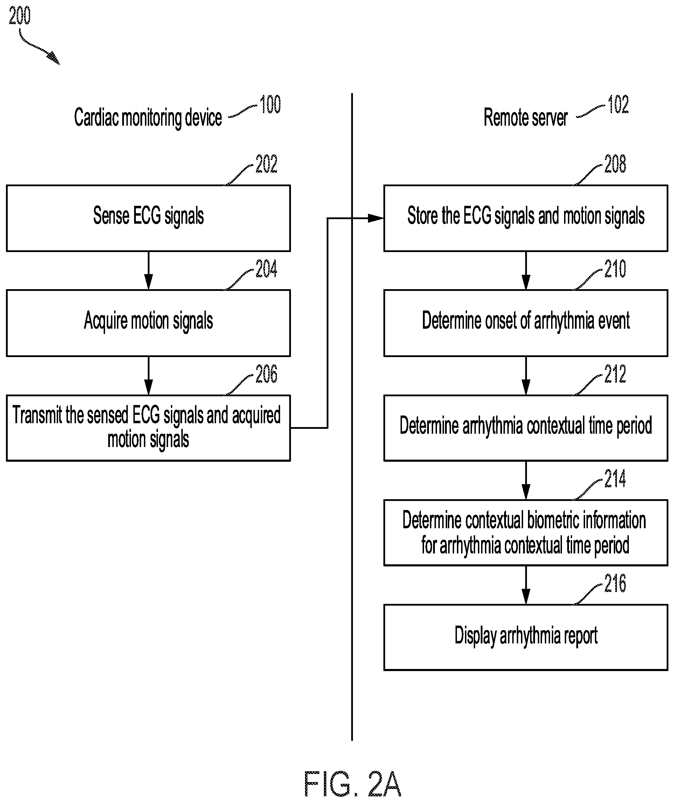

FIG. 2A illustrates a sample process flow for displaying contextual biometric information for arrhythmia events occurring in a patient. For example, the sample process 200 as shown in FIG. 2A can be implemented by the cardiac monitoring device 100 (e.g., a processor of the cardiac sensor 104 and/or a processor of the portable gateway 106) and by the remote server 102 (e.g., a processor of the remote server 102).

As shown in FIG. 2A, the cardiac monitoring device 100 senses ECG signals at step 202. The cardiac monitoring device 100 senses the ECG signals via the electrodes 112 of the cardiac monitoring device 100. As an illustration, in some embodiments, the cardiac monitoring device 100 may sense the ECG signals though electrodes 112 integrated into the adhesive patch 108, which are acquired at the sensor unit 110. As another illustration, in some embodiments, the cardiac monitoring device 100 may sense the ECG signals though ECG electrodes 112 that are coupled via cables 118 to a sensor unit 110 as shown in FIG. 15.

The cardiac monitoring device 100 also acquires motion signals at step 204. In various embodiments, the cardiac monitoring device 100 acquires the motion signals via one or more motion sensors incorporated into the cardiac monitoring device 100. The motion sensor(s) may include, for example, one or more accelerometers, gyroscopes, magnetometers, ballistocardiographs, and the like. For instance, the cardiac monitoring device 100 may include a sensor unit 110 that includes a 3D accelerometer with three axes. As such, the motion signals include motion and/or posture information. As another example, the cardiac monitoring device 100 includes an adhesive patch 108 with an integrated motion sensor, such as an integrated accelerometer. As another example, the cardiac monitoring device 100 includes individual electrodes 112 configured to be disposed at predetermined anatomical locations on the patient's body (e.g., adhered to specific locations on the patient's body). For example, the locations can be the patient's lateral left chest wall at the mid-axillary line at the nipple level or the patient's front lest chest location. As such, the motion signals may be acquired via one or more accelerometers coupled to the electrodes 112 and/or coupled to connections between the electrodes 112 and a sensor unit 110 (e.g., coupled to wires connecting the electrodes 112 to the sensor unit 110).

After sensing the ECG signals and acquiring motion signals, the cardiac monitoring device 100 transmits the sensed ECG signals and acquired motion signals to the remote server 102 at step 206. In some embodiments, the portable gateway 106 transmits the ECG signals and motion signals to the remote server 102. For example, the sensor unit 110 may receive ECG signals from the electrodes 112, and the sensor unit 110 may acquire motion signals via one or more motion sensors incorporated into the adhesive patch 108, incorporated into the sensor unit 110, and/or coupled to the electrodes 112. The sensor unit 110 may then transmit the ECG signals and motion signals to the portable gateway 106 (e.g., via Bluetooth®). The portable gateway 106 further transmits the ECG signals and motion signals to the remote server 102 (e.g., via cellular networks, via Wi-Fi). In other embodiments, the cardiac monitoring device 100 may not include a portable gateway 106, and the cardiac sensor 104 may instead transmit the ECG signals and motion signals to the remote server 102 (e.g., via cellular networks or via Wi-Fi as noted above).

The remote server 102 stores the ECG signals and motion signals at step 208. For example, the remote server 102 may include one or more databases and store the ECG signals and motion signals in a database of the remote server 102. The remote server 102 also determines the onset of an arrhythmia event based on the received ECG signals at step 210. The remote server 102 (or, in some embodiments, the sensor unit 110 itself) may execute ECG classification processes to detect and classify the beats and rhythms of the patient's heartbeat from the received data. For example, the ECG classification processes may detect ventricular ectopic beats, ventricular couplets, short (e.g., non-sustained) runs (e.g., ventricular runs of less than about 30-second duration), long (e.g., sustained) runs (e.g., ventricular runs of greater than about 30-second duration), supraventricular ectopic beats (SVEBs), ventricular couplets, and/or the like. In some embodiments, the ECG classification unit may detect bradycardia, tachycardia, atrial fibrillation episodes, pauses (e.g., in the heartbeat of the patient), ventricular tachycardia, bigeminy, trigeminy, multigeminy, supraventricular tachycardia, ventricular fibrillation, and heart block (e.g., atrioventricular (AV) blocks including first degree, second degree, and/or third degree heart blocks). In some instances, the AV second-degree blocks may include type I and/or type II blocks.

Further, in some embodiments, the ECG classification processes may include a feature extraction process that is configured to determine various features of the beats classified by the ECG classification processes. For example, the feature extraction process may calculate beat features including, but not limited to, QRS width, polarity, maximum-to-minimum ratio, P-wave, existence, and/or the like. The ECG classification processes can include rhythm change classifiers. Such example, such rhythm change classifies may be implemented in a non-transitory computer-readable medium (e.g., a memory, a programmable circuit board, a field programmable gate array, an integrated circuit, any combination thereof, and/or the like). The rhythm change classifier may include at least one neural network trained based on a historical collection of a plurality of ECG signal portions with known rhythm change information. Additionally, at least one processor may be operatively connected to the ECG channel(s) and the non-transitory computer-readable medium. The processor(s) may receive the ECG signal(s) via the ECG channel(s). The processor(s) may also use the rhythm change classifier to detect time data corresponding to a rhythm change (e.g., predetermined rhythm change and/or the like) in the ECG signal(s). For example, the time data may include a start time, a time interval, any combination thereof, and/or the like. The processor(s) may also determine, based on the detected time data, at least one ECG signal portion associated with the detected time data corresponding to the predetermined rhythm change in the ECG signal(s). The processor(s) may also transmit the determined ECG signal portion(s) to a remote computer system (e.g., a remote server, a cardiac monitoring facility, and/or the like). For example, in such embodiments, the rhythm change classifier (e.g., neural network(s) and/or the like thereof) may detect (e.g., identify and/or the like) the rhythm change without having to classify the specific rhythm type. For the purpose of illustration, the rhythm change classifier may detect a rhythm when heart rhythm changes from normal sinus rhythm (NSR) to Atrial Fibrillation (AFIB), from AFIB to NSR, from AFIB to Atrial Flutter (AFL), from one morphology to another, and/or the like. Additionally, every time a rhythm change is detected (e.g., identified and/or the like), an ECG signal portions (e.g., ECG strip and/or the like) containing the detected rhythm change may be transmitted to the remote computer system (e.g., with an indication, message, marking, and/or the like indicating a detected rhythm change). Additionally or alternatively, the rhythm change classifier may determine a confidence score associated with the detected rhythm change (e.g., output the confidence score associated with the probability that the detected rhythm change actually is a rhythm change). An example process flow for step 210 of determining the onset of the arrhythmia event is shown in FIG. 2B.

In the example process flow shown in FIG. 2B, the remote server 102 retrieves the stored ECG signals (e.g., from a database of the remote server 102) at step 230. The remote server 102 then determines heart rate and/or heart rate recovery information from the stored ECG signals at step 232. For example, the remote server 102 may be configured to perform QRS detection for the ECG signals. In some implementations, the remote server 102 may use a Pan-Tompkins-based QRS detector. In some implementations, the remote server 102 may use a Hilbert transform process to perform the QRS detection. In some implementations, the remote server 102 may use a phasor transform process to perform the QRS detection. The remote server 102 next uses the detected QRS complexes to determine the heart rate based on the number of QRS complexes over a certain time period. As an illustration, the remote server 102 may determine the number of R-waves over a ten-second period and multiply that number by six to determine the heart rate per minute for the patient. In some implementations, the remote server 102 may remote outliers from the ECG signals and average the QRS complexes over a time window as part of determining the heart rate. For instance, since ECG signals can suffer from noises and motion artifacts, time windows including identified noises and motion artifacts (e.g., based on comparison to an ECG template for QRS complexes) may be removed from the analysis.

For example, in some implementations, the remote server 102 may receive raw ECG data sampled at 250 Hz. The remote server 102 may then remove baseline wander, high frequency noise, and 50/60 Hz interference. This filtering may be represented mathematically by denoting the raw ECG data as *x* and representing the process as:

$$y_1 = bpf * x$$

In this equation, *bpf* represents band pass filtering, * stands for convolution, and $y_1$ represents the filtered signal. Subsequently, the remote server 102 may use a Pan-Tompkins-based QRS complex detector to identify the QRS complexes in the filtered signal. First, the remote server 102 finds the derivative of the filtered $y_1$ signal and square the result, which may be represented as:

$$y_2 = \left(\frac{dy_1}{dt}\right)^2$$

Next, the remote server 102 applies a moving average to the $y_2$ result, which may be further represented mathematically by:

$$y_3 = MovingAverageFilter * y_2$$

The remote server 102 then applies an adaptive power threshold to locate the QRS complexes in the $y3$ signal and find the locations of R peaks within the QRS complexes, where the R peaks location sequence may be represented as $\{R_i\}$.

The remote server 102 may then use $R_i$ as the input in the heart rate estimation stage. More particularly, the remote server 102 calculates heart rate for moving, overlapping, one-minute windows. Each window is tested for validity. If a window is not valid, no heart rate result is provided for that time window. Validity may be determined, for instance, based on determining no signal saturation and $RR_i$ distribution meeting validity conditions, where $RR_i$ represents a sequence of R-R intervals. For valid windows, the sequence of R-R intervals is computed as follows:

$$RR_i = R_{i+1} - R_i$$

If there are outlying R-R values, the remote server removes the two most extreme $Rri$ outliers are removed, and the heart rate is computed by:

$$heart\ rate = \frac{60}{(RR_i)}$$

In implementations, the remote server 102 compares the ECG signals to one or more stored thresholds at step 234. As an illustration, as shown in the example embodiment of FIG. 2B, the remote server 102 may compare the ECG signals to bradycardia thresholds at step 234a, to tachycardia thresholds at step 234b, to atrial fibrillation thresholds at step 234c, to pause thresholds at step 234d, and so on. Additionally or alternatively, the raw ECG signals may also be analyzed via the machine learning and/or neural network classifiers described above to determine other types of arrhythmias and/or heart abnormalities. Such analysis may or may not require calculation of the heart rate. As such, the heart rate determination step shown as step 232 is to illustrate the determination of certain arrhythmia types. In some embodiments, looking first to the bradycardia thresholds at step 234a, the remote server 102 may compare the heart rate determined from the ECG signals to a bradycardia onset threshold for heart rate. If the determined heart rate dropped below the bradycardia onset threshold (e.g., for a certain period of time, for a certain number of heartbeats), the remote server 102 determines that the patient entered bradycardia at that time. Additionally, the remote server 102 may determine when the patient left bradycardia by comparing the heart rate determined from the ECG signals to a bradycardia offset threshold for heart rate, which may or may not be the same as the bradycardia onset threshold. Once the determined heart rate rises above the bradycardia offset threshold (e.g., for a certain period of time, for a certain number of heartbeats), the remote server 102 determines that the patient left bradycardia at that time. As an illustration, the remote server 102 may determine that the patient entered bradycardia if the patient's determined heart rate dropped below 30 beats per minute for at least 45 seconds. The remote server 102 may then determine that the patient left bradycardia once the patient's determined heart rate rose above 40 beats per minute for at least 15 seconds.

In some implementations, the bradycardia onset threshold and/or the bradycardia offset threshold may be determined on a patient-by-patient basis. For instance, the bradycardia onset and offset thresholds may be determined based on physiological information about the patient (e.g., using the patient's height, weight, gender, age, health, etc.) to determine statistically probable bradycardia onset and offset thresholds based on one or more categories the patient falls into. To illustrate, the remote server 102 may determine that the bradycardia onset and offset thresholds for a 25-year-old man are higher than for a 64-year-old woman based on statistics for men between the ages of 20-30 and women between the ages of 60-70. As another example, the bradycardia onset and offset thresholds may be determined based on a baseline ECG taken, for instance, by the patient's physician. The remote server 102 may analyze the baseline ECG to identify features from the ECG, such as the patient's baseline heart rate, and set the bradycardia onset and offset thresholds based on the identified features.

In some embodiments, looking to the tachycardia thresholds at step 234b, the remote server 102 may compare the heart rate determined from the ECG signals to a tachycardia onset threshold for heart rate. If the determined heart rate rose above the tachycardia onset threshold (e.g., for a certain period of time, for a certain number of heartbeats), the remote server 102 determines that the patient entered tachycardia at that time. Further, the remote server 102 may determine when the patient left tachycardia by comparing the heart rate determined from the ECG signals to a tachycardia offset threshold for heart rate, which may or may not be the same as the tachycardia onset threshold. Once the determined heart rate drops below the tachycardia offset threshold (e.g., for a certain period of time, for a certain number of heartbeats), the remote server 102 determines that the patient left tachycardia at that time. As an illustration, the remote server 102 may determine that the patient entered tachycardia if the patient's determined heart rate rose above 140 beats per minute for at least 45 seconds. The remote server 102 may then determine that the patient left tachycardia once the patient's determined rate fell below 120 beats per minute for at least 15 seconds.

Additionally, similar to the bradycardia onset and offset thresholds, in some implementations, the tachycardia onset threshold and/or the tachycardia onset threshold may be determined on a patient-by-patient basis. For instance, the tachycardia onset and offset thresholds may be determined based on physiological information about the patient (e.g., using the patient's height, weight, gender, age, health, etc.) to determine statistically probable tachycardia onset and offset thresholds based on one or more categories the patient falls into. To illustrate, the remote server 102 may determine that the tachycardia onset and offset thresholds for a 55-year-old man are lower than for an 18-year-old man based on statistics for men between the ages of 15-22 and 50-58. As another illustration, similar to the bradycardia example discussed above, the tachycardia onset and offset thresholds may be determined based on a baseline ECG taken, for instance, by the patient's physician and features identified from the baseline.

In some embodiments, looking to the atrial fibrillation thresholds at step 234c, the remote server 102 may determine R-R intervals between successive R-waves of the ECG signals. The remote server 102 may also determine the difference between successive R-R intervals as Δ R-R. In some implementations, the remote server 102 may detect atrial fibrillation based on an analysis of sequences of R-R intervals and Δ R-R values considered as random variables, such as per a sliding window of length N centered on each beat. An application of the Kolmogorov-Smirnov test can return the probability of the hypothesis that a set of Δ R-R values over a window belongs to a standard distribution depending on a mean value of R-R intervals over the same window. In such cases, if the probability provided by the Kolmogorov-Smirnov test exceeds a threshold, then the remote server 102 may recognize the central beat (or subset of beats around the central beat) as atrial fibrillation. Further, in some implementations, the remote server 102 may require that the patient be in atrial fibrillation for a certain amount of time (e.g., one minute) before the remote server 102 determines that an atrial fibrillation event occurred.

In some embodiments, looking to the pause thresholds at step 236d, the remote server 102 may determine whether a distance between successive QRS complexes is above a pause threshold. For example, the remote server 102 may determine that a pause occurred if the distance between successive R-waves is greater than 3500 milliseconds. Further, in some implementations, the remote server 102 may impose a pause maximum, after which the remote server 102 will not identify the event as a pause. As an illustration, the remote server 102 may determine that ECG signals showing no activity for more than 20 seconds is likely due to the cardiac monitoring device 100 incorrectly receiving signals from the patient, rather than due to a cardiac event.

In some implementations, the remote server 102 may set the pause thresholds and/or the pause maximum on a patient-by-patient basis, similar to the bradycardia and tachycardia thresholds. For instance, the pause threshold may be determined based on physiological information about the patient (e.g., using the patient's height, weight, gender, age, health, etc.) to determine a statistically probable pause threshold based on one or more categories the patient falls into. To illustrate, the remote server 102 may determine that the pause threshold is higher for an 80-year-old than for a 20-year-old based on the fact that the average heart rate for an 80-year-old is lower than for a 20-year-old. As another example, similar to the bradycardia and tachycardia examples discussed above, the pause threshold may be determined based on a baseline ECG taken, for instance, by the patient's physician and features identified from the baseline.

In some embodiments, in addition to or in the alternative of, the bradycardia, tachycardia, atrial fibrillation, and pause thresholds discussed above, the remote server 102 may compare the ECG signals to other thresholds associated with other types of cardiac events at step 234. For example, the remote server 102 may use other thresholds to identify ventricular ectopic beats, ventricular couplets, short runs, long runs, SVEBs, ventricular couplets, ventricular tachycardia, bigeminy, trigeminy, multigeminy, supraventricular tachycardia, ventricular fibrillation, and/or heart blocks. Further, in some embodiments, the methods used to identify bradycardia, tachycardia, atrial fibrillation, and pause events may vary. Additional methods and details on identifying cardiac events from ECG signals are discussed in U.S. Patent Application No. 16/041,402, filed July 20, 2018, entitled "SYSTEMS, DEVICES, AND METHODS FOR PHYSIOLOGICAL MONITORING OF PATIENTS," now U.S. Patent No. 11,020,002. Yet additional methods and systems for identifying cardiac events from ECG signals based on machine learning and/or neural networks techniques are discussed in U.S. Patent Application No. 17/083,472, filed October 29, 2020, entitled "SYSTEMS, DEVICES, AND METHODS FOR CARDIAC DIAGNOSIS AND/OR MONITORING,".

The remote server 102 identifies an arrhythmia event from a threshold at step 236. For example, the remote server 102 may identify a bradycardia event, tachycardia event, atrial fibrillation event, pause event, etc. as discussed above. The remote server 102 also identifies the onset of the arrhythmia event at step 238. As an illustration, if the remote server 102 has determined that the ECG signals reflect a bradycardia event based on bradycardia onset and/or offset thresholds, the remote server 102 may determine that the bradycardia event onset occurred at the time the patient's heart rate fell below the bradycardia onset threshold. As another illustration, if the remote server 102 has determined that the ECG signals reflect a pause event based on the patient's ECG signals showing no activity for 5000 milliseconds, the remote server 102 may determine that the pause event onset occurred at the time the patient's ECG signals stopped showing activity. Alternatively, the remote server 102 may determine that the pause event onset occurred a certain amount of time into the pause event, such as 10 milliseconds after the patient's ECG signals stopped showing activity.

Additionally, in some embodiments, the remote server 102 may identify the offset of the arrhythmia event. To illustrate, if the remote server 102 has determined that the ECG signals reflect a tachycardia event based on tachycardia onset and/or offset thresholds, the remote server 102 may determine that the tachycardia event offset occurred at the time the patient's heart rate fell below the tachycardia event offset threshold. As another example, if the remote server 102 has determined that the ECG signals reflect an atrial fibrillation event based on the patient's R-R intervals and $\triangle$ R-R values, the remote server 102 may determine that the atrial fibrillation event offset occurred at the time the patient's R-R intervals and $\triangle$ R-R values reflected values.

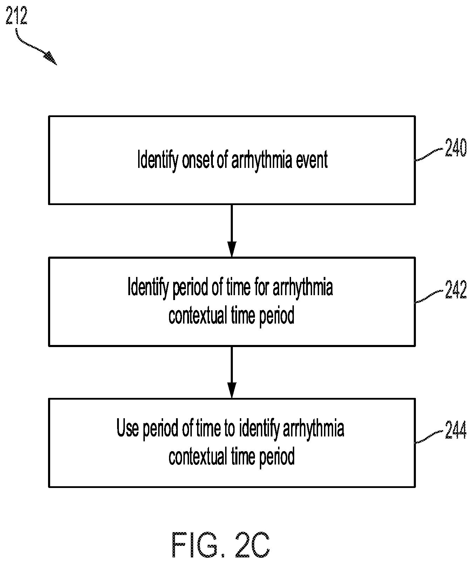

Returning to the sample process 200 shown in FIG. 2A, once the remote server 102 has determined the onset of the arrhythmia event, the remote server 102 also determines the arrhythmia contextual time period at step 212. An arrhythmia contextual time period includes a period of time surrounding the arrhythmia event onset. For example, the arrhythmia contextual time period includes time before, during, and/or after the arrhythmia event. As an example, the arrhythmia contextual time period may include a period of time before and after the arrhythmia event onset. As another example, the arrhythmia contextual time period may include a period of time before the arrhythmia event onset, during the arrhythmia event, and after the arrhythmia event offset. An example process flow for step 212 of determining the arrhythmia contextual time period is shown in FIG. 2C.

In the example process flow shown in FIG. 2C, the remote server 102 identifies the onset of the arrhythmia event at step 240. The remote server 102 may identify the onset of the arrhythmia event according to the processes discussed above with respect to step 210 and with reference to FIGS. 2A and 2B. The remote server 102 also identifies a period of time for the arrhythmia contextual time period. This period of time is an amount of time that will be used to determine the arrhythmia contextual time period. In some implementations, this period of time is a default time period for the arrhythmia event. The default time period may be the same for all arrhythmia events, or the default time period may vary depending on the type of arrhythmia event. For example, the default time period for a pause event may be shorter than the default time period for an atrial fibrillation event. In some implementations, this period of time may be input by a user. For example, a technician reviewing the identified arrhythmia event may input the period of time for the arrhythmia contextual time period via a technician interface 114. The input period of time may, in some cases, override a default period of time. As another example, a caregiver reviewing the identified arrhythmia event may input the period of time for the arrhythmia contextual time period via a caregiver interface 116.

Additionally, in some implementations, the period of time for the arrhythmia contextual time period may be a fixed period of time, while in other implementations, the period of time may be a variable period of time. For instance, the period of time may be a fixed amount of time around a portion of the arrhythmia event, such as a fixed amount of time around the onset or the offset of the arrhythmia event. Alternatively, the period of time may be a variable amount of time that depends, for example, on the length of the arrhythmia event. As an illustration, the period of time may consist of a certain amount of time before the onset of the arrhythmia event, the duration of the arrhythmia event, and a certain amount of time after the arrhythmia event.

The remote server 102 uses the period of time from step 242 to identify the arrhythmia contextual time period at step 244. In some implementations, the remote server 102 may determine that the arrhythmia contextual time period includes a time period that includes the onset of the arrhythmia event. For example, the remote server 102 may determine that the arrhythmia contextual time period includes a two-hour time period during which the onset of the arrhythmia event occurred, an hour time period during which the onset of the arrhythmia event occurred, a 30-minute time period during which the onset of the arrhythmia event occurred, a 15-minute time period during which the onset of the arrhythmia event occurred, and/or a 10-minute time period during which the onset of the arrhythmia event occurred.

As an illustration, the remote server 102 may determine that the arrhythmia event onset occurred at 9:15 am on December 2. The period of time for the arrhythmia contextual time period may be a 2-hour block. As such, the remote server 102 may determine that the arrhythmia contextual time period is 8:00-10:00 am on December 2. Alternatively, the period of time for the arrhythmia contextual time period may be 2 hours centered around the arrhythmia event onset. Thus, the remote server 102 may determine that the arrhythmia contextual time period is 8:15-10:15 am on December 2. As another alternative, the period of time for the arrhythmia contextual time period may be a first amount of time before the arrhythmia event onset and a second amount of time after the arrhythmia event onset, such as an hour before the arrhythmia event onset and 30 minutes after the arrhythmia event onset, or 90 minutes before the arrhythmia event onset and 30 minutes after the arrhythmia event onset. The remote server 102 may accordingly determine that the arrhythmia contextual time period is 8:15-9:45 am on December 2. As another alternative, the period of time for the arrhythmia contextual time period may be an amount of time that ends at the arrhythmia event onset, such that the arrhythmia contextual time period is 15 minutes before the arrhythmia event onset. Therefore, the remote server 102 may determine that the arrhythmia contextual time period is 9:00-9:15 am on December 2. As another alternative, the period of time for the arrhythmia contextual time period may be an amount of time that begins at the arrhythmia event onset, such that the arrhythmia contextual time period is 30 minutes after the arrhythmia event onset. In such cases, the remote server 102 may determine that the arrhythmia contextual time period is 9:15-9:45 am on December 2.

In some implementations, the remote server 102 may determine that the arrhythmia contextual time period includes the offset of the arrhythmia event. For example, the remote server 102 may determine that the arrhythmia contextual time period includes a two-hour time period during which the offset of the arrhythmia event occurred, an hour time period during which the offset of the arrhythmia event occurred, a 30-minute time period during which the offset of the arrhythmia event occurred, a 15-mintue time period during which the offset of the arrhythmia event occurred, and/or a 10-minute time period during which the offset of the arrhythmia event occurred.

As an illustration, the remote server 102 may determine that the arrhythmia event offset occurred at 5:24 pm (or alternatively, 17:24) on November 15. The period of time for the arrhythmia contextual time period may be a 1-hour block. As such, the remote server 102 may determine that the arrhythmia contextual time period is 5:00-6:00 pm (or alternatively, 17:00-18:00) on November 15. Alternatively, the period of time for the arrhythmia contextual time period may be 1 hour centered around the arrhythmia event offset. Thus, the remote server 102 may determine that the arrhythmia contextual time period is 4:54-5:54 pm (or alternatively, 16:54-17:54) on November 15. As another alternative, the period of time for the arrhythmia contextual time period may be a first amount of time before the arrhythmia event offset and a second amount of time after the arrhythmia event onset, such as an hour before the arrhythmia event offset and an hour and a half after the arrhythmia event offset. The remote server 102 may accordingly determine that the arrhythmia contextual time period is 4:24-6:54 pm (or alternatively, 16:24-18:54) on November 15. As another alternative, the period of time for the arrhythmia contextual time period may be an amount of time that ends at the arrhythmia event offset, such as 10 minutes before the arrhythmia event offset. Therefore, the remote server 102 may determine that the arrhythmia contextual time period is 5:14-5:24 pm (or alternatively, 17:14-17:24) on November 15. As another alternative, the period of time for the arrhythmia contextual time period may be an amount of time that begins at the arrhythmia event offset, such as an hour after the arrhythmia event offset. In such cases, the remote server 102 may determine that the arrhythmia contextual time period is 5:24-6:24 pm (or alternatively, 17:24-18:24) on November 15.

In some implementations, the remote server 102 may determine that the arrhythmia contextual time period includes a time period that includes both the onset and the offset of the arrhythmia event. In such implementations, the arrhythmia contextual time period may include, for example, various combinations of the periods of time surrounding the arrhythmia event onset and the arrhythmia event offset discussed above.

As an illustration, the remote server 102 may determine that the arrhythmia event offset occurred at 6:48 am on April 27 and that the arrhythmia event offset occurred at 8:33 am on April 27. The period of time for the arrhythmia contextual time period may be a 1-hour block that includes the arrhythmia event onset up to a 1-hour block that includes the arrhythmia event offset. As such, the remote server 102 may determine that the arrhythmia contextual time period is 6:00-9:00 am on April 27. Alternatively, the period of time for the arrhythmia contextual time period may be 30 minutes before the arrhythmia event onset up to 30 minutes after the arrhythmia event offset. Thus, the remote server 102 may determine that the arrhythmia contextual time period is 6:18-9:03 am on April 27. As another alternative, the arrhythmia contextual time period may be a first amount of time before the arrhythmia event onset and a second amount of time after the arrhythmia event offset, such as three hours before the arrhythmia event onset and an hour after the arrhythmia event offset. Accordingly, the remote server 102 may determine that the arrhythmia contextual time period is 3:48-9:33 am on April 27.

In some implementations, the remote server 102 may determine that the arrhythmia contextual time period includes a time period within the duration of the arrhythmia event. For example, the remote server 102 may determine that the arrhythmia contextual time period includes a time period a certain amount of time after the arrhythmia event onset, a certain amount of time before the arrhythmia event offset, a randomly sampled amount of time during the duration of the arrhythmia event, an amount of time during the midpoint of the arrhythmia event, and so on. The above arrhythmia contextual time periods and methods thereto are illustrative. Other arrhythmia contextual time period durations as well as other ways of calculating the arrhythmia contextual time period, are within the scope this disclosure.

In some implementations, as discussed above, the remote server 102 may set a default arrhythmia contextual time period. The default arrhythmia contextual time period may be modified by a user, such as a technician via a technician interface 114 or a caregiver via a caregiver interface 116. For example, a user may be able to select from a list of time periods for an arrhythmia contextual time period or may be able to input a custom arrhythmia contextual time period (e.g., up to 24 hours around the arrhythmia event onset).

In some implementations, the default arrhythmia contextual time period may depend on the type of arrhythmia (or symptom event, as described in further detail below). A benefit of this feature is that it provides a more relevant context for certain types of arrhythmias to enhance diagnostic yield and/or quality. As an illustration, the default arrhythmia contextual time period for a pause event may be shorter than the default arrhythmia contextual time period for a tachycardia event. In some implementations, the arrhythmia contextual time period may depend on the biometric status of the patient at the onset and/or offset of the arrhythmia event. For example, the remote server 102 may determine that the arrhythmia event onset occurred while the patient was asleep. As another example, the remote server 102 may determine whether the arrhythmia event onset occurred while the patient was active and use this information in determining the default values for the arrhythmia or symptom contextual time period.

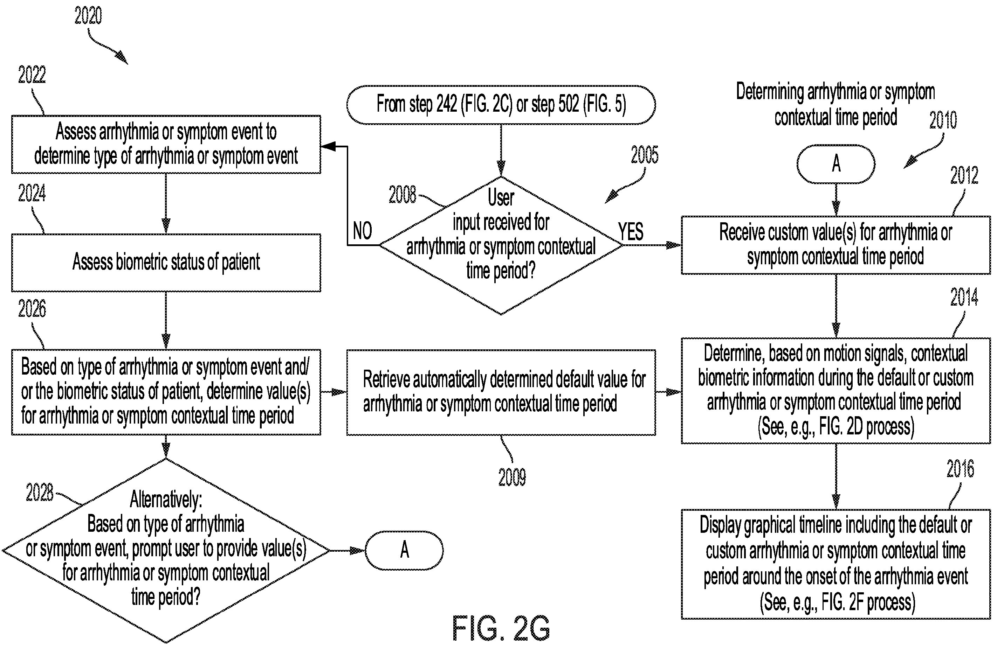

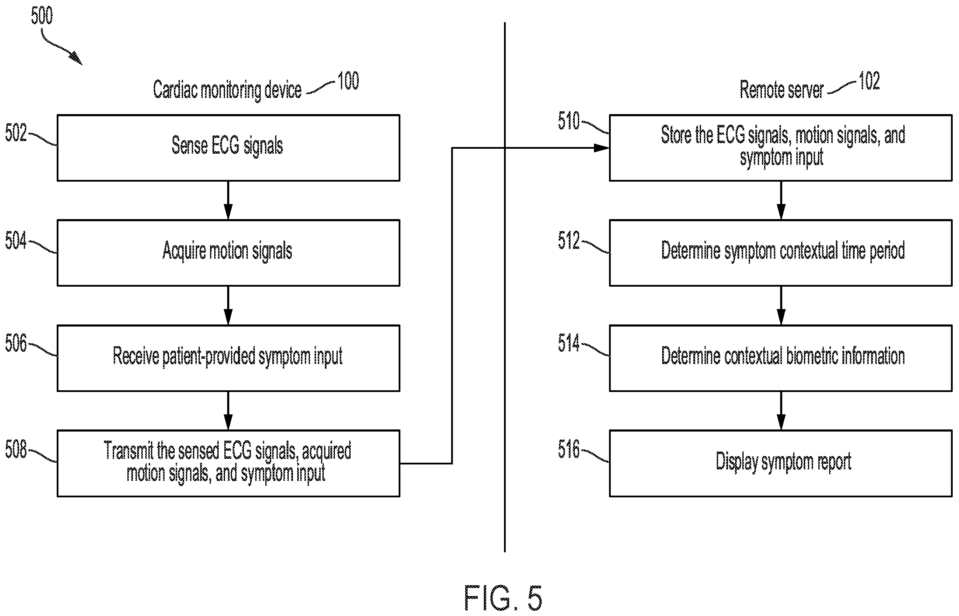

Referring to FIG. 2G, the remote server 102 may determine an arrhythmia or symptom contextual time period in accordance with a predetermined rules-based process 2005. While FIGS. 2A-2F address arrhythmia events, FIG. 2G also addresses patient input symptom events. Such patient input symptom events are described in more detail, for example, in connection FIG. 5 below. Process 2005 is initiated following a call from either step 242 of FIG. 2C or step 502 of FIG. 5.

Process 2005 determines at step 2008 whether user input is received specifying custom value(s) for an arrhythmia or symptom contextual time period. If no such user input is received, in step 2009, default value(s) for the arrhythmia or symptom contextual time period are automatically determined. Subroutine 2020, described in further detail below, automatically calculates the default value(s) for the arrhythmia or symptom contextual time period.

Subroutine 2010 is initiated if a user input for the arrhythmia or symptom contextual time period is received. In step 2012, a custom value for the arrhythmia or symptom contextual time period is received. For example, a technician user via a technician interface 114 or a caregiver user via a caregiver interface 116 may be presented with a default value for the arrhythmia or symptom contextual time period (determined as shown in subroutine 2020) and provided with a user interactive feature on the interface 114, 116 to modify the value. In some implementations, the technician or caregiver user can specify via the interface 114, 116 a custom value for the arrhythmia or symptom contextual time period. As noted above, the user may be able to select from a list of time periods for an arrhythmia or symptom contextual time period or may be able to input a custom arrhythmia or symptom contextual time period (e.g., up to 24 hours around the arrhythmia event onset or the patient-provided symptom input). In implementations, the user can select a first custom value for a preceding time period for the arrhythmia onset or symptom event, and a second custom value for a following time period for the arrhythmia onset or symptom event.

As an illustration, the user may note that the arrhythmia event onset or symptom event occurred at 6:48 am on April 27. The user may select or input a period of time for the arrhythmia or symptom contextual time period as a 1-hour block that includes the arrhythmia event onset or symptom event. In this example, the arrhythmia event onset or symptom event may be automatically centered within the 1-hour block. Accordingly, the period of time for the arrhythmia or symptom contextual time period may be 30 minutes before the arrhythmia event onset or the patient-provided symptom input indicating the symptom event and up to 30 minutes after the arrhythmia onset event or symptom input. Alternatively, the arrhythmia or symptom contextual time period may be a first custom amount of time (e.g., 20 minutes) before the arrhythmia event onset or symptom input and a second custom amount of time (e.g., 40 minutes) after the arrhythmia onset event or symptom input.

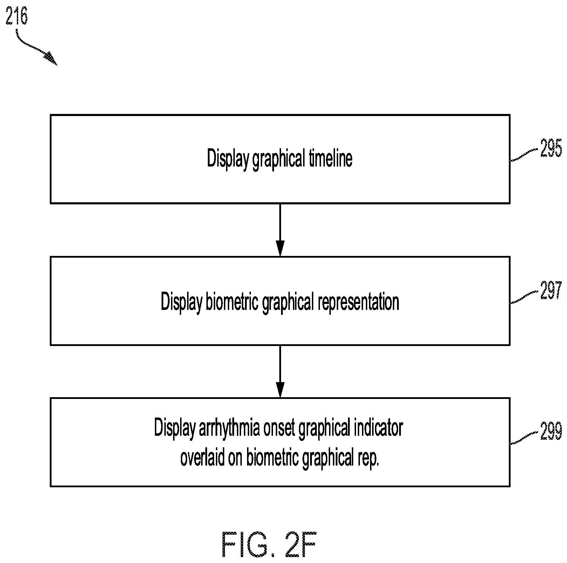

In step 2014, the contextual biometric information during the default or custom arrhythmia or symptom contextual time period is determined based on motion signals. An example of such a subroutine is described in connection with FIG. 2D above. In step 2016, a graphical timeline is displayed, the graphical timeline including a default or custom arrhythmia or symptom contextual time period around the onset of the arrhythmia event or the symptom input. An example of such a subroutine is described in connection with FIG. 2F above.

Subroutine 2020 is initiated if a default value for the arrhythmia or symptom contextual time period is to be calculated. In step 2022, the arrhythmia event or symptom event is assessed to determine the underlying event type. Alternatively or additionally, in step 2024, the biometric status of the patient is assessed. In step 2026, the value of the arrhythmia or symptom contextual time period is determined based on the event type and/or biometric status of the patient.

For example, in steps 2022-2026, if the arrhythmia event type is a pause, then a default value for the arrhythmia contextual time period may be 15 minutes before and 15 minutes after the onset of the pause event. For example, the default arrhythmia contextual time period for a tachycardia event may be 60 minutes before and 30 minutes after the onset of the tachycardia event. For example, the default arrhythmia contextual time period for a bradycardia event may be 90 minutes before and 60 minutes after the onset of the bradycardia event. For example, the default arrhythmia contextual time period for an atrial fibrillation onset event may be 2 hours before and 1 hour after the onset of the atrial fibrillation event. In examples, a user may specify in advance these default values.

Additionally or alternatively, in steps 2024-2026, the default value for the arrhythmia contextual time period may depend on the biometric status of the patient at or around the arrhythmia event onset and/or or symptom input. To illustrate, the remote server 102 may determine that the arrhythmia event onset occurred while the patient was asleep. The remote server 102 may determine whether the patient was still asleep 60 minutes before the arrhythmia event onset. If the patient was awake 60 minutes before the arrhythmia event onset, the remote server 102 may set the arrhythmia contextual time period as 60 minutes before and 30 minutes after the onset of the arrhythmia event. If the patient was asleep 60 minutes before the arrhythmia event onset, the remote server 102 may determine whether the patient was awake 120 minutes before the arrhythmia event onset. If the patient was awake 120 minutes before the onset of the arrhythmia event, the remote server 102 may set the arrhythmia contextual time period as 120 minutes before and 30 minutes after the onset of the arrhythmia event. If the patient was asleep 120 minutes before the arrhythmia event onset, the remote server 102 may set they arrhythmia contextual time period as 180 minutes before and 30 minutes after the onset of the arrhythmia event. The remote server 102 may also determine the last time the patient was awake before the arrhythmia event onset and provide amount of time the patient was asleep before the arrhythmia event onset as part of the arrhythmia report, discussed in further detail below.

As another example, the remote server 102 may determine whether the arrhythmia event onset occurred while the patient was active. If the patient was inactive when the arrhythmia event onset occurred, the remote server 102 may set the arrhythmia contextual time period as 60 minutes before and 30 minutes after the onset of the arrhythmia event. If the patient was active when the arrhythmia event onset occurred, the remote server 102 may determine the time when the patient transitioned from an inactive status to an active status and set the arrhythmia contextual time period as that time until 30 minutes after the arrhythmia event onset.

Alternatively or additionally, in step 2028, depending on the event type, the subroutine 2020 may prompt a user to specify custom value(s) for the arrhythmia or symptom contextual time period in accordance with subroutine 2010. This mandatory custom value feature provides a benefit that ensures care is taken for certain arrhythmias and/or symptom events. For example, a caregiver may prescribe the cardiac monitor to assess a patient suffering from frequent syncopal episodes. As such, it is desirable that, rather than using the default values, the process 2005 prompt the technician user or caregiver user to specify custom value(s) for the syncopal contextual time period to ensure that special attention is given to the preparation of reports relating to syncopes.

Returning to the sample process 200 shown in FIG. 2A, the remote server 102 further determines contextual biometric information for the arrhythmia contextual time period at step 214. An example process flow for step 214 of determining the contextual biometric information for the arrhythmia contextual time period is shown in FIG. 2D.

In the example process flow shown in FIG. 2D, the remote server 102 retrieves the stored motion signals (e.g., from a database of the remote server 102) at step 250. The remote server 102 then filters the motion signals at step 252. The remote server 102 may filter the motion signals to identify different types of posture and/or motion information for the patient. For example, if the motion signals were recorded by an accelerometer, the lower frequency recordings may correspond to respiration of the patient and higher frequency recordings may correspond to activity of the patient. As such, the remote server 102 may apply a low-pass filter to the motion signals to identify respiration signals and may apply a high-pass filter to the motion signals to identify activity signals. As another example, accelerometer signals may include recordings that correspond to movement of the patient but may also include recordings exerted by the Earth's gravity (e.g., as the patient changes position). In such cases, the remote server 102 may measure the amount of acceleration at any given time that is due to gravity and remote that amount of acceleration from the motion signals to separate signals corresponding to the posture of the patient and signals corresponding to motion of the patient. In some implementations, at least some of this filtering may be performed by the motion sensor and associated circuitry. As such, alternatively or additionally, filtering the motion signals may include parsing different signals recorded by the motion sensor. As an example, if the motion signals were recorded by a 3D accelerometer, the accelerometer may have recorded a device tilt signal that corresponds to the patient's posture and a patient movement signal that corresponds to the patient's activity, respiration, etc. In such cases, filtering the motion signals may include identifying the device tilt signals and identifying the patient movement signals.

The remote server 102 then determines various biometric information for the patient based on the filtered signals at step 254. As an illustration, as shown in the example embodiment of FIG. 2D, the remote server 102 may determine posture information at step 254a, activity information at step 254b, respiration information at step 254c, sleep information at step 254d, and so on. For example, with respect to the posture information determined at step 254a, the motion sensor may include a 3D accelerometer with three axes, and the motion signals may capture to posture information for the patient. More specifically, the posture may be calculated by measuring the projected earth gravity vector in each of three axes of the accelerometer. The device tilt may thus be measured as the arcsine of the ratio between the measured acceleration and earth's gravitational factor (g). In some cases, this posture information determination may be made at the remote server 102, which receives, for example, the projected earth gravity vectors from the motion sensor of the cardiac monitoring device 100. In other cases, this determination may be made by the motion sensor of the cardiac monitoring device 100, which transmits motion signals that include a device tilt signal that corresponds to posture information for the patient over time to the remote server 102.

For example, in some implementations, the cardiac monitoring device 100 may include a tri-axial accelerometer sensitive to the gravitational field, where the accelerometer reading samples during the measurement time window may be represented as $x_i$, $y_i$, and $z_i$. For each axis, the remote server 102 may normalize the accelerometer samples by the g (gravity) value. The remote server 102 may then average the accelerometer measurements over the measurement time window, which may be represented as:

$$x_a = mean(x_i)$$

$$y_a = mean(y_i)$$

$$z_a = mean(z_i)$$

If the accelerometer is being worn on the patient's front (e.g., on the patient's chest), the remote server 102 may apply an angle transformation to the averaged accelerometer measurements. This transformation rotates the axis reference from the front location to a side location to bring the data to a common frame of reference (e.g., a common frame of reference with accelerometer data from accelerometers worn on the patient's side). To do this transformation, the remote server 102 defines rotation matrices $ROT_1$ and $ROT_2$ as follows:

$$ROT_1 = \begin{bmatrix} \cos{(\theta)} & -\sin{(\theta)} & 0 \\ \sin{(\theta)} & \cos{(\theta)} & 0 \\ 0 & 0 & 1 \end{bmatrix}$$

$$ROT_2 = \begin{bmatrix} 1 & 0 & 0 \\ 0 & \cos{(\alpha)} & -\sin{(\alpha)} \\ 0 & \sin{(\alpha)} & \cos{(\alpha)} \end{bmatrix}$$

where $\alpha = 24°$ and $\theta = 45°$. The remote server 102 then rotates the acceleration vector as follows:

$$\begin{bmatrix} x_r \\ y_r \\ z_r \end{bmatrix} = \begin{bmatrix} x_a \\ y_a \\ z_a \end{bmatrix} * ROT_1 * ROT_2$$

For accelerometer data normalized to a common frame of reference, the remote server 102 may then calculate pitch and roll using the formulas:

$$pitch = atan2\left(-y_r, \sqrt{x_r^2 + z_r^2}\right)$$

$$roll = atan2\left(z_r, \sqrt{y_r^2 + \mu * x_r^2}\right)$$

where *atan2* is the four quadrant inverse tangent, $\mu = 0.1$, and $\mu * x_r^2$ is used to remove calculation instabilities. Additionally, in the formula above, the pitch angle is the tilt angle from a vertical orientation. This angle is used to determine if the patient is supine, reclined, or upright.

In some embodiments, the remote server 102 determines the posture information by classifying the patient's posture over time into various posture types. The posture types may include, for example, "supine," "reclined," "upright," "standing," "sitting," "resting," "lying on the right side," "lying on the left side," and/or the like. To illustrate, the remote server 102 may either determine or receive from the motion sensor a signal indicating the device tilt of the motion sensor in degrees over time. The remote server 102 may compare the degree of the motion sensor's tilt to various degree thresholds to classify whether a patient's posture at any given time fits under various categories, such as "supine," "reclined," and "upright." For instance, the remote server 102 may determine that the patient was supine where the motion sensor's tilt (e.g., the patient's pitch angle) was less than 30 degrees, determine that the patient was reclined when the motion sensor's tilt was between 30 degrees and 60 degrees, and determine that the patient was upright when the motion sensor's tilt was greater than 60 degrees.

The posture for the patient may also be calibrated, for example, the first time that the patient wears the cardiac monitoring device 100 to determine an upright posture for the patient. As an example, the patient may wear the cardiac monitoring device 100 in a caregiver's office. The caregiver may indicate to the cardiac monitoring device 100, or directly to the remote server 102 via a caregiver interface 116, when the patient is upright versus reclined. The remote server 102 may then set degree thresholds for classifying posture based on this calibration.

As another example, with respect to the activity information determined at step 254b, the motion sensor may include an accelerometer, and the motion signals may capture activity information for the patient. In particular, activity may be measured in accelerometer counts, which are correlated with energy expenditure due to physical activity (e.g., a commonly accepted measure of activity level). Counts may be calculated by taking patient acceleration, removing the component exerted by the Earth's gravity, taking the absolute value, and integrating over time. In some implementations, the determining the activity information for the patient may include determining accelerometer counts for a certain amount of time. For instance, the number of accelerometer counts per second may be determined. As another example, the number of accelerometer counts per second may be determined and multiplied by 60 to find the number of accelerometer counts per minute at any given time for the patient. As another example, these number of accelerometer counts per minute may be averaged over the course of a certain period, such as five minutes, of time to determine an average number of accelerometer counts over the five-minute period. In some cases, the count determination may be made at the remote server 102, which may receive raw or filtered accelerometer signals from the motion sensor. In other cases, this determination may be made by the motion sensor of the cardiac monitoring device 100, which transmits motion signals that include accelerometer counts corresponding to activity information for the patient over time to the remote server 102.

In some embodiments, the remote server 102 to determines the activity information by classifying the patient's activity level over time into various activity level types. The activity level types may include, for example, "active," "non-active," "walking," "low activity," "intermediate activity," "sleeping," "high activity," "patient fall," and/or the like. As an illustration, the remote server 102 may either determine or receive from the motion sensor a signal indicating the accelerometer counts of the motion sensor over time. The remote server 102 may compare the accelerometer counts to various count thresholds to classify whether a patient's activity level at any given time fits under various categories, such as "active" and "non-active." For instance, the remote server 102 may determine that the patient was active when the accelerometer counts per minute were 1000 or more and that the patient was non-active when the accelerometer counts per minute were less than 1000. To illustrate another example, the remote server 102 may determine that the patient was active when the accelerometer counts per second were greater than 15 for at least 30 seconds of a minute time interval and that the patient was non-active the rest of the time.

As another example, the remote server 102 may determine that the patient was in an "active" state for a given minute when the patient showed activity (e.g., accelerometer counts above a predetermined threshold) for at least 30 seconds and had an average posture greater than 35 degrees. Additionally, the remote server 102 may determine that the patient was in an "asleep" state for a given minute when the patient showed activity for less than 12 seconds and had an average posture less than or equal to 35 degrees for that minute. The remote server 102 may finally determine that the patient was in an "inactive" state if the patient had a combination of activity and posture values for a given minute that did not satisfy the active or sleep state thresholds.

Systems and techniques are described for classifying activity and posture information based on "Classification of basic daily movements using a triaxial accelerometer," M. J. Mathie, B. G. Cellar, N. H. Lovell, and A. C. F. Coster, Med. Biol. Eng. Comput., 2004, 42, 679-687. Mathie et al. describes systems and methods for automated classification of human movements using an accelerometry monitoring system. Actual subjects were recruited for the study: "26 normal, healthy subjects (seven female, 19 male; mean age 30.5 years, 4-6.3 years standard deviation)." The results from the study: "sensitivity of every classification exceeded 87%, and the specificity exceeded 94%. The overall accuracy of the system, measured as the number of correct classifications across all levels of the hierarchy, was a sensitivity of 97.7% and a specificity of 98.7% over a data set of 1309 movements." A binary decision tree divides the movements into classes and subclasses at different hierarchical levels. General distinctions between movements are applied in the top levels, and successively more detailed subclassifications were made at the lower levels of the tree. A classifier is used to identify basic movements from the signals obtained from a single, triaxial accelerometer mounted on the patient's body. Initially, the movements are divided into "activity" and "rest." The activities are further classified as "falls," "walking," "transition between postural orientations," and/or other movement. The "postural orientations" during rest were classified as "sitting," "standing" or "lying." The movements were performed in "a set sequence: stand; lie supine; lie left side; lie face down; lie fight side; stand; sit; stand; walk along a level corridor; stand; sit; stand; walk up a flight of stairs; walk down a flight of stairs; stand; sit; stand; walk along a level corridor; and stand." Each of the resting postures was held for 30 s, with the exception of periods of quiet standing between activities, which were held for 10 s.

As noted, "activity and rest were discriminated by integration of the area under the acceleration curves every second, to produce a measure of metabolic energy expenditure, and then comparison of the measured value with a predetermined threshold, if the measured value exceeded the preset threshold, then the subject was classified as engaged in activity. Otherwise, a classification of resting was made." Further, an "upright posture and lying were discriminated using the measured tilt angle of the subject. Lying substates were discriminated using the measured angle of rotation of the subject when lying." Additionally, "[s]tanding and sitting were discriminated using a probability rule-based system that used a range of parameters, including tilt angle, duration over which the subject maintained the posture, measured metabolic energy expenditure and previous and next activities, and determined the probabilities that the subject was sitting and standing. The state with the higher probability was then selected as the actual state of the subject." For determining falls, sit-to-stand, and stand-to-sit transitions the measured signals were compared with signal templates for these movements.

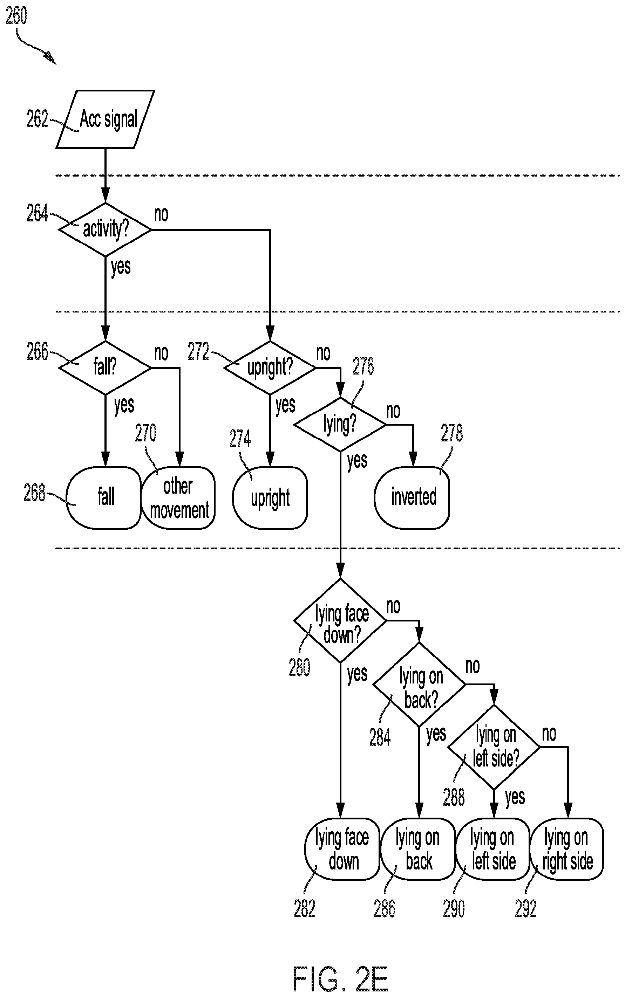

FIG. 2E illustrates a sample process flow for motion classification based on Mathei et al. For example, the process of FIG. 2E can be executed by a processor within the cardiac sensor 110 and/or the portable gateway 106 of FIG. 1, or a processor within the cardiac sensor 110 of FIG. 15. In examples, the raw motion and/or posture data can be transmitted to the remote server 102, where the process of FIG. 2E is executed. Process 260 is used to determine whether a patient has experienced a fall, has collapsed, or has transitioned from an upright, sitting, or walking state to a lying state, as well as posture of the patient after such a fall. Accelerometer signals are received at step 262. The process determines whether the patient is engaged in an activity at step 264. If the patient is engaged in an activity, the process determines whether the patient has experienced a fall at step 266. If the patient has experienced a fall (e.g., based on one or more predetermined classification thresholds), the patient's motion/posture is classified as "fall" at step 268. Alternatively, if the patient has not experienced a fall, the patient's motion/posture is classified as "other movement" at step 270. Otherwise, if the patient is not engaged in activity, whether the patient is upright is determined at step 272. If the patient is upright, the patient's motion/posture is classified as "upright" at step 274.

If the patient is not upright at step 272, the process determines whether the patient is experiencing a lying posture at step 276. If the patient is not experiencing a lying posture, the patient's motion/posture is classified as "inverted" at step 278. Otherwise, if the patient is in a lying posture, the process determines whether the patient is lying face down at step 280. If the patient is lying face down, the patient's motion/posture is classified as "lying face down" at step 282. If the patient was not lying face down, the process determines whether the patient is lying on their back at step 284. If the patient is lying on their back, the patient's motion/posture is classified as "lying on back" at step 286. If the patient is not lying on their back, the process determines whether the patient is lying on their left side at step 288. If the patient is lying on their left side, the patient's motion/posture is classified as "lying on left side" at step 290. Otherwise, if the patient is not lying on their left side, the patient's motion/posture is classified as "lying on right side" at step 292.

In some implementations, the remote server 102 may determine when the patient is walking, for example, based on regular peaks in the motion signals that indicate a regular gait. In some cases, the remote server 102 may determine that the patient is walking by matching the motion signals to a walking baseline recorded for the patient, such as recorded for the patient in a caregiver's office when the patient first wears the cardiac monitoring device 100. The determination of when the patient is walking may be used as part of the biometric context, as described in further detail below.

In implementations, the motion sensor can be disposed in either or both of the cardiac sensor 110 attached to the patch affixed to the patient's upper torso and the portable gateway 106 carried by the patient, e.g., in their pants pocket or clipped to a belt worn about the patient's waist (FIG. 1). In implementations, the motion sensor can be disposed in either or both of the cardiac sensor 110 that is attached via cables to adhesive ECG electrodes 112, or in one or more of the adhesive ECG electrodes 112 (FIG. 15).

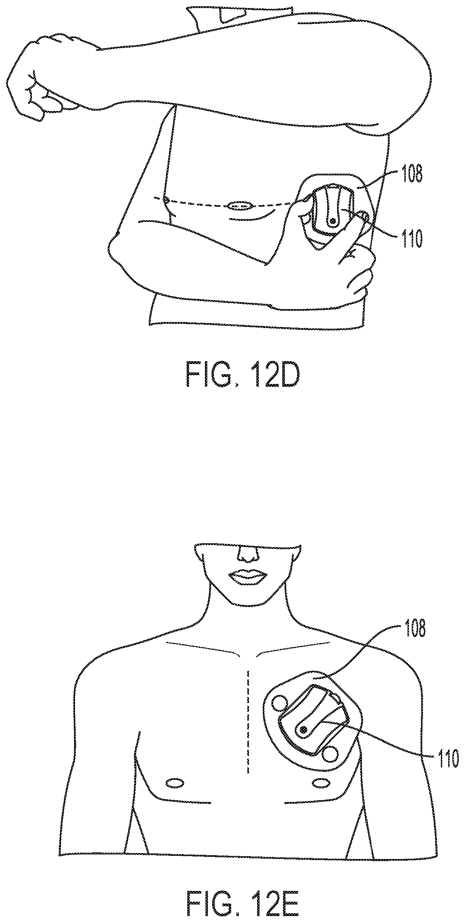

An activity classification is determined based on the motion and/or posture information as described above. Further classification of the activity can be established as follows. For example, the activity can be classified as "walking," "running," "climbing stairs," or "walking down stairs." For this illustration, the motion and/or posture data includes acceleration in three axes. Further, the motion sensor is assumed to be located within a patch-based cardiac sensor 104 and affixed to the upper front portion of the patient's torso (See FIG. 12E). For example, the z-axis data includes forward movement of the patient's upper torso. For example, the y-axis data includes upward and downward motion of the patient. For example, the x-axis data includes lateral movement of the patient's upper torso.

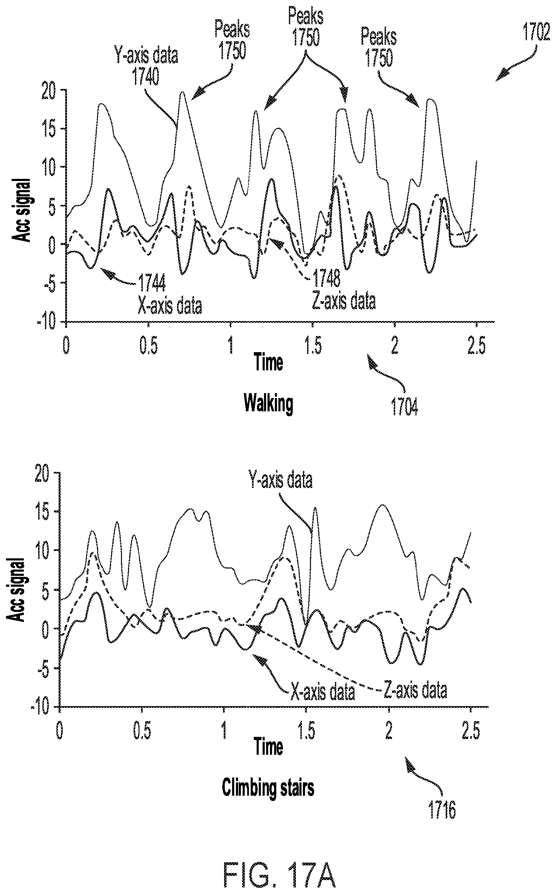

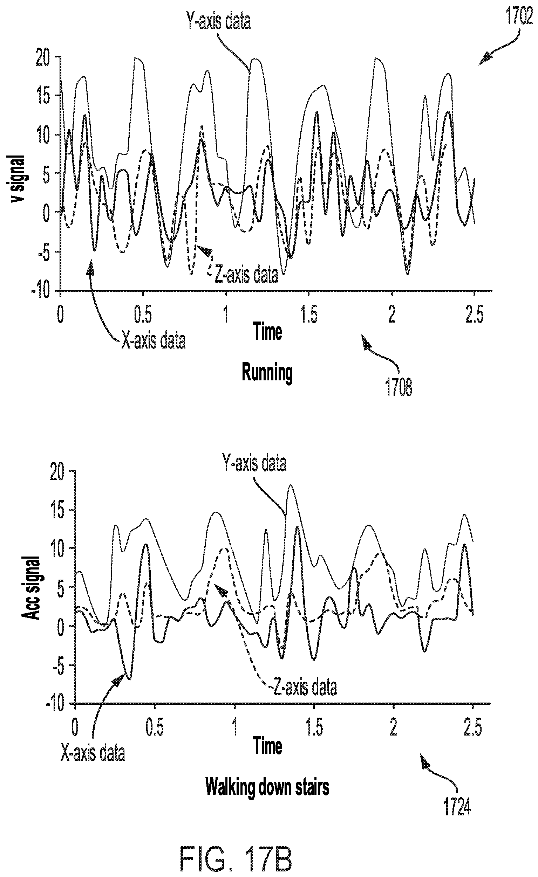

FIGS. 17A and 17B shows example plots 1702 for the accelerometer data. As shown data from the three axes (X, Y, and Z axes) are depicted for each of the four activities "walking" 1704, "running" 1708, "climbing stairs" 1716 or "walking down stairs" 1724. Each of the activities comprises repetitive or periodic movements. For example, the y-axis data 1740 comprises the largest acceleration signals relative to the x-axis data 1744 or the z-axis data 1748, as a result of the gravitational pull, which causes the accelerometer to measure a value of 9.8 m/s2.

The periodic patterns for walking, running, climbing stairs, and walking down stairs includes a plurality of peaks (e.g., peaks 1750 for the walking plot 1704), and durations between the peaks. Further, the plots comprise relative magnitudes of the acceleration values for each of the activities. As an example, the plot for walking 1704 comprises a series of high peaks 1750 for the y-axis, spaced out at approximately 0.5 second intervals. The peaks for the z-axis data have similarly timed peaks but with a lower amplitude. The duration between the peaks of the z-axis data and y-axis data comprises a time taken by the patient to complete a single stride length. The x-axis data comprises an even lower amplitude but also includes peaks that are synchronized with the other axes data. For the running plot 1708, the z-axis and y-axis data have synchronized peaks, but the duration between peaks is less than the duration for walking, around 0.25 seconds. Further, a range of y-axis acceleration values for running is larger than for walking. For climbing down stairs, a series of small peaks in the y-axis data occur every 0.5 seconds. Each small peak comprises a movement down a single step. The z-axis data comprises a negative acceleration, indicating regular movement down each step. The x-axis data comprises a series of small peaks, with acceleration ranging between positive and negative values. For climbing up stairs, a series of regular peaks for the z-axis data and y-axis data can be seen. These peaks are spaced approximately 0.75 second seconds apart. The longer duration implies longer time to take to climb up stairs.

Returning to FIG. 2D, as another example, with respect to the respiration information determined at step 254c, the motion sensor may include an accelerometer, and the motion signals may capture respiration information for the patient. More specifically, after signal filtering (e.g., filtering the motion signals from the accelerometer to isolate low-frequency portions of the signals), the filtered motion signals may be divided into recording intervals. Each recording interval may be checked for regularity characteristic of breathing cycles. This regularity may be marked, for instance, by a combination of requirements regarding signal peak and dip levels and distribution. For example, each recording interval may be compared to one or more breathing cycle templates to identify breathing cycles, and a certain number of breathing cycles may be compared to one another to determine if there is regularity in their signal peaks and dip levels. Once a "regular" stretch is configured, inhalation peaks are detected and counted. Respiration rate is then calculated as the average time interval between consecutive peaks in the regular stretch. In some cases, this respiration rate determination may be made at the remote server 102, which may receive raw or filtered accelerometer signals from the motion sensor. In other cases, this determination may be made by the motion sensor of the cardiac monitoring device 100, which transmits motion signals that include respiration rates for the patient over time to the remote server 102.

Systems and techniques for determining respiration from triaxial accelerometer data are described, based on "Respiratory rate and flow waveform estimation from tri-axial accelerometer data," A. Bates, M. J. Ling, J. Mann, and D. K. Arvind, Sensors, 2016, 16, 750-756. The accelerometer is assumed worn on the chest of the patient. In this case, if the patient is still, then the measured and normalized acceleration vector, $a$, will be close to the acceleration due to gravity, g, because the linear accelerations due to breathing would be relatively small. As noted in Bates et al., "as the accelerometer rotates, the gravity vector will rotate in the co-ordinate frame of the device. The axis of this rotation may be arbitrarily oriented in the device frame, and may change due to differences in the way the patient breathes at different times. It can also change with the orientation of the patient." More specifically, the angle $\theta_t$ and the axis of rotation $r_t$ between consecutive measurements of the acceleration vector at times $t - 1$ and $t$ were determined by dot and cross products as follows:

$$\theta_t = \cos^{-1}(a_t \cdot a_{t-1})$$

$$r_t = a_t \times a_{t-1}$$

Because oscillatory rotation $r_t$ inverts when the direction of rotation reverses, Bates et al. normalized the axis direction by comparison to a reference axis $r_{ref}$ as follows:

$$r_t' \begin{cases} r_t, & r_t \cdot r_{ref} \geq 0 \\ -r_t, & r_t \cdot r_{ref} < 0 \end{cases}$$

The predominant axis of rotation was then identified from measurements over a window of length W. Additionally, to reduce the noise, the estimate was weighted by the angle $\theta_t$ associated with each measurement, and to weight measurements closer in time to $t$, a Hamming window function $H(n)$ was used. This process may be represented as follows:

$$\bar{r}_t = normalize\left(\sum_{i=-W/2}^{W/2} H(i)\theta_{t+i}r_{t+i}'\right)$$

The central point of the present rotations was estimated similarly:

$$\bar{a}_t = normalize\left(\sum_{i=-W/2}^{W/2} a_{t+i}\right)$$

Using these values, the current rotation angle $\phi_t$, measured from the mean direction of gravity, was determined as follows:

$$\phi_t = \sin^{-1}\left((\bar{a}_t \times \bar{r}_t) \cdot a_t\right)$$

The angular velocity on the axis $\omega_t$ was then determined as the derivative of $\phi_t$:

$$\omega_t \frac{d\phi}{dt}$$

The angular rate in radians per second is used to determine the respiration rate. For example, thresholds in the angular rate signals, scaled based on the RMS level of the signal, and dividing the amplitude range of the signal into low, middle and high bands can be determined. Accordingly, identification of a complete breath can be established using a state machine. where the signal transitions through a middle, a high, the middle, a low and then middle bands, in order to register a breath. The instantaneous respiratory rate is then given by the number of such qualifying breaths within a predetermined period of time.

In some embodiments, the remote server 102 determines the respiration information by classifying the patient's respiration over time into various respiration types. The respiration types may include, for example, "regular breathing," "rapid breathing," "shallow breathing," "rapid shallow breathing," "deep breathing," "wheezing," "sleep disordered breathing," "Cheyne-Stokes respiration," and/or the like. As an illustration, the remote server 102 may either determine or receive from the motion sensor a signal indicating the respiration rate of the patient over time. The remote server 102 may compare the respiration rates to various respiration thresholds to classify whether a patient's respiration rate at any time fits under various categories, such as "regular breathing," "shallow breathing," and "deep breathing." For instance, the remote server 102 may determine that the patient was experiencing regular breathing when the patient was breathing between 12 and 20 breaths per minute, that the patient was experiencing shallow breathing when the patient when the patient was breathing over 20 breaths per minute, and that the patient was experiencing deep breathing when the patient was breathing less than 12 breaths per minute. In some cases, the respiration thresholds used to determine which category the patient's respiration rate fits into are determined on a patient-by-patient basis, such as based on a respiration rate baseline for the patient taken by a clinician.

As another example, with respect to the sleep information determined at step 254d, the sleep status for the patient may be determined from a combination of other biometric information of the patient, such as a combination of posture, activity level, and/or respiration of the patient. For instance, the patient may be determined to be asleep when the patient is identified as supine (e.g., at a tilt of less than 35 degrees) and non-active (e.g., showed a non-active accelerometer count for at least 12 seconds of a minute time interval). To illustrate another example, the patient may be determined to be asleep when the patient is identified as supine, non-active, and experiencing deep breathing (e.g., less than 12 breaths per minute) for at least five consecutive minutes.

Other types of biometric information may be determined at step 254, and other processes for determining biometric information may be used at step 254, such as processes for determining biometric information from gyroscopes, magnetometers, ballistocardiographs, ECG, and the like. Further, other types of biometric information may be determined from a combination of other biometric information of the patient. Additional types of biometric information may include, for example, blood pressure, steps taken per day, body temperature, heart rate variability, oxygen saturation, environmental conditions, and/or the like. As such, in some implementations, the remote server 102 may receive other biometric signals from the cardiac monitoring device 100 that the remote server 102 may use to determine the biometric information for the patient.

As an illustration, the remote server 102 may determine heart rate recovery information for the patient by identifying a period of activity (e.g., a certain amount of time, such as five or ten minutes, during which the patient's accelerometer counts showed that the patient was active) and determining the patient's heart rate (e.g., determined from the ECG signals at step 232 of FIG. 2B) at one or more interval following the end of the period of activity. For example, the remote server may determine the patient's heart rate at the cessation of activity and one minute after the cessation of activity, and further determine the difference between the heart rates as the heart rate recovery. As another example, the remote server 102 may determine the patient's heart rate at the cessation of activity and 10, 20, 30, 40, 50, and 60 seconds after the cessation of activity. The remote server 102 may further determine the difference between the heart rates at cessation and each of the 10-second intervals after the cessation of activity as the patient's heart rate recovery.

As another illustration, the remote server 102 may determine, using data from one or more sensors of the cardiac monitoring device 100, environmental conditions for the patient and/or cardiac monitoring device 100 at the time of an arrhythmia. For example, the remote server 102 may determine that an arrhythmia occurred at 8:45 pm, and the remote server 102 may use location data for the patient (e.g., as determined by a GPS sensor on the cardiac monitoring device 100) to further identify environmental data from a separate weather server as the biometric contextual information. Such environmental data can include, for example, outside temperature (e.g., 70°F), relative humidity (e.g., 60%), air quality (e.g., an Air Quality Index value of 25), and/or elevation (e.g., 145 feet) around the time of the arrhythmia. As another example, the remote server 102 may determine that an arrhythmia occurred at 8:45 pm, and the remote server 102 may receive a temperature read from a temperature sensor of the cardiac monitoring device 100 to use as the biometric contextual information around the time of the arrhythmia.

Additionally, in some implementations, the remote server 102 may determine the biometric information for the patient based at least partially on patient inputs regarding the patient's biometric status at various times over the period the motion signals were recorded. For example, a patient may input to the cardiac monitoring device 100 that the patient anticipates a change in the patient's biometric status, such as the patient is going to sleep or that the patient is about to exercise. To illustrate, a patient may interact with a touchscreen of the portable gateway 106, interact with a digital assistant of the portable gateway 106, tap the sensor unit 110, and so on to indicate an upcoming change in the patient's biometric status.

Once biometric information for the patient is determined, the remote server 102 may then determine the biometric information for the arrhythmia contextual time period at step 256. Accordingly, the remote server 102 may identify the arrhythmia contextual time period determined at step 212 of FIGS. 2A and 2C and isolate the biometric information that corresponds to the arrhythmia contextual time period. This isolated biometric information forms the contextual biometric information for the arrhythmia contextual time period. In one example, the remote server 102 has determined that the arrhythmia contextual time period is from 8:00-10:00 am on December 2, and the remote server 102 has determined posture, activity, respiration, and sleep information for the patient. As such, the remote server 102 may identify the portion of posture information, the portion of activity information, the portion of respiration information, and the portion of sleep information that corresponds to 8:00-10:00 am on December 2 as the contextual biometric information. Alternatively, in some cases, depending on the patient information desired for reporting, the remote server 102 may identify a subset of the determined biometric information as the contextual biometric information. Referring to the previous example, the remote server 102 may just isolate the activity and sleep information corresponding to 8:00-10:00 am as the contextual biometric information.

In some implementations, the type and/or depth of biometric information isolated may depend on an aspect of the arrhythmia event, such as the type of the arrhythmia event. For instance, for an atrial fibrillation event, the remote server 102 may determine whether the patient was inactive or active from the motion signals and determine the inactive/active status of the patient over the arrythmia contextual time period. In some cases, the remote server 102 may determine a statistical measure, e.g., an average or median value, for the patient over the arrhythmia contextual time period, such as whether the patient was active or inactive for a majority of the arrhythmia contextual time period, and/or the inactive/active status of the patient at the onset of the atrial fibrillation event. However, for a tachycardia event, the remote server 102 may determine a type of activity the patient was engaged in over the arrhythmia contextual time period. As an illustration, the remote server 102 may determine whether the patient was lying down, sitting, standing, walking, or moving briskly from the motion signals and determine the type of activity of the patient over the arrhythmia contextual time period for the tachycardia event. The remote server 102 may, in some cases, determine an average or median type of activity of the patient over the arrhythmia contextual time period and/or a type of activity the patient was engaged in at the tachycardia event onset.

In some implementations, the remote server 102 may identify the contextual biometric information after further analysis of an arrhythmia. For instance, in some cases, the remote server 102 may identify a series of arrhythmia event onsets and arrhythmia event offsets for the same type of arrhythmia in a relatively short time period (e.g., over 12 hours, over 24 hours, over 48 hours). As such, the remote server 102 may determine that the series of arrhythmia event onsets and arrhythmia event offsets belong to one continuous arrhythmia event spanning from the first arrhythmia event onset to the last arrhythmia event offset. In such cases, the remote server 102 may determine the arrhythmia contextual time period and, subsequently, the contextual biometric information based on the determination of the one continuous arrhythmia, rather than determining arrhythmia contextual time periods and contextual biometric information for each arrhythmia event onset and/or arrhythmia event offset.

Returning to the sample process 200 shown in FIG. 2A, the remote server 102 is configured to display an arrhythmia report for the arrhythmia event at step 216. In some implementations, the remote server 102 is configured to prepare an arrhythmia report and transmit the arrhythmia report to an interface, such as a technician interface 114 or a caregiver interface 116, as a discrete file, report format, or user interface dynamic or still graphics (e.g., in one or more proprietary file formats such as AAC, CDR, PSD, RAR, RTF, DWD, PSD, GIF, PDF, GIF, DOC, DOCX, XLS, XLSX, PPT, PPTX, and HIPAA-compliant medical documentation, among others). The arrhythmia report is then displayed on the technician interface 114 or caregiver interface 116 when the user opens the file. In some implementations, the remote server 102 is configured to provide the arrhythmia report to an interface via an interactive application executing on the interface. For example, the technician interface 114 may execute a technician application configured to receive and display the arrhythmia report via a user interface. The technician may interact with the user interface by, for instance, providing one or more inputs via the user interface, which the technician interface 114 transmits to the remote server 102. The remote server 102 may adjust the arrhythmia report based on the user input(s) and transmit an updated arrhythmia report to the technician interface 114, which the technician application displays via the user interface. As another example, the caregiver interface 116 may execute a caregiver application (e.g., which may or may not be the same as the technician application, as discussed above). The caregiver may interact with the user interface, and the remote server 102 may provide an updated arrhythmia report to the caregiver interface 116 similar to the process described above. Alternatively, in some cases, the caregiver application may provide read-only access to the physician or limited read-only access (e.g., allowing the caregiver limited access to, for instance, navigate through menus and sort arrhythmia reports).

An example process flow for preparing the display of the arrhythmia report as part of step 216 is shown in FIG. 2F. In the example process flow shown in FIG. 2F, the remote server 102 displays a graphical timeline at step 295, where the graphical timeline covers the arrhythmia contextual time period. The remote server 102 also displays a biometric graphical representation at step 297. The remote server 102 may display, for instance, the biometric graphical representation over the graphical timeline. As an illustration, the remote server 102 may plot the contextual biometric information for the arrhythmia contextual time period over the graphical timeline.

For example, if the contextual biometric information for the arrhythmia contextual time period includes posture information for the patient, the biometric graphical representation may include changes in the patient's posture over the arrhythmia contextual time period. In some cases, these posture changes may be displayed in degrees (e.g., as degrees of the device tilt over the arrhythmia contextual time period. In other cases, these posture changes may be displayed as changes in the type of posture over the arrhythmia contextual time period. As such, viewers may be able to discern either or both absolute or relative posture information. For example, absolute posture information includes absolute values of the patient's posture state in degrees, radians, and the like relative to the horizontal lying position or to the vertical upright position. For example, relative posture information includes changes in the patient's posture from an initial reference such as the horizontal lying position or the vertical upright position. To illustrate, the remote server 102 may classify the patient's posture at any given time or over averaged time intervals of the arrhythmia contextual time period into posture type categories, as discussed above with reference to FIG. 2D. These posture type categories may include, for example, "supine," "reclined," "upright," "standing," "sitting," "resting," "lying on the right side," "lying on the left side," and/or the like. The biometric graphical representation may thus chart the posture type of the patient over the arrhythmia contextual time period.

As another example, if the contextual biometric information for the arrhythmia contextual time period includes activity level information for the patient, the biometric graphical representation may include changes in the patient's activity level over the arrhythmia contextual time period. In some cases, these activity level changes may be displayed in accelerometer counts (e.g., accelerometer counts per second, accelerometer counts per minute, average accelerometer counts per minute calculated from the accelerometer counts per second over the minute). In other cases, these activity level changes may be displayed as changes in the types of activity level over the arrhythmia contextual time period. To illustrate, the remote server 102 may classify the patient's activity level at any given time or over averaged time intervals of the arrhythmia contextual time period into activity level type categories, as discussed above with reference to FIG. 2D. These activity level type categories may include, for example, "active," "non-active," "walking," "low activity," "intermediate activity," "high activity," and/or the like. The biometric graphical representation may thus chart the activity level type of the patient over the arrhythmia contextual time period. Alternatively, the biometric graphical representation may chart when the patient was experiencing a certain activity level type, such as when the patient's activity level was classified as active for at least 30 seconds of a 60 second time interval or the number of seconds per minute that the patient's activity level was classified as active. Further, in some implementations, the biometric graphical representation may include changes in heart rate recovery for the patient over the arrhythmia contextual time period. For instance, heart rate recovery may be plotted on the graphical representation for each active period (e.g., each period of at least five minutes where the patient's accelerometer counts showed the patient as active for at least 30 seconds of a 60-second interval).

In some implementations, the biometric graphical representation may display when the patient is walking versus when the patient is at rest or active at a level above walking. Additionally, in some cases, the walking information may be further contextualized with respect to arrhythmia (or, as described below, symptom) information and/or other physiological information of the patient. For example, a biometric graphical representation, or a human-readable sentence summary at the end of a biometric graphical representation, may indicate that the patient input that the patient was dizzy while walking or had low heart rates while walking.

As another example, if the contextual biometric information for the arrhythmia contextual time period includes respiration information for the patient, the biometric graphical representation may include changes in the patient's respiration over the arrhythmia contextual time period. In some cases, these respiration changes may be displayed as respiration rates over time (e.g., average respiration rate for a minute time interval). In other cases, these respiration changes may be displayed as changes in the types of respiration over the arrhythmia contextual time period. To illustrate, the remote server 102 may classify the patient's respiration rate at any given time or over averaged time intervals of the arrhythmia contextual time period into respiration type categories, as discussed above with reference to FIG. 2D. These activity level type categories may include, for example, "regular breathing," "rapid breathing," "shallow breathing," "rapid shallow breathing," "deep breathing," "wheezing," "sleep disordered breathing," "Cheyne-Stokes respiration," and/or the like. The biometric graphical representation may thus chart the respiration type of the patient over the arrhythmia contextual time period.

As another example, if the contextual biometric information for the arrhythmia contextual time period includes sleep status information for the patient, the biometric graphical representation may include changes in the patient's sleep status over the arrhythmia contextual time period. In some cases, these sleep status changes may be displayed as whether the patient was asleep or awake over time.

In some implementations, if the contextual biometric information includes more than one biometric, the biometrics may be charted against separately, though still on the same graphical timeline (e.g., the x-axes of the biometric charts may be aligned). For instance, if the contextual biometric information includes respiration rate, body posture, and activity level, the respiration rate over the arrhythmia contextual time period may be plotted on a first graph encompassing the graphical timeline, the body posture over the arrhythmia contextual time period may be plotted on a second graph encompassing the graphical timeline, and the activity level over the arrhythmia contextual time period may be plotted on a third timeline. In implementations, more than one biometric may be charted on the same graph encompassing the graphical timeline.

In some embodiments, additional or alternative types of biometric information may be used, such as blood pressure, steps taken per day, body temperature, heart rate, heart rate variability, oxygen saturation, environmental conditions, and/or the like. In some embodiments, the biometric graphical representation may also include patient-reported information, such as sleep times reported by the patient, activity times reported by the patient, meals eaten by the patient, symptoms experienced by the patient, and/or the like. Additionally, in some cases, the biometric graphical representation may configured differently from the contextual biometric information plotted or graphed over time, as discussed above. For example, rather than displaying a graphical timeline and a biometric graphical representation, the remote server 102 may determine a summary for each desired biometric over the arrhythmia contextual

The remote server 102 also displays an arrhythmia onset graphical indicator, which is displayed as overlaid on the biometric graphical representation at step 299. The arrhythmia onset graphical indicator corresponds to the onset of the arrhythmia event. In this regard, an onset or other fiducial aspect of the arrhythmia event is graphically presented within a same display area as the contextual biometric information. For example, overlaying the arrhythmia event on the contextual biometric information includes aligning the arrhythmia event information within the contextual biometric information to convey to a viewer one or more of arrhythmia occurrence time, patient state information, and/or whether the patient was undergoing a transition from or to such state (e.g., active, sleeping, etc.). As an illustration, the remote server 102 may display the arrhythmia onset graphical indicator as a line over the graphical representation at the time of the arrhythmia event onset. The line may extend over all of the contextual biometric information shown in the biometric graphical representation such that a user can easily see how the contextual biometric information corresponds to the arrhythmia event onset. The arrhythmia onset graphical indicator may alternatively be formatted differently from a line. As an illustration, points plotting the contextual biometric information may change color or shape after the arrhythmia event onset, the graphical timeline may become a different color after the arrhythmia event onset, and so on.

Additionally, in some cases, the remote server 102 may display one or more additional graphical indicators on the biometric graphical representation, such as an arrhythmia offset graphical indicator corresponding to the offset of the arrhythmia event. The arrhythmia offset graphical indicator may be formatted similarly to the arrhythmia onset graphical indicator, such as by being shown as a second line labelled as the "arrhythmia event offset." Alternatively, the arrhythmia offset graphical indicator may be formatted differently from the arrhythmia onset graphical indicator, such as by changing the color of the graphical timeline at the time of the arrhythmia event offset where the arrhythmia onset graphical indicator was shown as a line marking the arrhythmia event onset on the graphical timeline.

In some implementations, the biometric graphical representation may also or alternatively include other types of formatting to present the contextual biometric information for the arrhythmia event. For instance, the biometric graphical representation may include a color change that indicates portions of the biometric information recorded during the daytime (e.g., between 6 am and 6 pm) versus portions of the biometric information recorded during the nighttime (e.g., between 6 pm and 6 am the next day).

In an example, the remote server 102 executes computer-readable instructions or code (e.g., stored in a database of the remote server 102) that accepts an input of "patientID" corresponding to an ID number for the patient for whom the remote server 102 is preparing an arrhythmia event report and an input of "eventID" for an arrhythmia event detected for the patient. The output is a plot of heart rate, respiration rate, posture pitch, and activity in seconds for the period of 90 minutes prior to the onset of the arrhythmia event and 30 minutes after the onset of the arrhythmia event. Additionally, the code outputs the heart rate, respiration rate, pitch, and activity at the onset of the arrhythmia event. As such, example pseudocode for preparing an arrhythmia event report for a patient may be as follows:

```
            query event database (cm.db.event_info) to get patientID and
    event onset time for eventID
                if eventide does not exist in the cm.db.event_info
                    exit program;
                            warning message indicates that eventID does
    not exist in current database;
                        else if patientID from cm.db.event_info is
    different from input_patientID
                            exit program;
                            warning message indicates that the input of
    eventID and patientID are not correct;
                        else
                            obtain metric_data that includes heart rate,
    respiration rate, posture, and activity for 90 minutes prior and 30
    minutes post the onset of the event;
                            if metric data is empty
                                exit the program;
                                error message indicates that metric
    does doesn't exist in the database;
```

```
                            else
                                 generate figures for heart rate,
   respiration rate, posture, and activity for 90 minutes prior and 30
   minutes post the onset of the event;
                                 get heart rate, respiration rate, and
      posture at the onset of the event and the activity_status of
      active/not active/sleep
                definition of activity_status
                       if activity_time and pitch are all available
                            if activity_time < 30
                                 if activity_time < 12 and pitch <= 35:
      'sleep;'
                                 else: 'not active';
                       if activity_time >= 30
                            if pitch > 35: 'active';
                            else: 'not active'
```

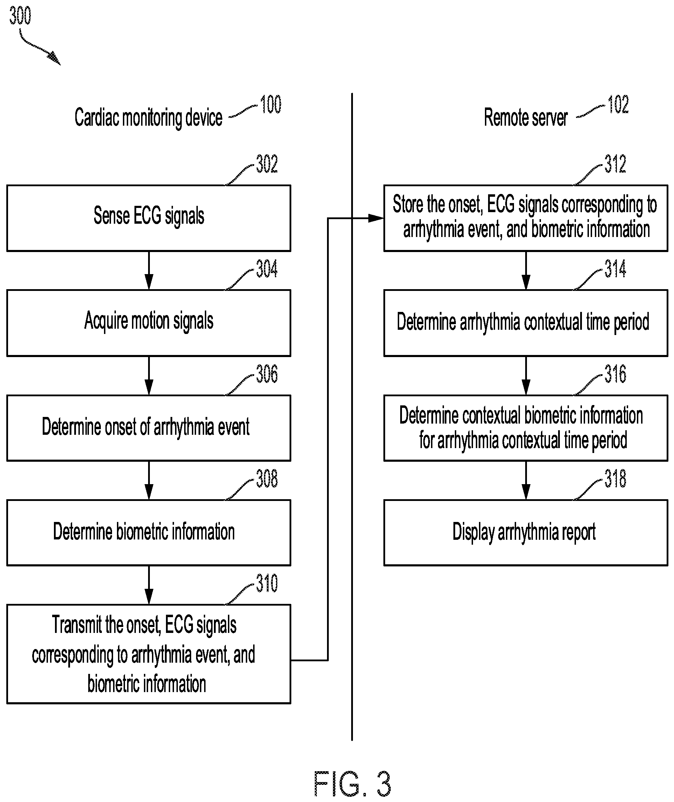

FIG. 3 illustrates another sample process flow for displaying contextual biometric information for arrhythmia events occurring in a patient. For example, the sample process 300 as shown in FIG. 3 can be implemented by the cardiac monitoring device 100 (e.g., a processor of the cardiac sensor 104 and/or a processor of the portable gateway 106) and by the remote server 102 (e.g., a processor of the remote server 102).

As shown in FIG. 3, the cardiac monitoring device 100 senses ECG signals at step 302. The cardiac monitoring device 100 also acquires motion signals via one or more motion sensors incorporated into the cardiac monitoring device 100 at step 304. In various embodiments, the cardiac monitoring device 100 senses the ECG signals and acquires the motion signals according to the processes described above with reference to steps 202 and 204 of FIG. 2A, respectively, discussed above.

The cardiac monitoring device 100 then determines the onset of the arrhythmia event at step 306. For example, the cardiac sensor 104 and/or the portable gateway 106 may determine the onset of the arrhythmia event from the ECG signals similarly to the processes described above with reference to step 210 and FIGS. 2A and 2B. In some implementations, the cardiac monitoring device 100 may also determine the offset of the arrhythmia event from the ECG signals similarly to the processes described above with reference to step 210 of FIGS. 2A and 2B. The cardiac monitoring device 100 also determines biometric information for the patient at step 308. As such, the cardiac sensor 104 and/or the portable gateway 106 may determine the biometric information for the patient similarly to the processes described above with reference to step 214 of FIGS. 2A and 2D. More specifically, the cardiac sensor 104 and/or the portable gateway 106 may determine posture information, activity information, respiration information, sleep information, and/or the like similarly to the processes described above with reference to steps 250-254 of FIG. 2D and, for example, process 260 of FIG. 2E.

The cardiac monitoring device 100 then transmits the arrhythmia event onset, at least the ECG signals corresponding to the arrhythmia event, and the biometric information to the remote server 102 at step 310. The ECG signals corresponding to the arrhythmia event may be, for example, ECG signals including the entire arrhythmia event; ECG signals including the arrhythmia event onset and a certain period of time before the arrhythmia event onset; ECG signals including the arrhythmia event onset, the duration of the arrhythmia event, the arrhythmia event offset, and a certain period of time before and after the arrhythmia event; and so on. In cases where the cardiac monitoring device 100 determines the cardiac event offset, the cardiac monitoring device 100 also transmits the cardiac event offset to the remote server 102.

In some embodiments, the portable gateway 106 transmits the arrhythmia event onset, the ECG signals, and the biometric information to the remote server 102, as described above with reference to step 206 of FIG. 2A. In other embodiments, the cardiac sensor 104 may instead transmit the arrhythmia event onset, the ECG signals, and the biometric information to the remote server 102, as described above with reference to step 206 of FIG. 2A. In some implementations, the cardiac monitoring device 100 may also transmit other signals to the remote server 102, such as ECG signals that do not correspond to the arrhythmia event and/or raw motion signals to the remote server 102.

The remote server 102 stores the arrhythmia event onset, ECG signals corresponding to the arrhythmia event, and biometric information (as well as any other information and/or signals received from the cardiac monitoring device 100) at step 312. For example, the remote server 102 may include one or more databases and store the arrhythmia event onset, ECG signals corresponding to the arrhythmia event, and biometric information in a database of the remote server 102.

The remote server 102 determines the arrhythmia contextual time period at step 314. In various embodiments, the remote server 102 determines the arrhythmia contextual time period according to the processes discussed above with respect to step 212 and with reference to FIGS. 2A and 2C. The remote server 102 also determines the contextual biometric information for the arrhythmia contextual time period at step 316. For example, the remote server 102 may determine the contextual biometric information similarly to the processes discussed above with respect to step 214 and FIGS. 2A and 2D. More specifically, the remote server 102 may determine the contextual biometric information similarly to the processes discussed above with respect to step 256 of FIG. 2D, given that the biometric information for the patient has already been determined by the cardiac monitoring device 100. Finally, the remote server 102 displays an arrhythmia report for the arrhythmia event at step 318. In various embodiments, the remote server 102 displays the arrhythmia report according to the processes discussed above with respect to step 216 and with reference to FIGS. 2A and 2E.

In some embodiments, the cardiac monitoring device 100 and the remote server 102 may implement a process flow that is a combination of the sample processes 200 and 300. For example, the cardiac monitoring device 100 may identify the onset of an arrhythmia event and determine a first portion of biometric information for the patient. The cardiac monitoring device 100 may then transmit the arrhythmia event onset, the ECG signals corresponding to the arrhythmia event, the first portion of biometric information, and motion signals corresponding to a second portion of biometric information to the remote server 102. Subsequently, the remote server 102 may determine the second portion of the biometric information from the received motion signals. Alternatively, the remote server 102 may process the first portion of biometric information to identify the second portion of biometric information. As an illustration, the cardiac monitoring device 100 may transmit patient heart rate information, patient posture information, patient activity level information, and patient respiration information to the remote server 102. The remote server 102 may then use the patient heart rate information and the patient activity level information to determine heart rate recovery information for the patient, and/or one or more arrhythmias such as bradycardia, tachycardia, atrial fibrillation, pauses, atrial flutter, ventricular paced beats (VPB), ectopic beats, and so on. The remote server 102 may further user the patient posture information, patient activity level information, and patient respiration information to determine sleep status information for the patient.

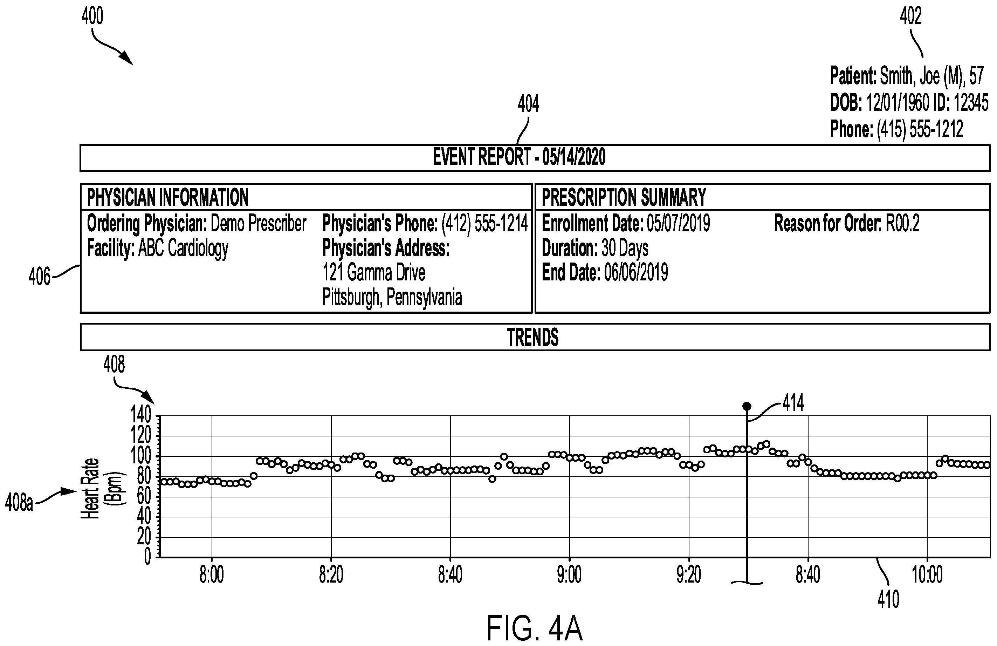

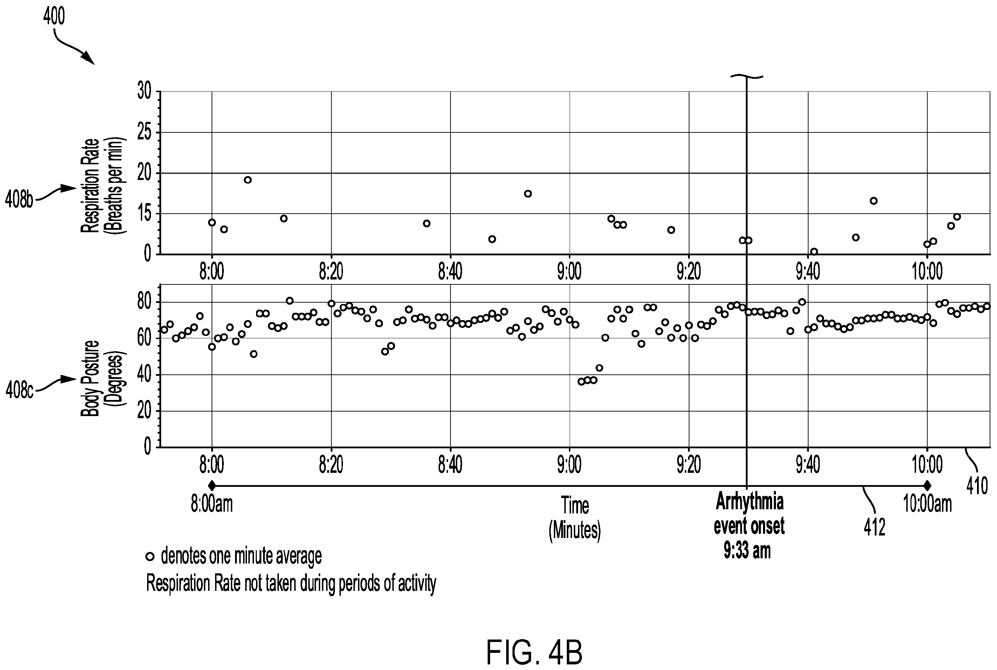

FIGS. 4A and 4B illustrate an example arrhythmia event report 400 (e.g., with FIG. 4A showing a first part of the report 400 and with FIG. 4B showing a second part of the report 400) that may be displayed, for example, on a technician interface 114 and/or a caregiver interface 116. As shown in FIGS. 4A and 4B, an arrhythmia event report 400 may include information 402 that identifies the patient, such as the patient's name, date of birth, identification number, phone number, and/or the like. An arrhythmia event report 400 may also include a header 404 identifying the type of report, as well as subsections 406 identifying other information relating to the patient, the prescribing physician, other caregivers for the patient, the patient's prescription, and/or the like. For instance, as shown in FIGS. 4A and 4B, an arrhythmia event report 400 may include subsections 406 with information on the physician who ordered the cardiac monitoring of the patient, as well as a summary of the patient's prescription (e.g., including the patient's enrollment date, the duration of the cardiac monitoring, the end date for the cardiac monitoring, and a code indicating the reason for the cardiac monitoring).

The arrhythmia event report 400 further includes a biometric graphical representation 408 for an arrhythmia event determined (e.g., by the cardiac monitoring device 100 worn by the patient or the remote server 102) to have occurred in the patient. In the example shown in FIGS. 4A and 4B, the biometric graphical representation 408 includes a representation 408a of the heart rate of the patient (e.g., plotted as beats per minute over time, such the average heart rate for each minute of the arrhythmia contextual time period), a representation 408b of the respiration rate of the patient (e.g., plotted as breaths per minute over time, such as the average respiration rate for each minute of the arrhythmia contextual time period), and a representation 408c of the body posture of the patient (e.g., plotted as degrees over time, such as the average degree of posture for each minute of the arrhythmia contextual time period). As indicated at the bottom of the biometric graphical representation 408, in the example of FIGS. 4A and 4B, each plotted point for the representations 408a, 408b, and 408c of the heart rate, respiration rate, and body posture represent one-minute averages of each of these metrics.

Additionally, as shown in FIGS. 4A and 4B, the representations 408a, 408b, and 408c of the heart rate, respiration rate, and body posture, respectively, are plotted against a graphical timeline 410. As shown, the graphical timeline 410 is the same for all of the representations 408a, 408b, and 408c. Moreover, the graphical timeline 410 includes the arrhythmia contextual time period for the arrhythmia event. For example, as illustrated in FIGS. 4A and 4B, the arrhythmia event onset occurred during a two-hour block 412 shown at the bottom of the biometric graphical representation 408 (e.g., 8:00 am to 10:00 am). The graphical timeline 410 thus includes the two-hour block 412, as well as thirty minutes before and after the two-hour block 412.

In addition to the biometric graphical representation 408, the arrhythmia event report 400 includes an arrhythmia onset graphical indicator 414 that corresponds to the onset of the arrhythmia event. The arrhythmia onset graphical indicator 414 is overlaid on the biometric graphical representation 408. As an illustration, in the example of FIGS. 4A and 4B, the arrhythmia onset occurred at 9:33 am. The arrhythmia onset graphical indicator 414 is thus shown as a line marking 9:33 am overlaid over the biometric graphical representation 408 (e.g., over all of the representations 408a, 408b, and 408c).

Alternatively, in some implementations, a biometric graphical representation 408 may display multiple arrhythmia onset graphical indicators corresponding to multiple arrhythmia events. Each arrhythmia onset graphical indicator may be displayed in a different color, with a different shape, or so on to illustrate that they correspond to different arrhythmia events. In some implementations, a biometric graphical representation 408 may include an arrhythmia offset graphical indicator corresponding to the offset of an arrhythmia event. In some implementations, a biometric graphical representation 408 may include an arrhythmia event duration graphical indicator showing the duration of the arrhythmia event. For example, an arrhythmia duration graphical indicator may be displayed as a line across the biometric graphical representation 408, starting at the time of the arrhythmia event onset and ending at the time of the arrhythmia event offset. In some implementations, a biometric graphical representation 408 may be color-coded to show when the arrhythmia event was occurring versus when no arrhythmia event was occurring. Additional methods for displaying an arrhythmia in the context of biometric information are contemplated herein.

In some implementations, the arrhythmia event report 400 may be shown to a user as illustrated in FIGS. 4A and 4B. For example, the arrhythmia event report 400 may be exported (e.g., as a .pdf file) and transmitted by the remote server 102 to a caregiver interface 116. Alternatively, in some cases, a technician interface 114 may export and transmit the arrhythmia event report 400 to the caregiver interface 116 (e.g., once a technician has finished preparing or modifying the arrhythmia event report 400 through interactions with the remote server 102). As another example, the remote server 102 may communicate with the caregiver interface 116, such as via an application being executed by the caregiver interface 116, and transmit the arrhythmia event report 400 as shown in FIGS. 4A and 4B as part of those communications.

In implementations, the graphical display may present such information in an interactive manner. For example, the user of a technician interface 114 or caregiver interface 116 may be able to interact with the arrhythmia event report 400 and, in some cases, modify the arrhythmia event report 400 based on those interactions. As an illustration, a technician may be able to adjust the arrhythmia contextual time period for the arrhythmia event report 400 and thus the graphical timeline 410 shown on the report. In this way, the technician may be able to increase or decrease the amount of biometric information included in the arrhythmia event report 400.

As another illustration, a user may be able to interact with the arrhythmia event report 400 and, for example, see additional information relating to the arrhythmia event. For instance, a user may be able to interact with the arrhythmia onset graphical indicator 414, such as by clicking on the arrhythmia onset graphical indicator 414. The user's interface (e.g., a technician interface 114 or a caregiver interface 116) transmits the user's action to the remote server 102. For example, such exchange between the remote server and the user's interface may be facilitated via XML and/or JSON data exchange formats. For example, to implement interactivity as described here, the user's actions can on the frontend user interface can be implemented as a form which the user's action (e.g., specific location on display area) as an input and converts it into JSON object using javascript and send it to the server. After clicking the arrhythmia onset graphical indicator 414, a send.JSON() is called which is defined below.

```
<!DOCTYPE html>
<html>
    <body style="center;" id="body">
        <!-object to send user click location to server-!>
        <object onclick="send.JSON"(">Send JSON")</object>
    </body>
</html>
```

When sending data to a web server, the data can be converted into a string using, for example, JSON.stringify() function to convert data to string and send it via XHR request to the server. In implementations, on the remote server, a scripting language such as PHP can be used to respond to the user's request. For example, a file named Object.php, can be implemented to decode the received user click location to JSON and return textual or other human readable information formed using the received data. The server thus to the interface 114 or 116 a portion of the ECG signals corresponding to the arrhythmia event. For example, the portion of the ECG signals corresponding to the arrhythmia event is displayed within a pop-out area that is displayed in response to the user's action. In some cases, the display of the portion of ECG signals corresponding to the arrhythmia event may replace the display of the arrhythmia event report 400 shown in FIGS. 4A and 4B, such as by redirecting the user to another screen. In other cases, the interface 114 or 116 may continue to display the arrhythmia onset graphical indicator 414 overlaid on the biometric graphical representation 408 on a first screen, and display the portion of ECG signals corresponding to the arrhythmia event on a second screen. The first screen may be shown next to the second screen, the first screen may be shown below the second screen, the second screen may be overlaid on top of the first screen, and so on. In examples, one or more human readable sentences may also be included with the displayed portion of the ECG signal. For example, such a textual information may state: "Bradycardia onset event: Normal Sinus Rhythm with PAC, Min HR 16 bpm, Max HR 31 bpm, Avg HR 26 bpm."

In some embodiments, the arrhythmia event report 400 may include additional or alternative information, layouts, formats, and/or graphical representations. For example, in some implementations, the remote server 102 may additionally determine a biometric summary for an arrhythmia event. The biometric summary may include various summary metrics for biometric information that corresponds to the arrhythmia event, such as an average, a median, a minimum, or a maximum of a biometric parameter of the patient over the duration of the arrhythmia event. As an illustration, and with reference to the example arrhythmia event report 400 shown in FIGS. 4A and 4B, a biometric summary may include the minimum heart rate, maximum heart rate, average heart rate, average respiration rate, and average posture for the duration of the arrhythmia event. Additional biometric parameters may be used to determine the biometric summary, in other cases, such as activity level, sleep status, heart rate recovery, and/or the like. The remote server 102 may then include the biometric summary for the arrhythmia event in the arrhythmia event report 400. For example, the biometric summary may be written as one or more human-readable or natural language phrases/sentences that describe the biometric summary for the arrhythmia event. As additional examples, the biometric summary may be shown as a separate chart of the summary metrics for the biometric summary, as one or more biometric summary indicators overlaid on the biometric graphical representation 408, and/or the like.

In some implementations, the remote server 102 may be configured to prepare the arrhythmia event report 400 to display the biometric status (e.g., as part of a biometric summary for the arrhythmia event or separate from a biometric summary for the arrhythmia event) of the patient at the time the arrhythmia event occurred. For example, the remote server 102 may determine, for a given arrhythmia event, whether a patient was active at the time of the arrhythmia event onset, whether the patient was active a certain amount of time before the arrhythmia event onset (e.g., 30 minutes before the arrhythmia event onset), a level to which the patient was active (e.g., whether the patient was standing, walking, moving briskly, etc.), and so on. As another example, the remote server 102 may determine, for a given arrhythmia event, whether a patient was asleep at the time of the arrhythmia event onset, whether the patient was asleep a certain amount of time before or after the arrhythmia event onset, whether the patient appeared to be having trouble sleeping after the arrhythmia event onset, and so on. In some cases, these determinations may be made at least partially based on feedback or input from a technician via a technician interface 114. For instance, a technician may provide biometric parameters (e.g., activity level, sleep status, posture, etc.) to the remote server 102 that the technician wants included in the arrhythmia event report 400. The remote server 102 may accordingly prepare the arrhythmia event report 400 to display the arrhythmia event, along with the biometric status of the patient, such as in a graphical representation described above and/or in a natural language or human-readable phrase/sentence that summarizes or describes the biometric status of the patient for the arrhythmia event.

The remote server 102 may prepare human-readable phrases or sentences for the patient report from a bank of words and/or phrases related to biometric information and arrhythmia events (and, in some cases, symptom events, as discussed in further detail below). The remote server 102 may analyze the contextual biometric information for an arrhythmia event (or symptom event) to determine features to present to a user. For example, the remote server 102 may determine if the patient was active at the arrhythmia event onset, asleep at the arrhythmia event onset, had a normal respiration rate over the arrhythmia event onset, experienced any concurrent symptoms during the arrhythmia event (e.g., as reported by the patient via a patient-provided symptom input, as discussed in further detail below), and so on. The remote server 102 may then use machine-learning processes to determine which words and/or phrases from the bank best describe the features to present to the user and construct natural language phrases or sentences from those words and/or phrases. These machine-learning processes may employ recurrent neural networks, recursive neural networks, bidirectional recurrent neural networks, and the like. Example processes for constructing the natural language descriptions may be similar to those described in "Connecting images and natural language," A. Karpathy, Doctoral Dissertation, Stanford University, 2016. For instance, the remote server 102 may encode the features to present to the user as vectors. The remote server 102 then matches the vectors to the words and/or phrases in the bank based on scores for similarities between the feature vectors and sentence vectors for the words and/or phrases.

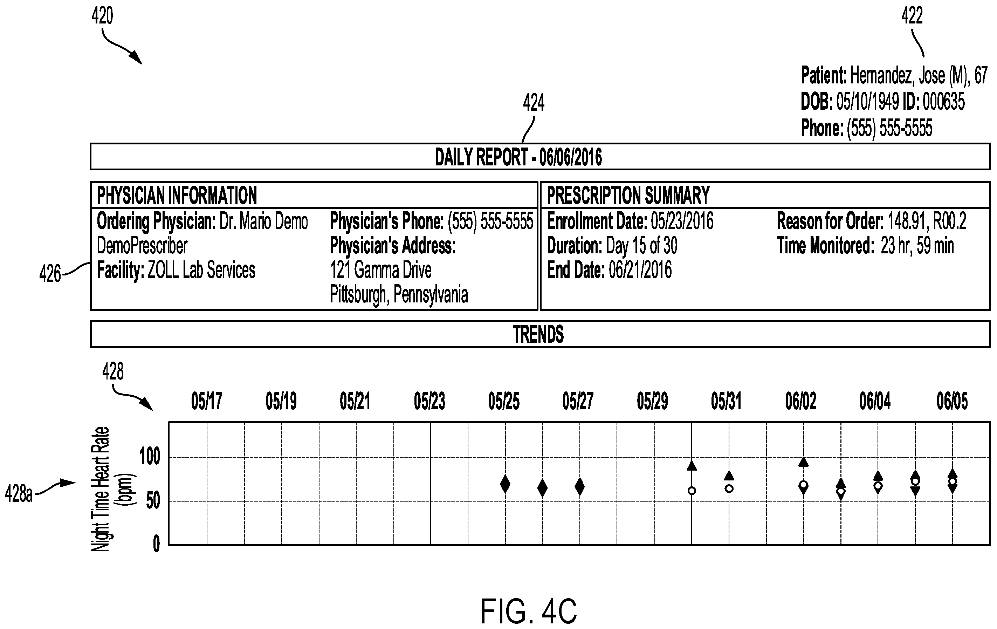

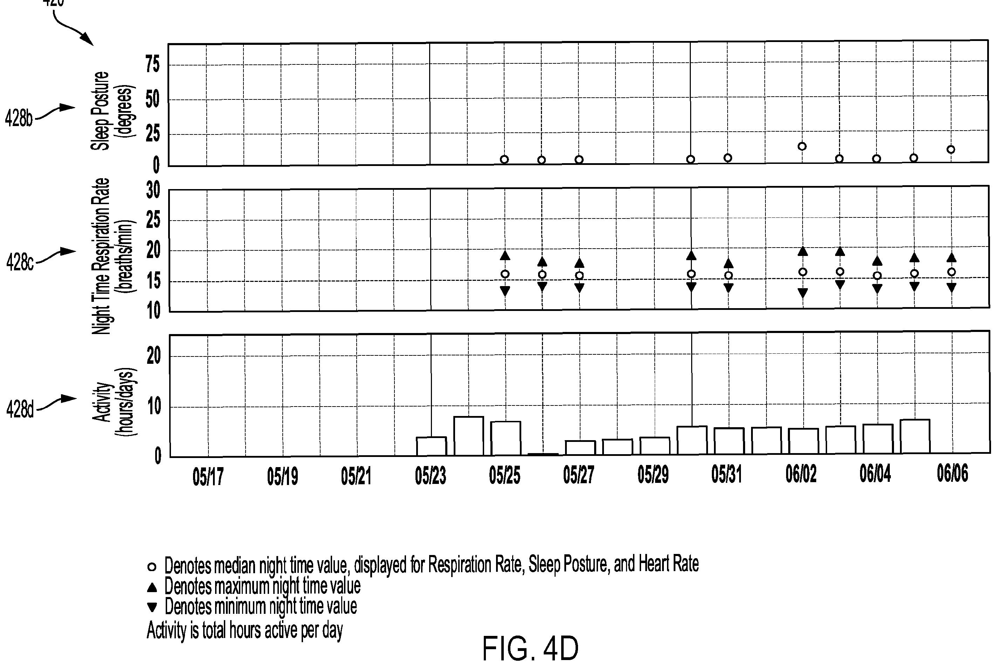

As another illustration of a patient report, FIGS. 4C and 4D illustrates a daily report 420 (e.g., with FIG. 4C showing a first part of the daily report 420 and with FIG. 4D showing a second part of the daily report 420) that may be displayed, for example, on a technician interface 114 and/or a caregiver interface 116. As shown in FIGS. 4C and 4D, and similar to the arrhythmia event report 400, the daily report 420 may include information 422 that identifies the patient, a header 424 identifying the type of report, and subsections 426 identifying other information relating to the patient. The daily report 420 further includes a biometric graphical representation 428 for an arrhythmia event determined to have occurred in the patient. In the example shown in FIGS. 4C and 4D, the biometric graphical representation 428 includes a representation 428a of the night time heart rate of the patient (e.g., plotted the median, maximum, and minimum heart rate per day), a representation 428b of the sleep posture of the patient (e.g., plotted the median, maximum, and minimum degrees per day), a representation 428c of night time respiration rate of the patient (e.g., plotted as the median, maximum, and minimum breaths per minute per day), and a representation 428d of the activity of the patient (e.g., plotted as hours active per day). As such, the daily report 420 provides a caregiver with a biometric summary for the patient over a predetermined period (e.g., since the patient started using the cardiac monitoring device 100). In some implementations, the daily report 420 may be updated each day and transmitted to the caregiver (e.g., transmitted to a caregiver interface 116).In some implementations, the daily report 420 may be a new report generated each day and transmitted to the caregiver, such as a report that summarizes biometric information for the patient for just the day in question.

FIG. 5 illustrates a sample process flow for displaying contextual biometric information for patient-provided symptom inputs. For example, the sample process 500 as shown in FIG. 5 can be implemented by the cardiac monitoring device 100 (e.g., a processor of the cardiac sensor 104 and/or a processor of the portable gateway 106) and by the remote server 102 (e.g., a processor of the remote server 102).

As shown in FIG. 5, the cardiac monitoring device 100 senses ECG signals at step 502. The cardiac monitoring device 100 also acquires motion signals via one or more motion sensors incorporated into the cardiac monitoring device 100 at step 504. In various embodiments, the cardiac monitoring device 100 senses the ECG signals and acquires the motion signals according to the processes described above with reference to steps 202 and 204 of FIG. 2A, discussed above.

The cardiac monitoring device 100 also receives a patient-provided symptom input at step 506. The symptom input is provided by the patient and indicates that the patient is experiencing a symptom that is suspected to be cardiac-related. For example, the cardiac-related symptom may include light-headedness, a racing heart, fatigue, fainting, a fall, chest discomfort, a skipped heartbeat, a shortness of breath, and/or the like.

In some embodiments, the patient may provide the symptom input via the portable gateway 106 of the cardiac monitoring device 100. For example, the portable gateway 106 may include a visual display and a processor for the gateway in communication with the visual display. As such, the portable gateway 106 may be configured to display, on the visual display, a screen that includes a list of potential cardiac-related symptoms to the patient, such as the aforementioned list of example cardiac-related symptoms. The portable gateway 106 may be further configured to receive a selection by the patient of one or more of the displayed potential cardiac-related symptoms. For instance, the visual display may include a touchscreen, where the patient interacts with the touchscreen to select the cardiac-related symptom(s) that the patient is experiencing. As another example, the patient may use an input device of the portable gateway, such as arrow buttons or a tracking pad, to select the cardiac-related symptom(s) that the patient is experiencing. The portable gateway 106 thus records the selected cardiac-related symptom(s) and the time the patient submitted the cardiac-related symptom(s).

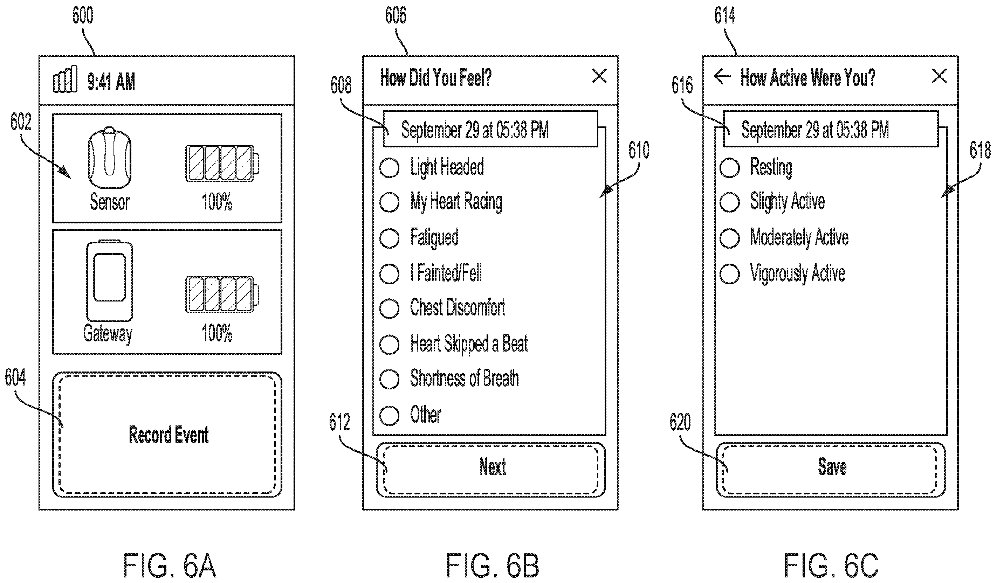

To illustrate, FIGS. 6A-6C illustrate example screens that may be displayed on a visual display of the portable gateway 106 and used by a patient to provide the symptom input. FIG. 6A shows an example home screen 600 that may be displayed by the portable gateway 106, for instance, as a default screen for the portable gateway 106. The home screen 600 may include information on the cardiac monitoring device 100 such as battery level indicators 602 (e.g., for the portable gateway 106 and the sensor unit 110). Additionally, the home screen 600 includes a "Record Event" button 604 that the patient can press if the patient is experiencing a suspected cardiac-related symptom.

In response to the patient selecting the "Record Event" button 604, the portable gateway 106 may display a symptom screen 606, an example of which is shown in FIG. 6B. The symptom screen 606 may include a subsection 608 listing the time and date that the patient is reporting the suspected cardiac-related symptom (e.g., the time and date the patient selected the "Record Event" button 604). In addition, the symptom screen 606 includes a list 610 of potential cardiac-related symptoms. As an illustration, in the example of FIG. 6B, the list 610 of potential cardiac-related symptoms may include "Light-Headed," "My Heart Racing," "Fatigued," "I Fainted/Fell," "Chest Discomfort," "Heart Skipped a Beat," "Shortness of Breath," and "Other" (e.g., in response to the selection of which the patient is shown a screen onto which the patient may type their "other" symptom(s)). The symptom screen 606 may also include a "Next" button 612 that the patient can press once the patient has selected the suspected cardiac-related symptom(s) from the list 610.

In response to the patient selecting the "Next" button 612, the portable gateway 106 may display an activity screen 614, an example of which is shown in FIG. 6C. The activity screen 614 may include a subsection 616 that again lists the time and date that the patient is reporting the suspected cardiac-related symptom. The activity screen 614 also includes a list 618 of types of potential types of activity the patient may have been engaging in when the patient started experiencing the reported symptom(s). As shown in the example of FIG. 6C, the list 618 of potential types of activity may include "Resting," "Slightly Active," "Moderately Active," and "Vigorously Active." The activity screen 614 also may also include a "Next" button 620 that the patient can press once the patient has selected the type of activity the patient was experiencing from the list 618. Once the patient has selected the "Next" button 620, the portable gateway 106 may transmit the selected cardiac-related symptom(s) from the list 610 of potential cardiac-related symptoms, the selected type of activity from the list 618 of potential types of activity, and the time and date of the symptom input.

In implementations, the example screens described above may be displayed on a user interface disposed on the cardiac sensor, e.g., on a housing of the sensor unit 110. The patient may provide the symptom input via the sensor unit 110 of the cardiac monitoring device. For example, the front of the sensor unit 110 (e.g., the face of the sensor unit 110 facing away from the patient when the sensor unit 110 is being worn by the patient) may be configured to receive a patient input. As an illustration, the front of the sensor unit 110 may include a button the patient can tap, or the sensor unit 110 may sense changes in electrical charge on the front surface by the patient tapping on the front surface. The patient can then provide a symptom input to the sensor unit 110 via the front face of the sensor unit 110. An example process for providing a symptom input to the sensor unit 110 may include (1) the patient remaining still, (2) double tapping the front face of the sensor unit 110 with the palm of the patient's hand, and (3) waiting for a beeping sound from the sensor unit 110, which indicates that the symptom input has been recorded. If the beeping sound does not occur, the patient may need to re-tap the sensor unit 110 to record the symptom input.

In some embodiments, the cardiac monitoring device 100 may include a portable gateway 106 configured to receive the symptom input, as well as a sensor unit 110 configured to receive the symptom input. In such embodiments, the patient may be able to provide the symptom input to the cardiac monitoring device 100 via either the portable gateway 106 or the sensor unit 110. For example, in one cardiac monitoring device 100, both the portable gateway 106 and the sensor unit 110 may be configured to receive the symptom input. However, the patient may be encouraged to use the portable gateway 106 to record the symptom input (e.g., because the patient can input more information about the suspected cardiac-related symptom, such as the type of symptom it is and the activity level of the patient when the symptom occurred).

Additionally, in some implementations, the cardiac monitoring device 100 may be configured to receive additional inputs from the patient. For instance, the cardiac monitoring device 100 may be configured to receive a second input from the patient once the patient has stopped experiencing the suspected cardiac-related symptom.

Returning to the sample process 500 shown in FIG. 5, the cardiac monitoring device 100 then transmits the sensed ECG signals, acquired motion signals, and symptom input to the remote server 102 at step 508. In some embodiments, the portable gateway 106 transmits the sensed ECG signals, acquired motion signals, and symptom input to the remote server 102, as described above with reference to step 206 of FIG. 2A. In other embodiments, the cardiac sensor 104 may instead transmit the sensed ECG signals, acquired motion signals, and symptom input to the remote server 102, as described above with reference to step 206 of FIG. 2A.

The remote server 102 stores the ECG signals, motion signals, and symptom input at step 510. For example, the remote server 102 may include one or more databases and store the ECG signals, motion signals, and symptom input in a database of the remote server 102.

The remote server 102 determines the symptom contextual time period at step 512. In various embodiments, the remote server 102 determines the arrhythmia contextual time period similarly to the processes discussed above with respect to step 212 and with reference to FIGS. 2A and 2C, except using the time of the symptom input instead of the arrhythmia event onset. As an example, the remote server 102 may receive, as part of the symptom input, an indication that the patient experienced a suspected cardiac-related symptom at 10:02 am on January 9. The period of time for the symptom input may be a 2-hour block. As such, the remote server 102 may determine that the symptom contextual time period is 10:00-12:00 am on January 9. Alternatively, the period of time for the symptom contextual time period may be 2 hours centered around the time of the symptom input. Thus, the remote server 102 may determine that the symptom contextual time period is 9:02-11:02 am on January 9. As another alternative, the period of time for the symptom contextual time period may be a first amount of time before the symptom input and a second amount of time after the symptom input, such as an hour before the symptom input and 30 minutes after the symptom input. The remote server 102 may accordingly determine that the symptom contextual time period is 9:02-10:32 am on January 9. As another alternative, the period of time for the symptom contextual time period may be an amount of time that ends at the symptom input, such as 15 minutes before the symptom input. Therefore, the remote server 102 may determine that the symptom contextual time period is 9:47-10:02 am on January 9. As another alternative, the period of time for the symptom contextual time period may be an amount of time that begins the symptom event onset, such as 30 minutes after the symptom event onset. In such cases, the remote server 102 may determine that the symptom contextual time period is 10:02-10:32 am on January 9. Additional details regarding the determination of the symptom contextual time period are provided described in connection with FIG. 2G above.

The remote server 102 also determines contextual biometric information for the symptom contextual time period at step 514. Finally, the remote server 102 displays a symptom report for the symptom event at step 516. In various embodiments, the remote server 102 determines the contextual biometric information for the symptom input similarly to the processes discussed above with respect to step 212 and with reference to FIGS. 2A and 2C. The remote server 102 also displays the symptom report similarly to the processes discussed above with respect to step 216 and with reference to FIGS. 2A and 2E.

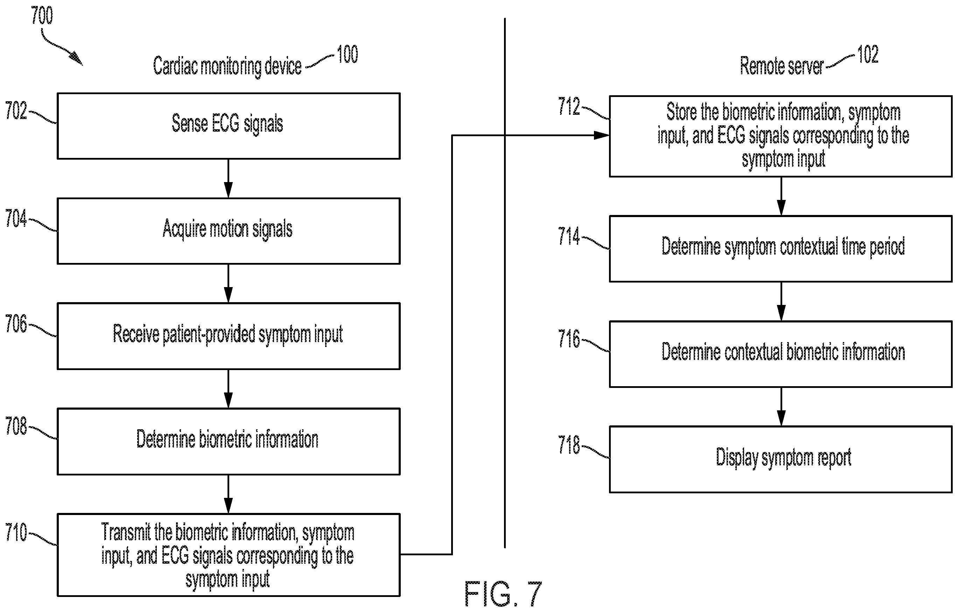

FIG. 7 illustrates another sample process flow for displaying contextual biometric information for patient-provided symptom inputs. For example, the sample process 700 as shown in FIG. 7 can be implemented by the cardiac monitoring device 100 (e.g., a processor of the cardiac sensor 104 and/or a processor of the portable gateway 106) and by the remote server 102 (e.g., a processor of the remote server 102).

As shown in FIG. 7, the cardiac monitoring device 100 senses ECG signals at step 702. The cardiac monitoring device 100 also acquires motion signals via one or more motion sensors incorporated into the cardiac monitoring device 100 at step 704. In various embodiments, the cardiac monitoring device 100 senses the ECG signals and acquires the motion signals according to the processes described above with reference to steps 202 and 204 of FIG. 2A, respectively, and with reference to steps 502 and 504 of FIG. 5, respectively, discussed above. The cardiac monitoring device 100 further receives a patient-provided symptom input indicating that the patient is a suspected cardiac-related symptom at step 706. In various embodiments, the cardiac monitoring device 100 receives the patient-provided symptom input according to the processes described above with reference to steps 506 of FIG. 5 (as well as, in some implementations, using screens 600, 606, and 614 of FIGS. 6A-6C), discussed above.

The cardiac monitoring device 100 determines biometric information for the patient at step 708. As such, the cardiac sensor 104 and/or the portable gateway 106 may determine the biometric information for the patient similarly to the processes described above with reference to step 214 of FIGS. 2A and 2D. More specifically, the cardiac sensor 104 and/or the portable gateway 106 may determine posture information, activity information, respiration information, sleep information, and/or the like similarly to the processes described above with reference to steps 250-254 of FIG. 2D.

The cardiac monitoring device 100 then transmits the biometric information, the symptom input, and at least the ECG signals corresponding to the symptom input to the remote server 102 at step 710. The ECG signals corresponding to the symptom input may be, for example, ECG signals including a certain period of time before and/or after the symptom input. In some embodiments, the portable gateway 106 transmits the biometric information, the ECG signals, and the biometric information to the remote server 102, as described above with reference to step 206 of FIG. 2A. In other embodiments, the cardiac sensor 104 may instead transmit the biometric information, the ECG signals, and the biometric information to the remote server 102, as described above with reference to step 206 of FIG. 2A. In some implementations, the cardiac monitoring device 100 may also transmit other signals to the remote server 102, such as ECG signals that do not correspond to the symptom input and/or raw motion signals to the remote server 102.

The remote server 102 stores the biometric information, symptom input, and ECG signals corresponding to the symptom input (as well as any other information and/or signals received from the cardiac monitoring device 100) at step 712. For example, the remote server 102 may include one or more databases and store the biometric information, symptom input, and ECG signals corresponding to the symptom input in a database of the remote server 102.

The remote server 102 determines the symptom contextual time period in step 714. In various embodiments, the remote server 102 determines the symptom contextual time period according to the processes discussed above with respect to step 512 and with reference to FIG. 5 (which, in turn, references step 212 and FIGS. 2A and 2C). The remote server 102 also determines the contextual biometric information for the symptom input at step 716. For example, the remote server 102 may determine the contextual biometric information similarly to the processes discussed above with respect to step 514 and FIG. 5 (which, in turn, references step 214 and FIGS. 2A and 2D). More specifically, the remote server 102 may determine the contextual biometric information similarly to the processes discussed above with respect to step 256 of FIG. 2D, given that the biometric information for the patient has already been determined by the cardiac monitoring device 100. Finally, the remote server 102 displays a symptom report for the symptom input at step 718. In various embodiments, the remote server 102 displays the symptom input according to the processes discussed above with respect to step 516 and FIG. 5 and with reference to step 216 and FIGS. 2A and 2E.

In some embodiments, the cardiac monitoring device 100 and the remote server 102 may implement a process flow that is a combination of the sample processes 500 and 700. For example, the cardiac monitoring device 100 may determine a first portion of biometric information for the patient. The cardiac monitoring device 100 may then transmit the symptom input, the ECG signals corresponding to the symptom input, the first portion of biometric information, and motion signals corresponding to a second portion of biometric information to the remote server 102. Subsequently, the remote server 102 may determine the second portion of the biometric information from the received motion signals. Alternatively, the remote server 102 may process the first portion of biometric information to identify the second portion of the biometric information. As an illustration, the cardiac monitoring device 100 may transmit patient heart rate information, patient posture information, patient activity level information, and patient respiration information to the remote server 102. The remote server 102 may then use the patient heart rate information and the patient activity level information to determine heart rate recovery information for the patient. The remote server 102 may further user the patient posture information, patient activity level information, and patient respiration information to determine sleep status information for the patient.

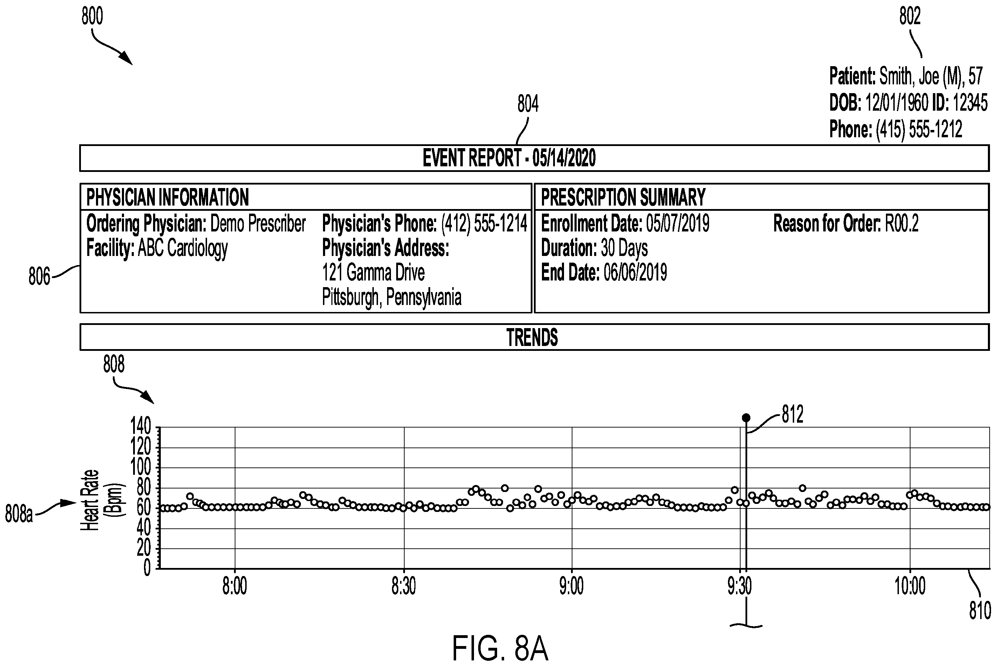

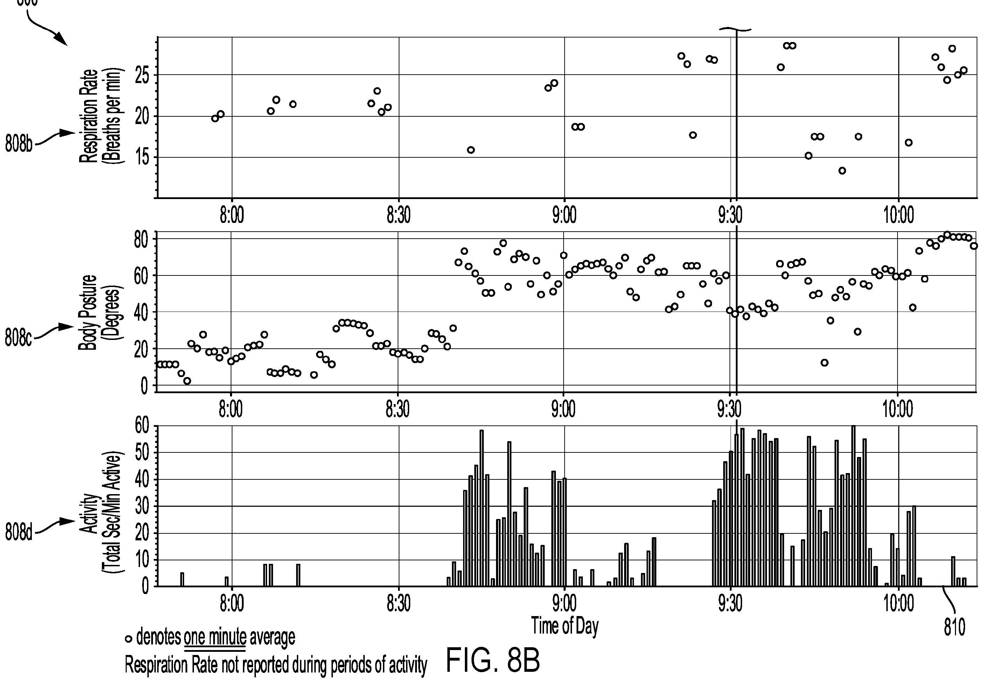

FIGS. 8A and 8B illustrate an example symptom report 800 (e.g., with FIG. 8A showing a first part of the symptom report 800 and FIG. 8B showing a second part of the symptom report 800) that may be displayed, for example, on a technician interface 114 and/or a caregiver interface 116. As shown in FIGS. 8A and 8B, and similar to the example arrhythmia event report 400 shown in FIGS. 4A and 4B, the symptom report 800 may include information 802 that identifies the patient, such as the patient's name, date of birth, identification number, phone number, and/or the like. Further similar to the example arrhythmia event report 400, a symptom report 800 may also include a header 804 identifying the type of report, as well as subsections 806 identifying other information relating to the patient, the prescribing physician, other caregivers for the patient, the patient's prescription, and/or the like. For instance, as shown in FIGS. 8A and 8B, a symptom report 800 may include subsections 806 with information on the physician who ordered the cardiac monitoring of the patient, as well as a summary of the patient's prescription (e.g., including the patient's enrollment date, the duration of the cardiac monitoring, the end date for the cardiac monitoring, and a code indicating the reason for the cardiac monitoring).

The symptom report 800 further includes a biometric graphical representation 808 for a patient-provided symptom input. In the example shown in FIGS. 8A and 8B, the biometric graphical representation 808 includes a representation 808a of the heart rate of the patient (e.g., plotted as beats per minutes over time), a representation 808b of the respiration rate of the patient (e.g., plotted as breaths per minute over time), a representation 808c of the body posture of the patient (e.g., plotted as degrees over time), and a representation 808d of the activity of the patient (e.g., plotted as a bar graph of total seconds active per minute over time). As indicated at the bottom of the biometric graphical representation 808, in the example of FIGS. 8A and 8B, each plotted point for the representations 808a, 808b, 808c, and 808d of the heart rate, respiration rate, body posture, and activity, respectively, represent one-minute averages of each of these metrics.

Additionally, as shown in FIGS. 8A and 8B, the representations 808a, 808b, 808c, and 808d of the heart rate, respiration rate, body posture, and activity, respectively, are plotted against a graphical timeline 810. As shown, and similar to the graphical timeline 410 of the example arrhythmia event report 400, the graphical timeline 810 is the same for all of the representations 808a, 808b, 808c, and 808d. The graphical timeline 810 also includes the symptom contextual time period for the symptom input. For example, the patient provided the symptom input at 9:31 am. The graphical timeline 810 thus includes a period from 7:30 am before the symptom input to 10:30 am after the symptom input.

In addition to the biometric graphical representation 808, the symptom report 800 includes a symptom graphical indicator 812 that corresponds to the patient-provided symptom input. Similar to the arrhythmia onset graphical indicator 414 of the example arrhythmia event report 400, the symptom graphical indicator 812 is overlaid on the biometric graphical representation 808. As an illustration, in the example of FIGS. 8A and 8B, the patient provided the symptom input at 9:31 am. The symptom graphical indicator 812 is thus shown as a line marking 9:31 am overlaid over the biometric graphical representation 808 (e.g., over all of the representations 808a, 808b, 808c, and 808d).

Similar to the arrhythmia event report 400 shown in FIGS. 4A and 4B, in some implementations, the symptom report 800 may be shown to a user as illustrated in FIGS. 8A and 8B. For example, the symptom report 800 may be exported (e.g., as a .pdf file) and transmitted by the remote server 102 or a technician interface 114 to a caregiver interface 116. As another example, the remote server 102 may communicate with a caregiver interface 116 via an application being executed on the caregiver interface 116 and transmit the symptom report 800 to the caregiver interface 116 as part of those communications. In implementations, the graphical display may present such information in an interactive manner. For example, the user of a technician interface 114 or caregiver interface 116 may be able to interact with the symptom report 800 similarly to the interactions with the arrhythmia event report 400 discussed above. For instance, a user may be able to interact with the symptom graphical indicator 812, in response to which a portion of the ECG signals corresponding to the symptom input are displayed to the user.

In some embodiments, the symptom report 800 may include additional or alternative information, layouts, formats, and/or graphical representations. For example, in some representations, the remote server 102 may additionally determine a biometric summary for the symptom input. The biometric summary may include various summary metric for biometric information that corresponds to the symptom input, such as an average, a median, a minimum, or a maximum of a biometric parameter of the patient over a time interval corresponding to the symptom input. This time interval may be a default time interval (e.g., two hours following the symptom input, four hours centered around the symptom input), or the time interval may be a user-specified time interval (e.g., set by a technician reviewing the symptom report 800). In some cases, the time interval may also depend on the types of symptoms reported by the patient. For instance, the time interval may be longer for patient-reported fatigue than for patient-reported shortness of breath. Similar to the biometric summary discussed with reference to the arrhythmia event report 400 of FIGS. 4A and 4B, the biometric summary for the symptom report 800 may include summary metrics for heart rate, respiration rate, posture, activity level, sleep status, heart rate recovery, and/or the like. The remote server 102 may also include the biometric summary for the symptom input as part of the symptom report 800. For example, the biometric summary may be written as one or more human-readable sentences, as a separate chart of the summary metrics for the biometric summary, as one or more biometric summary indicators overlaid on the biometric graphical representation 808, and/or the like.

In some implementations, the remote server 102 may be configured to prepare the symptom report 800 to display the biometric status of the patient at the time of the patient-provided symptom input. For example, the remote server 102 may determine, for a given symptom input, what the patient's respiration rate was at the time of the symptom input, what the patient's respiration rate was a certain amount of time before the symptom input (e.g., an hour before the symptom input), what the patient's respiration rate was a certain amount of time after the symptom input, and so on. In some cases, these determinations may be made at least partially based on feedback or input from a technician via a technician interface 114. The remote server 102 may accordingly prepare the symptom report 800 to display the symptom input, along with the biometric status of the patient, such as in a graphical representation described above and/or in a human-readable sentence that summarizes the biometric status of the patient for the arrhythmia event. As noted above, for example, such sentences may state: "Patient-triggered event: Normal Sinus Rhythm with PAC occurred during activity. Min HR 77 bpm, Max HR 134 bpm, Avg HR 92 bpm, Average respiration rate was > 20 breaths / min, Body position was upright."

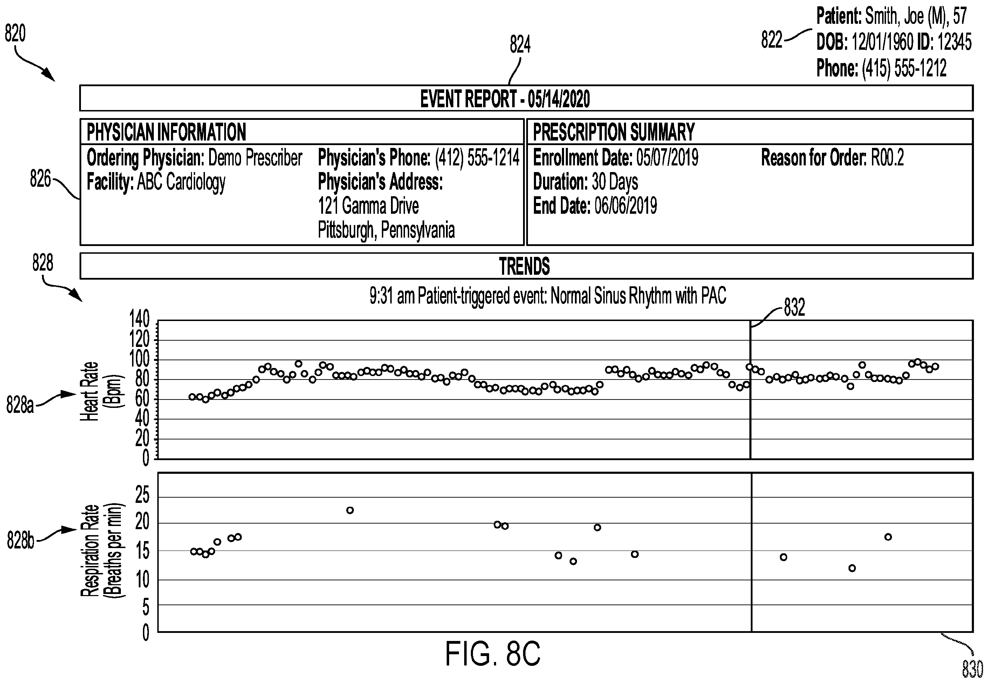

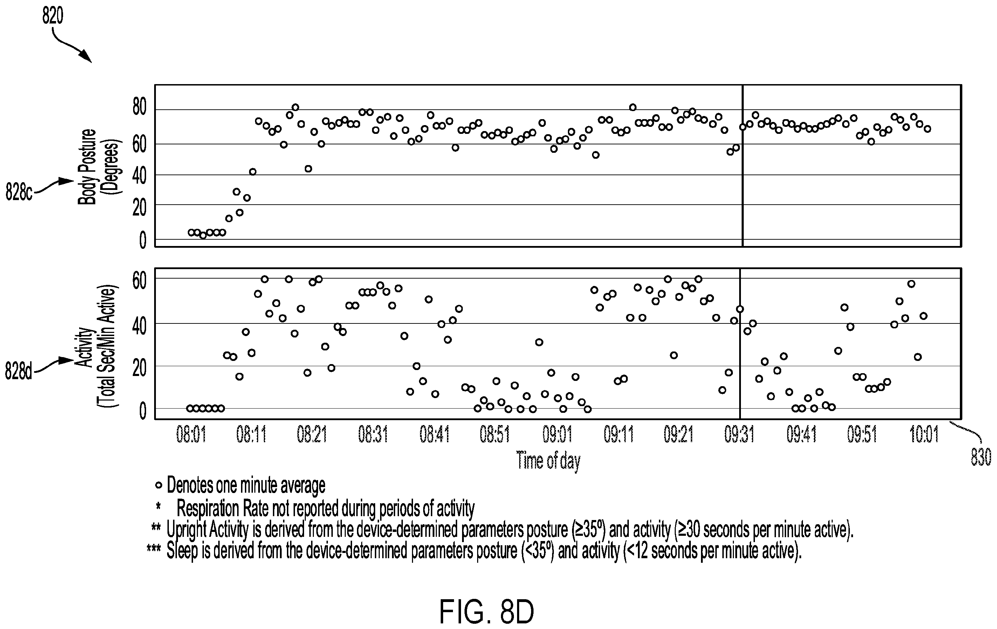

FIGS. 8C and 8D illustrates another example symptom report 820 (e.g., with FIG. 8C showing a first part of the symptom report 820 and with FIG. 8D showing a second part of the symptom report 820) that may be displayed, for example, on a technician interface 114 and/or a caregiver interface 116. As shown in FIGS. 8C and 8D, and similar to the symptom report 800 shown in FIGS. 8C and 8D, the symptom report 820 may include information 822 that identifies the patient, a header 824 identifying the type of report, and subsections 826 identifying other information relating to the patient, etc. The symptom report 820 further includes a biometric graphical representation 828 for a patient-provided symptom input. In the example shown in FIGS. 8C and 8D, and similar to the symptom report 800, the biometric graphical representation 828 includes a representation 828a of the heart rate of the patient, a representation 828b of the respiration rate of the patient, a representation 828c of the body posture of the patient, and a representation 828d of the activity of the patient. The representations 828a, 828b, 828c, and 828d are plotted against a graphical timeline 830 that is the same for all of the representations 828a, 828b, 828c, and 828d and includes the symptom contextual time period for the symptom input. In addition, the symptom report 820 includes a symptom graphical indicator 832. In the example of FIGS. 8C and 8D, the symptom graphical indicator 832 is overlaid on the biometric graphical representation 828, with the symptom graphical indicator 832 separately overlaid on each of the representations 828a, 828b, 828c, and 828d.

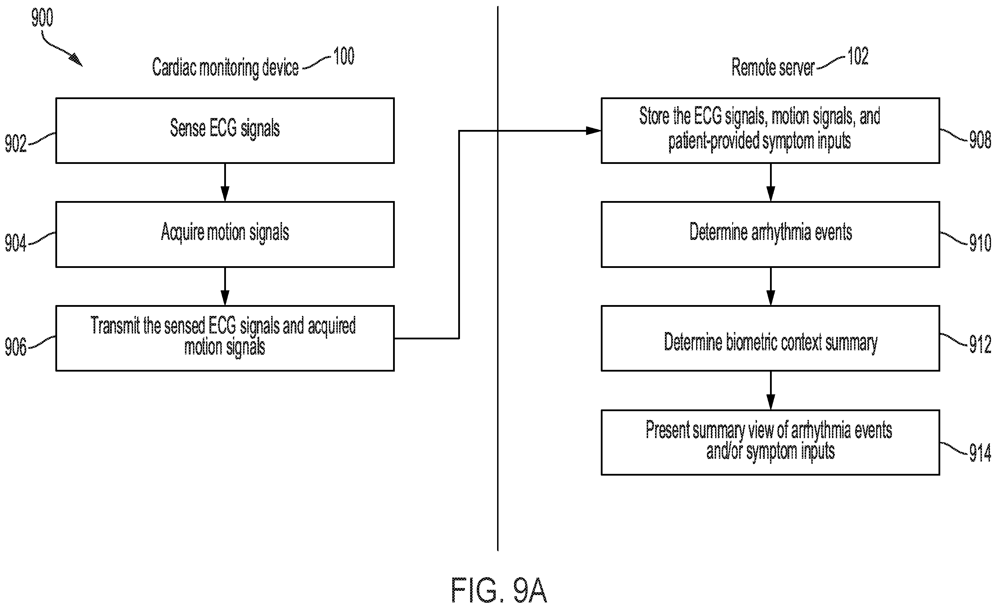

FIG. 9A illustrates a sample process flow for displaying graphical summary views of arrhythmia events occurring in a patient and/or patient-provided symptom inputs over predetermined use periods. For example, the sample process 900 shown in FIG. 9A can be implemented by the cardiac monitoring device 100 (e.g., a processor of the cardiac sensor 104 and/or a processor of the portable gateway 106) and by the remote server 102 (e.g., a processor of the remote server 102).

As shown in FIG. 9A, the cardiac monitoring device 100 senses ECG signals at step 902. The cardiac monitoring device 100 also acquires motion signals via one or more sensors incorporated into the cardiac monitoring device 100 at step 904. The cardiac monitoring device 100 transmits the sensed ECG signals and acquired motion signals at step 906. In various embodiments, the cardiac monitoring device 100 senses the ECG signals, acquires the motion signals, and transmits the sensed ECG signals and acquired motion signals according to the processes described above with reference to steps 202, 204, and 206 of FIG. 2A, discussed above.

The remote server 102 stores the ECG signals, the motion signals, and patient-provided symptom inputs at step 908. For example, the remote server 102 may include one or more databases and store the ECG signals, motion signals, and symptom inputs in a database of the remote server 102. In some implementations, the symptom inputs are patient-provided, such as patient-provided to the cardiac monitoring device 100 as described above with reference to step 506 of FIG. 5. Alternatively, the symptom inputs may be manually provided to the remote server 102 by the patient or a caregiver of the patient. As an example, the patient may record symptoms and information related to the symptoms (e.g., time/date of the symptom, activity level of the patient when the patient started experiencing the symptom, etc.) in a patient diary. The patient or a caregiver may then enter the patient symptoms (e.g., via the portable gateway 106, via a caregiver interface 116), which are transmitted to the remote server 102. In some implementations, the symptom inputs may be generated by a device, such as the cardiac monitoring device 100. For instance, the cardiac sensor 104 may be configured to determine a respiration rate of the patient and provide a symptom input to the remote server 102 when the patient is experiencing shortness of breath (e.g., the patient is breathing more than 20 times per minute while non-active).

The remote server 102 determines arrhythmia events for a predetermined use period from the ECG signals at step 910. In various embodiments, the remote server 102 may determine arrhythmia events using processes similar to the processes described above with reference to step 210 of FIG. 2A. More specifically, the remote server 102 may implement steps 230-236 of FIG. 2B to identify arrhythmia events from the ECG signals. In some implementations, the remote server 102 may also determine the burden of the arrhythmia events. For example, the remote server 102 may calculate the burden of arrhythmia events as the time within arrhythmia events divided by the total valid ECG signal time for the predetermined use period. The burden of arrhythmia events may also be determined based on the type of arrhythmia event. As an example, the remote server 102 may calculate the burden of ectopic beats as the number of ectopic beats divided by the number of normal beats for the predetermined use period.

In some implementations, the predetermined use period may be a default use period for the cardiac monitoring device 100. For example, the predetermined use period may be 24 hours, 48 hours, 72 hours, a week, two weeks, 30 days, a month, the entire duration that the patient is wearing the cardiac monitoring device 100, and/or the like. In some implementations, the predetermined use period may be user-specified, such as by a technician via a technician interface 114 or a caregiver via a caregiver interface 116. For instance, a technician may specify the predetermined use period by selecting a default use period (e.g., from a list displayed to the technician). Alternatively, the technician may enter in a custom use period, such as a custom use period not included in the list of default use periods.

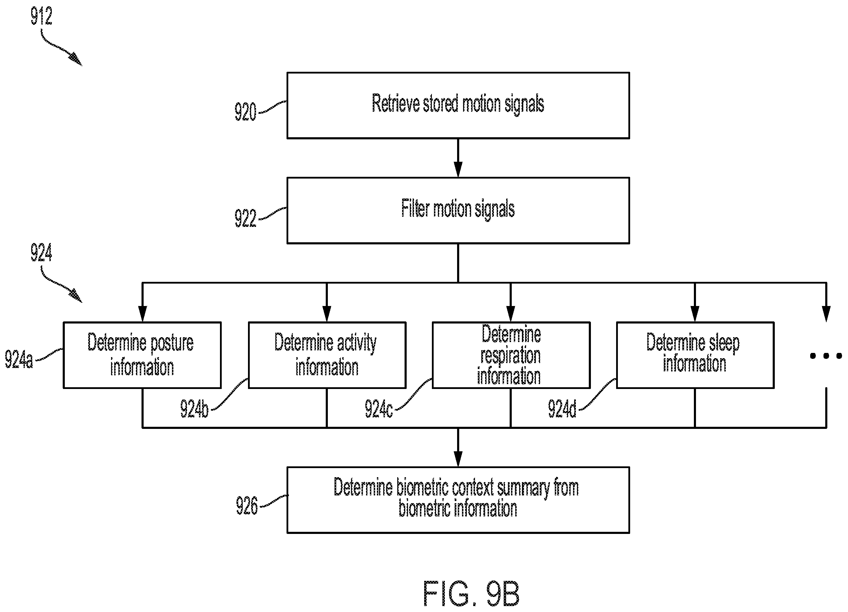

The remote server 102 also determines a biometric context summary for the predetermined use period at step 912. An example process flow for step 912 of determining the biometric context summary for the predetermined use period is shown in FIG. 9B. In the example process flow shown in FIG. 9B, the remote server 102 retrieves the stored motion signals (e.g., from a database of the remote server 102) at step 920. The remote server 102 then filters the motion signals at step 922. In various embodiments, the remote server 102 may filter the motion signals according to the processes discussed above with reference to step 252 of FIG. 2D. The remote server 102 determines various biometric information for the patient based on the filtered signals at step 924. As an illustration, as shown in the example embodiment of FIG. 9B, the remote server 102 may determine posture information at step 924a, activity information at step 924b, respiration information at step 924c, sleep information at step 924d, and so on. In various embodiments, the remote server 102 may determine the biometric information for the patient according to the processes discussed above with reference to step 254 (and steps 254a, 254b, 254c, and 254d) and FIG. 2D above.

Once biometric information for the patient is determined, the remote server 102 may determine a biometric context summary from the biometric information at step 926. Accordingly, the remote server 102 may isolate the biometric information that corresponds to the predetermined use period. In some cases, the remote server 102 may isolate biometric information corresponding to the predetermined use period for all biometric parameters determined for the patient. In other cases, the remote server 102 may isolate biometric information corresponding to the predetermined use period for a subset of biometric parameters determined for the patient, such as for biometric parameters specified by a technician via a technician interface 114 or a caregiver via a caregiver interface 116.

The remote server 102 uses the isolated biometric information to determine a biometric context summary for the predetermined use period. In some implementations, the remote server 102 may determine various summary metrics for the isolated biometric information, such as an average, a median, a minimum, or a maximum of a biometric parameter of the patient over the predetermined use period. For example, the remote server 102 may determine an average, minimum, and maximum hours per day that the patient was active over the predetermined use period. As another example, the remote server 102 may determine an average, minimum, and maximum hours of sleep the patient received per day of the predetermined use period. As another example, the remote server 102 may determine a maximum and a minimum respiration rate of the patient over the predetermined use period. In some cases, the remote server 102 may determine summary metrics for biometric parameters that overlapped. For instance, the remote server 102 may determine a median respiration rate for time periods when the patient was awake and a median respiration rate for time periods when the patient was asleep.

In some implementations, the remote server 102 may determine a value for a biometric parameter for the patient for a certain time period within the predetermined use period. The certain time period may be an hour, a day, a week, and so on. For example, the remote server 102 may determine how many hours per day and/or hours per week the patient was active over the predetermined use period. As another example, the remote server 102 may determine how many hours per day and/or hours per week the patient slept over the predetermined use period.

In some implementations, the remote server 102 may determine trends for the isolated biometric information over the predetermined use period. For instance, the remote server 102 may determine whether the amount of time the patient was active per day increased, decreased, or stayed the same over the predetermined use period. As another example, the remote server 102 may determine whether the amount of time the patient slept per day increased, decreased, or stayed the same over the predetermined use period.

In some implementations, the remote server 102 may determine whether arrhythmia events and/or symptom events (e.g., times when a patient provided an input indicating they were experiencing a potential cardiac-related symptom) occurred while the patient had a certain biometric status. As an illustration, the remote server 102 may determine whether a given arrhythmia event or symptom event occurred while the patient was active, while the patient was asleep, and so on. Additionally, in some implementations, the remote server 102 may determine a burden of arrhythmia events and/or symptom events while the patient had a certain biometric status. For example, the remote server 102 may determine a proportion of arrhythmia events that occurred while the patient was asleep and a percentage of symptom events that occurred while the patient was asleep. As another example, the remote server 102 may determine a percentage of arrhythmia events that occurred while the patient was active and a percentage of symptom events that occurred while the patient was active.

In some implementations, the remote server 102 may identify each type of arrhythmia event that occurred in the patient (e.g., from the determination of the arrhythmia events made at step 910 of FIG. 9A). The types of arrhythmia events may include, for instance, atrial fibrillation, supraventricular tachycardia, ventricular tachycardia, pauses, 2nd and 3rd degree AV block, and/or the like. For all or a subset of the types of arrhythmia events (e.g., the subset being specified by a user, such as a technician via a technician interface), the remote server 102 may determine a proportion of arrhythmia events that occurred while the patient had a certain biometric status. As an illustration, the remote server 102 may determine the percentage of atrial fibrillation events that occurred while the patient was asleep, the percentage of supraventricular tachycardia events that occurred while the patient was asleep, the percentage of ventricular tachycardia events that occurred while the patient was asleep, and so on. If an arrhythmia event spanned times when the patient had more than one biometric status (e.g., started when the patient was active and ended when the patient was inactive), the remote server 102 may determine a proportion of the arrhythmia event that occurred when the patient held the first biometric status, a proportion of the arrhythmia event that occurred when the patient held the second biometric status, and so on. In some cases, the remote server 102 may classify each proportion towards the total proportion of arrhythmia events that occurred while the patient had each biometric status. In other cases, the remote server 102 may classify the entire arrhythmia event under a certain biometric status if the majority of the arrhythmia event occurred while the patient had that biometric status. In other cases, the remote server 102 may classify the entire arrhythmia event under a certain biometric status if the onset of the arrhythmia event occurred while the patient had that biometric status.

In some implementations, the remote server 102 may determine a biometric summary for each arrhythmia event and/or symptom input that occurred in the patient. The remote server 102 may determine these biometric summaries as discussed above with reference to the arrhythmia event report 400 shown in FIGS. 4A and 4B and the symptom report 800 shown in FIGS. 8A and 8B. For example, the remote server 102 may determine an average respiration rate and sleep status for each arrhythmia event and/or symptom input. In implementations, additional or alternative processes for determining a biometric context summary, such as processes that use different biometric parameters, may be used.

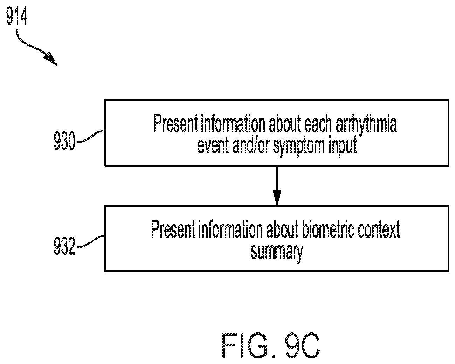

Returning to the sample process 900 shown in FIG. 9A, once the remote server 102 has determined the biometric context summary, the remote server 102 presents a summary view of the arrhythmia events and/or symptom inputs at step 914. An example process flow for step 914 of presenting a summary view of the arrhythmia events and/or symptom inputs is shown in FIG. 9C. In the example process flow shown in FIG. 9C, the remote server 102 presents information about each arrhythmia event and/or symptom input at step 930. As an example, the remote server 102 may display a total number of arrhythmia events and symptom events over the predetermined use period, the number of arrhythmia events and symptom events per day of the predetermined use period, and so on. In some cases, the remote server 102 may display each type of arrhythmia event and/or symptom event that occurred in the patient over the predetermined use period.

Additionally, the remote server 102 presents information about the biometric context summary at step 932. For example, the information about the biometric context summary may include summary metrics for the predetermined use period (e.g., an average hours per day the patient slept, an average hours per day the patient was active, etc.). As another example, the information about the biometric context summary may include biometric information for the patient shown over the predetermined use period (e.g., a plot of the number of hours per day the patient slept). As another example, the information about the biometric context summary may include trends in the biometric information over the predetermined use period (e.g., an indicating showing that the patient's activity decreased over the predetermined use period).

Further, the remote server 102 is configured to present the information about each of the arrhythmia events and/or symptom inputs in a predetermined relationship to the information about the biometric context summary. In some implementations, the predetermined relationship may include presenting the information about each of the arrhythmia events and/or symptom inputs on a first axis and the information about the biometric context summary on a second axis. As an example, the remote server 102 may plot the hours that the patient was active against a graphical timeline of the predetermined use period. On the same chart, the remote server 102 may plot a timing of each arrhythmia event and/or symptom event against the graphical timeline. For instance, the remote server 102 may plot an indication for each day that a type of arrhythmia event occurred. The remote server 102 may also plot an indication for each day that a symptom event occurred, or an indication for each day that a type of symptom event occurred. As another example, the remote server 102 may plot a proportion of arrhythmia events and/or symptom events that occurred while the patient had a certain biometric status, such as a percentage of arrhythmia events and/or symptom events that occurred while the patient was asleep versus while the patient was awake. As another example, the remote server 102 may plot a proportion of types of arrhythmia events and/or symptom events that occurred while the patient had a certain biometric status.

In some implementations, the predetermined relationship may be a human-readable sentence or a human-readable phrase that includes the information about each of the arrhythmia events and/or symptom input and the information about the biometric context summary. As an illustration, the remote server 102 may present a written summary indicating the average number of hours the patient was active per day, the minimum number of hours the patient was active per day, the number of arrhythmia events detected while the patient was active, and the number of symptom events reported while the patient was active. The written summary may further include the type of arrhythmia events that occurred while the patient was active, the type of symptom events (e.g., which symptoms the patient reported) while the patient was active, the percentage of the arrhythmia events that occurred while the patient was active to the total number of arrhythmia events that occurred over the predetermined use period, and so on.

In an example, the remote server 102 executes computer-readable instructions or code that accepts an input of "patientID" corresponding to an ID number for the patient for whom the remote server 102 is preparing a use-period report for a predetermined use period. The output is various statistics and figures for heart rate, respiration rate, activity, and posture, as well as their relationship with events over the use period. As such, example pseudocode for preparing a use-period report for a patient may be as follows:

```
        query event database to get technician-identified events, including
ventricular tachycardia, atrial fibrillation, SVT, pause, or symptomatic
events;
        download metric data (heart rate, respiration rate, activity, posture
in per minute units) from database;
        join the metric data with the event data;
        calculate the day/night heart rate median, total hours of activity per
day median, total hours of sleep per night median using activity_status
values;
                definition of activity_status
                        if activity_time and pitch are all available
                                if activity_time < 30
                                        if activity_time < 12 and pitch <=
        35: 'sleep;'
                                        else: 'not active';
                                if activity_time >= 30
                                        if pitch > 35: 'active';
                                        else: 'not active;
        definition of night: 9 pm to 9 am;
output summary statistics
        heart rate and respiration rate at minute level;
        maximum heart rate and respiration rate;
        activity and sleep per day/per night level
                mean/min/max of activity hours per day;
                mean/min/max of sleep per night;
```

```
            activity and events
                    for all events, count number of events that patient was active
    at onset of event;
                    for each type of event, calculate percentage of event that
    patient was active at onset of event;
            sleep and events
                    for all events, count events that patient was asleep at onset
    of event;
                    for all events, calculate percentage of event that patient was
    asleep at onset of event;
```

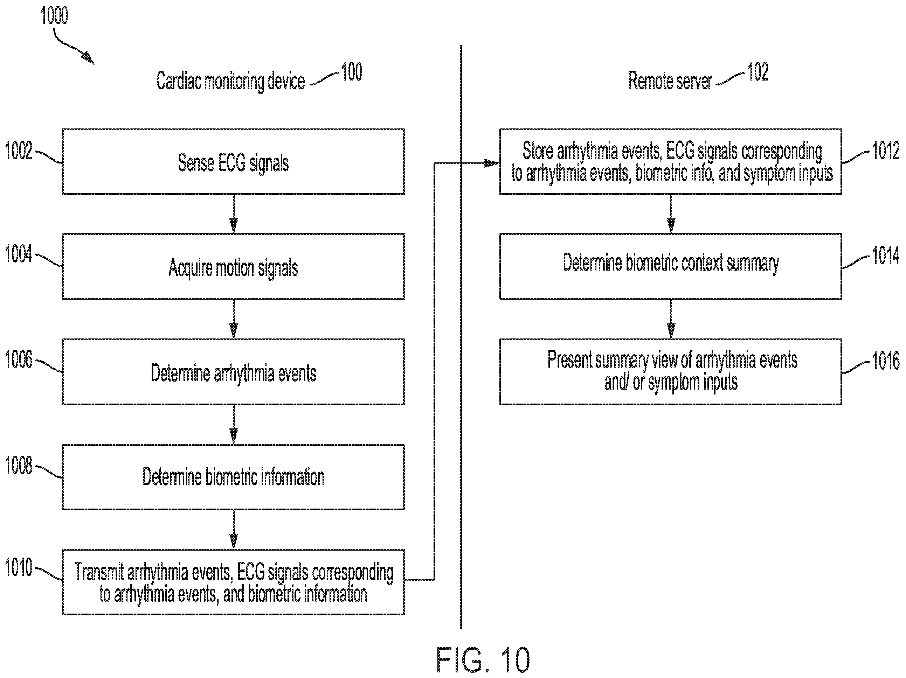

FIG. 10 illustrates another sample process flow for displaying graphical summary views of arrhythmia events occurring in a patient and/or patient-provided symptom inputs over predetermined use periods. For example, the sample process 1000 as shown in FIG. 10 can be implemented by the cardiac monitoring device 100 (e.g., a processor of the cardiac sensor 104 and/or a processor of the portable gateway 106) and by the remote server 102 (e.g., a processor of the remote server 102).

As shown in FIG. 10, the cardiac monitoring device 100 senses ECG signals at step 1002. The cardiac monitoring device 1004 also acquires motion signals via one or more motion sensors incorporated into the cardiac monitoring device 100 at step 1004. In various embodiments, the cardiac monitoring device 100 senses the ECG signals and acquires the motion signals according to the processes described above with reference to steps 202 and 204 of FIG. 2A, respectively, and with reference to steps 902 and 904 of FIG. 9A, respectively, discussed above.

The cardiac monitoring device 100 determines arrhythmia events occurring over a predetermined use period at step 1006. For example, the cardiac sensor 104 and/or the portable gateway 106 may determine the arrhythmia events from the ECG signals similarly to the processes described above with reference to step 910 of FIG. 9A. The cardiac monitoring device 100 further determines biometric information for the predetermined use period at step 1008. For instance, the cardiac sensor 104 and/or the portable gateway 106 may determine biometric information for the predetermined use period similarly to the processes described above with reference to step 214 of FIGS. 2A and 2D. More specifically, the cardiac sensor 104 and/or the portable gateway 106 may determine posture information, activity information, respiration information, respiration information, sleep information, and/or the like similarly to the processes described above with reference to steps 250-254 of FIG. 2D. In some cases, the cardiac monitoring device 100 may only determine biometric information for the predetermined use period, and in other cases, the cardiac monitoring device 100 may determine biometric information for a longer time period including the predetermined use period. In such cases, the remote server 102 may apply the predetermined use period to the biometric information and determine the portion of the biometric information that corresponds to just the predetermined use period.

The cardiac monitoring device 100 then transmits the arrhythmia events, at least the ECG signals corresponding to the arrhythmia events, and the biometric information to the remote server 102 at step 1010. In some embodiments, the portable gateway 106 transmits the arrhythmia events, at least the ECG signals corresponding to the arrhythmia events, and the biometric information to the remote server 102, as described above with reference to step 206 of FIG. 2A. In other embodiments, the cardiac sensor 104 may instead transmit the arrhythmia events, at least the ECG signals corresponding to the arrhythmia events, and the biometric information to the remote server 102, as described above with reference to step 206 of FIG. 2A. In some implementations, the cardiac monitoring device 100 may also transmit other signals to the remote server 102, such as ECG signals that do not correspond to arrhythmia events and/or raw motion signals to the remote server.

The remote server 102 stores the arrhythmia events, the ECG signals corresponding to the arrhythmia events, the biometric information, and symptom inputs (as well as any other information and/or signals received from the cardiac monitoring device 100) at step 1012. For example, the remote server 102 may include one or more databases and store the arrhythmia events, the ECG signals corresponding to the arrhythmia events, the biometric information, and symptom inputs in a database of the remote server 102. In various embodiments, the symptom inputs may be patient-provided or provided, for example, by a caregiver of the patient as described above with reference to step 908 of FIG. 9A.

The remote server 102 determines the biometric context summary for the predetermined use period at step 1014. The remote server 102 further presents summary views of the arrhythmia events and/or symptom inputs at step 1016. In various embodiments, the remote server 102 determines the biometric context summary and presents the summary views according to the processes described above with respect to steps 912 and 914 of FIG. 9A, respectively.

In some embodiments, the cardiac monitoring device 100 and the remote server 102 may implement a process flow that is a combination of the sample processes 900 and 1000. For example, the cardiac monitoring device may determine arrhythmia events that occurred over the predetermined use period and a first portion of biometric information for the patient. The cardiac monitoring device 100 may then transmit the arrhythmia events, the ECG signals corresponding to the arrhythmia events, the first portion of biometric information, and motion signals corresponding to a second portion of biometric information to the remote server 102. Subsequently, the remote server 102 may determine the second portion of the biometric information from the received motion signals. Alternatively, the remote server 102 may process the first portion of biometric information to identify the second portion of the biometric information. As an illustration, the cardiac monitoring device 100 may transmit patient heart rate information, patient posture information, patient activity level information, and patient respiration information to the remote server 102. The remote server 102 may then use the patient heart rate information and the patient activity level information to determine heart rate recovery information for the patient. The remote server 102 may further user the patient posture information, patient activity level information, and patient respiration information to determine sleep status information for the patient.

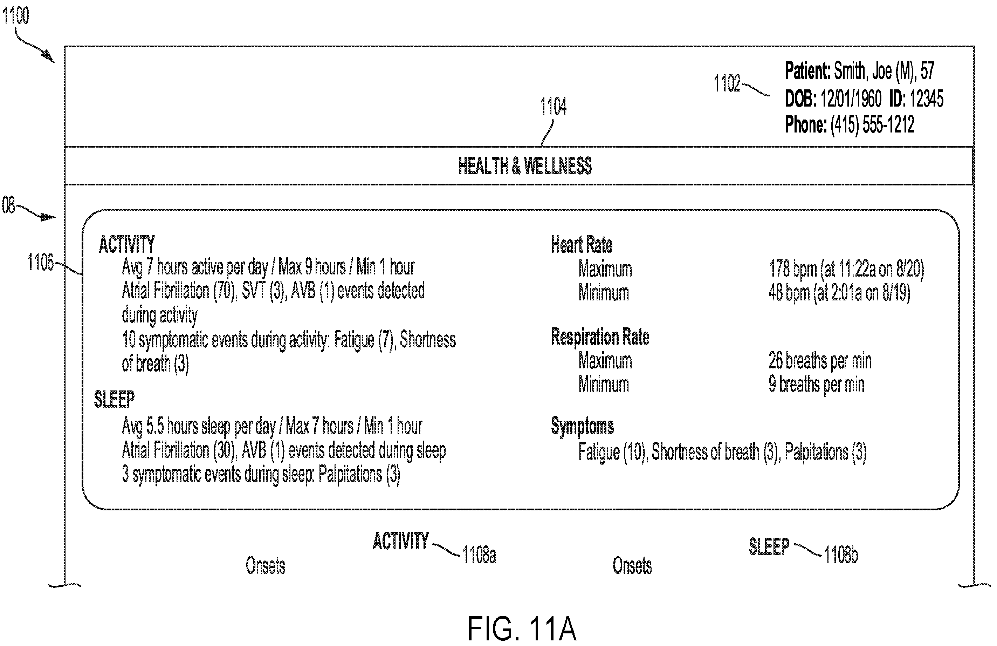

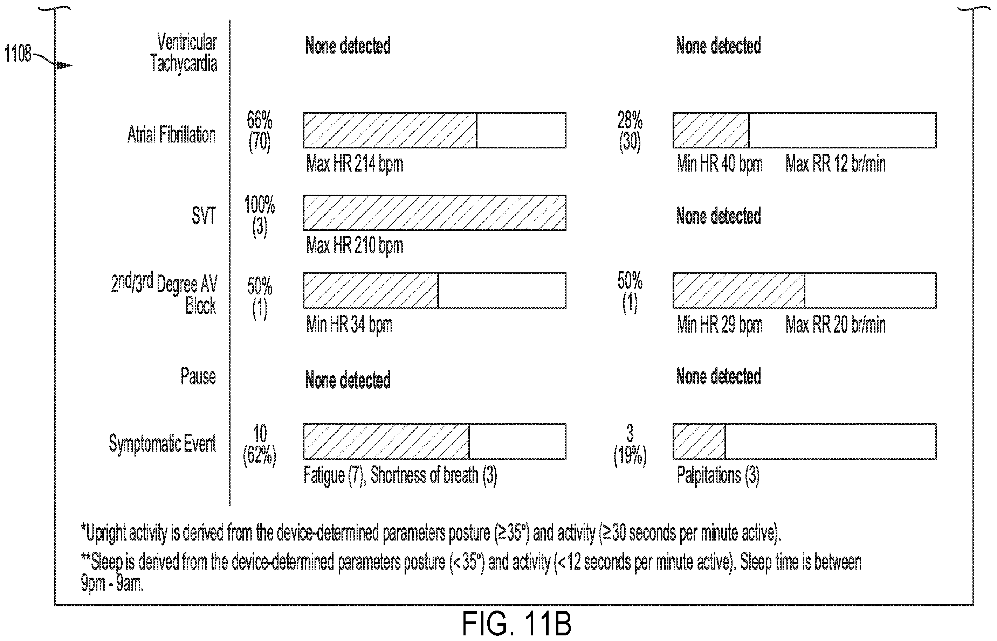

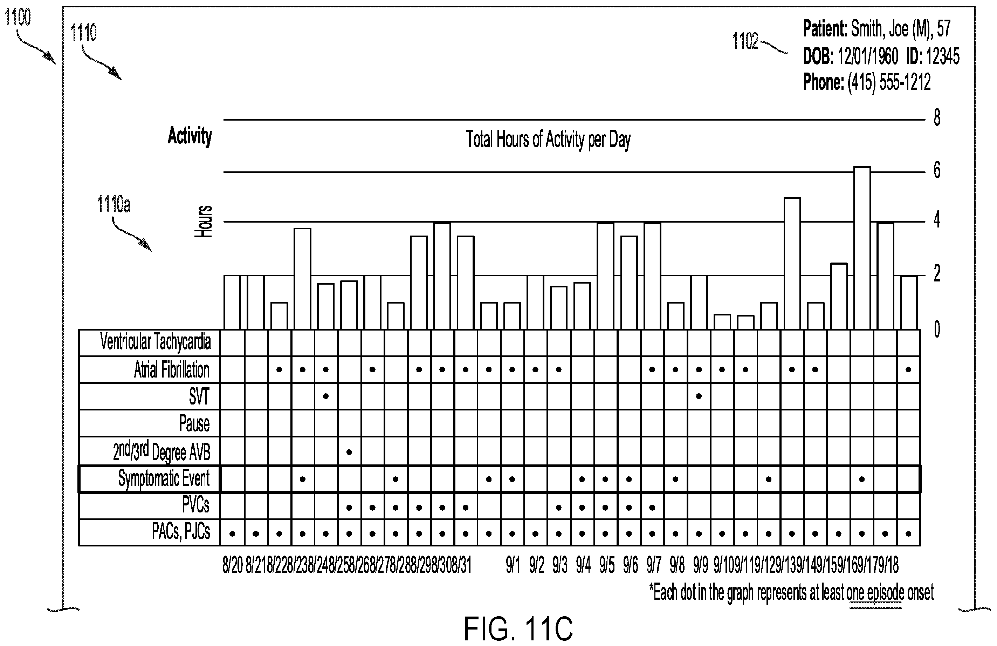

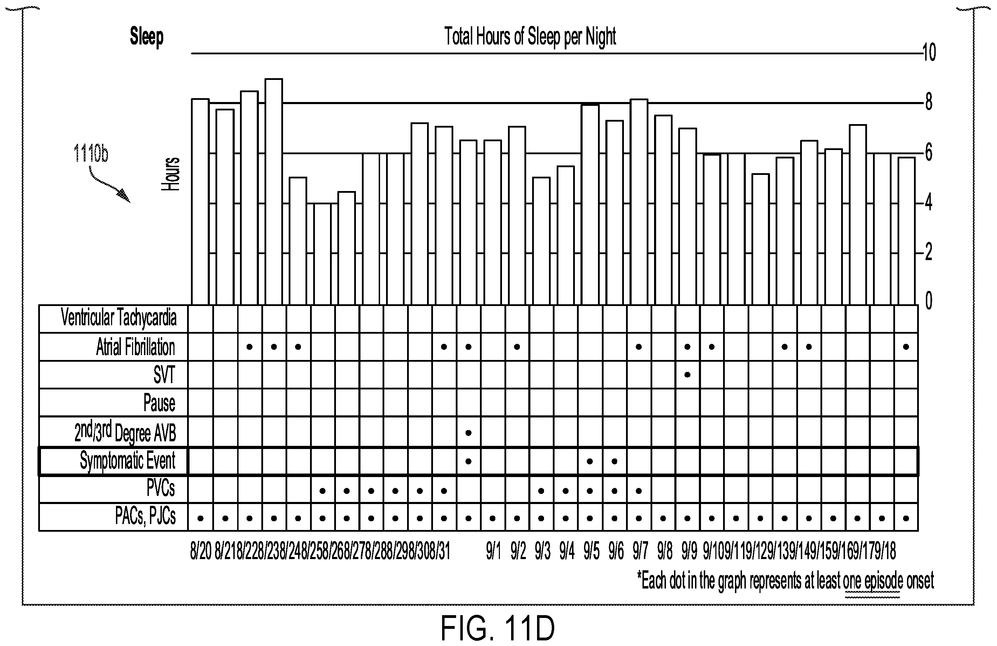

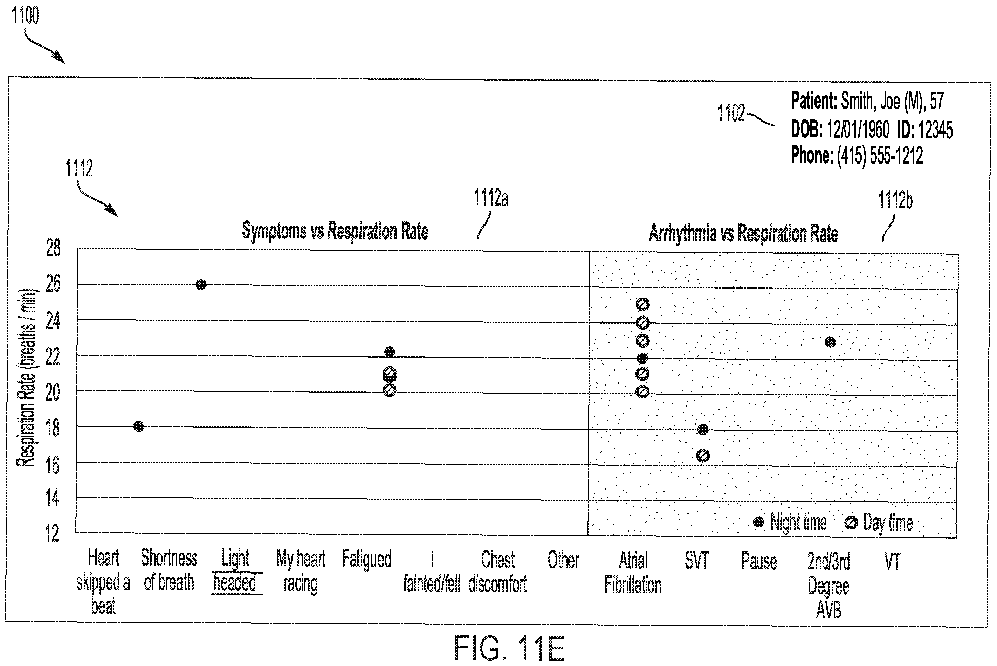

FIGS. 11A-11E illustrate an example use-period report 1100 (e.g., with FIG. 11A showing a first part of the use-period report 1100, with FIG. 11B showing a second part of the use-period report 1100, with FIG. 11C showing a third part of the use-period report 1100, with FIG. 11D showing a fourth part of the use-period report 1100, and with FIG. 11E showing a fifth part of the use-period report 1100) that may be displayed, for example, on a technician interface 114 and/or a caregiver interface 116. As shown in FIGS. 11A and 11B, and similar to the example arrhythmia event report 400 and example symptom report 800, the use-period report 1100 may include information 1102 that identifies the patient, such as the patient's name, date of birth, identification number, phone number, and/or the like. A use-period report 1100 may also include a header 1104 identifying the type of report.

The use-period report 1100 further includes a first summary section 1106. The first summary section 1106 presents human-readable phrases that present information about the arrhythmia events and/or symptom inputs, as well as biometric context summary information, occurring over the predetermined use period. As an illustration, in the example of FIGS. 11A and 11B, the first summary section 1106 includes the average, maximum, and minimum number of hours per day the patient was active; the number and types of arrhythmia events detected while the patient was active; and the number and types of symptom events the patient reported experiencing while the patient was active. The first summary section 1106 also includes similar information for the patient's sleep schedule. In addition, the first summary section 1106 includes a maximum and minimum heart rate for the patient over the predetermined use period, a maximum and minimum respiration rate over the predetermined use period, and a list of all symptoms reported by the patient.

Underneath the first summary section 1106, the use-period report 1100 includes a second summary section 1108. The second summary section 1108 presents charts that present information about the arrhythmia events and/or symptom inputs, as well as biometric context summary information, occurring over the predetermined use period. In the example of FIGS. 11A and 11B, the charts in the second summary section 1108 include a chart 1108a indicating the percentage of different types of arrhythmia events that occurred while the patient was active, as well as the percentage of symptom events reported as for times when the patient was active. The second summary section 1108 also includes a chart 1108b indicating the percentage of different types of arrhythmia events that occurred while the patient was asleep, as well as the percentage of symptom events reported for times when the patient was asleep.

Looking next to FIGS. 11C and 11D, the use-period report 1100 further includes a third summary section 1110 presenting additional charts that present information about the arrhythmia events and/or symptom inputs, as well as biometric context summary information, occurring over the predetermined use period. In the example of FIGS. 11C and 11D, the charts in the third summary section 1110 include a chart 1110a that plots the number of hours per day that the patient was active for each day of the predetermined use period. On the same chart is plotted the types of arrhythmia events that occurred for each day of the predetermined use period, as well as days of the predetermined use period where the patient reported experiencing a suspected cardiac-related symptom. The third summary section also includes another similar chart 1110b, except this second chart plots the total hours of sleep the patient experienced at night (e.g., defined as 8:00 pm to 6:00 am the next day) per day of the predetermined use period.

Looking next to FIG. 11E, the use-period report 1100 additionally includes a fourth summary section 1112. The fourth summary section 1112 presents an additional chart the presents information about the arrhythmia events and/or symptom inputs, as well as biometric context summary information, occurring over the predetermined use period. In the example of FIG. 11E, the chart in the fourth summary section 1112 illustrates a first section 1112a including the average respiration rate per type of symptom reported by the patient, for each symptom event reported for that symptom type over the predetermined use period. Additionally, the color of the indicators charting the average respiration rate per type of symptom reported by the patient indicate whether the symptom was reported during the nighttime or during the daytime. Similarly, the chart also illustrates a second section 1112b including the average respiration rate per type of arrhythmia event, for each arrhythmia event determined for that arrhythmia type over the predetermined use period.

The use-period report 1100 in some examples may include additional or different information. For example, the use-period report 1100 may include activity and sleep durations for each day of cardiac monitoring device 100 wear along with arrhythmic events and patient-reported symptoms, daytime and nocturnal median heart rate and respiration trends, and respiration rates at the onsets of arrhythmias and patient-reported symptoms. As another example, the use-period report 1100 may include summary statistics for upright activity duration, sleep duration, heart rate, and respiration for the use period; the summary of the arrhythmia and symptom onsets experienced by the patient during the use period; the median heart rate trend data during the day (e.g., shown as "daytime") and during the night (e.g., shown as "nocturnal"); duration of upright activity and sleep for each of the monitored days along with any arrhythmia or symptom onsets experienced by the patient during each day of the use period; median respiration rate trends during the day and during the night; graphical representations of the median respiration rates during the onset of the subject-reported symptoms and during arrhythmia onsets; and a "Technician Summary" section that provides a concise review of the percent of the time arrhythmias or symptoms occurred during upright activity and sleep. These data may provide insights to clinicians regarding health status of the patient during the monitoring over a period of time.

Similar to the arrhythmia event report 400 and symptom report 800, in some implementations, the use-period report 1100 may be shown to a user as illustrated in FIGS. 11A-11E. In implementations, the graphical display may present such information in an interactive manner. For example, the user of a technician interface 114 or caregiver interface 116 may be able to interact with the use-period report 1100 similarly to the interactions with the arrhythmia event report 400 and symptom report 800 discussed above. Additionally, the use-period report 1100 is described herein to illustrate some of the features of the techniques and systems described herein. The use-period report 1100 can include additional or alternative information, layout, formats, and/or graphical representations. For example, the x-axis of a graph or chart shown in a summary section of the use-period report 1100 may be in day units, in hour units, as day versus night information (e.g., displayed in two colors), and so on.

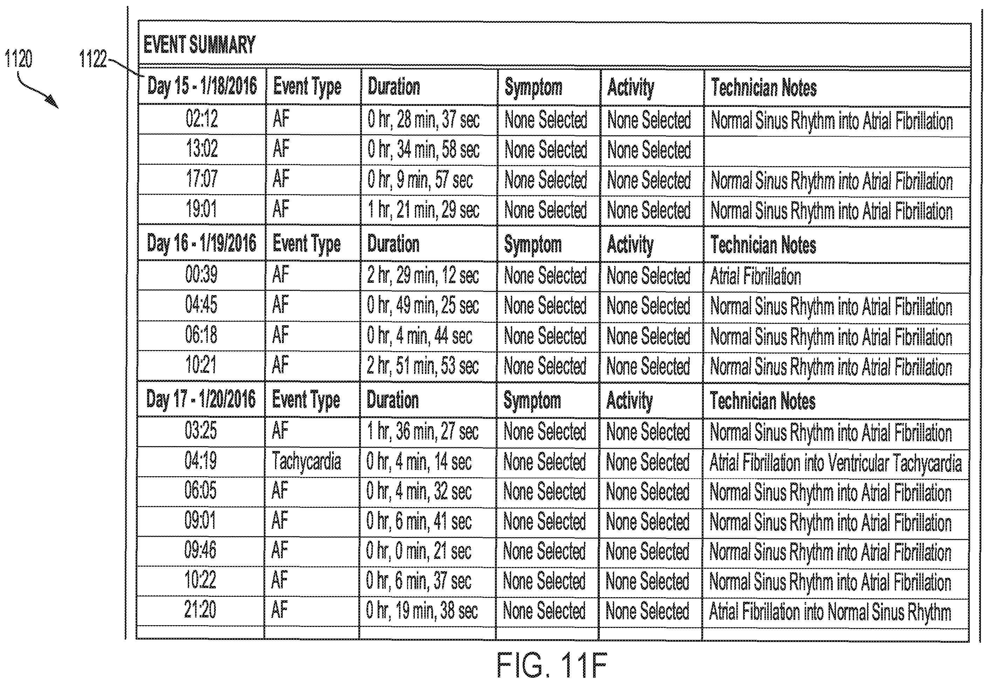

FIG. 11F illustrates an additional event summary 1120 that may be included in a use-period report, such as use-period report 1100 shown in FIGS. 11A-11E. The event summary 1120 may include an entry 1122 for each day of the use period, or for a subset of days of the use period (e.g., randomly sampled days; the first, last, and middle days of the use period; and so on). Each entry 1122 includes a list of all of the arrhythmia and/or symptom events that occurred in the patient during the use period. For example, as shown in FIG. 11F, each entry 1122 lists the time that an event occurred, the type of event that occurred, the duration of the event, whether the patient recorded any symptoms associated with the event (e.g., as a patient-provided symptom input), whether the patient was engaged in activity when the event occurred (e.g., as input by the patient or determined by the remote server 102 using the biometric information for the patient), and technician notes for the event. Additional or alternative types of data may be included in each entry 1122, in some implementations. The event summary 1120 may be provided to a caregiver of the patient, for instance, so that the caregiver can view all of the events that occurred in the patient to better understand the patient's cardiac health over the use period.

In some cases, each arrhythmia and/or symptom event in the event summary 1120 may be selectable. For instance, upon selecting an arrhythmia event or symptom event, the user may be redirected to a screen showing ECG information for the event and/or biometric information for the event (e.g., an activity level summary, posture level summary, respiration rate summary, sleep status, etc.). Alternatively, the user may be shown a pop-up screen overlaid on the event summary 1120 displaying the ECG information and/or biometric information for the event.

As an example, the cardiac monitoring device 100 was used in a study where the cardiac monitoring device 100 and the remote server 102 recorded heart rhythm, heart rate, subject-reported symptoms, and multiple parameters that include respiration rate, activity, and body posture (e.g., biometric data). The study was carried out to determine whether systems, devices, and methods as described herein provide clinicians with better insights into the context of detected arrhythmias, subject-reported symptoms, and/or wellness status. Subjects were prescribed to wear a cardiac monitoring device 100 as described herein for a maximum of thirty days. If there was an arrhythmic event or a subject-reported symptom event, investigators (e.g., the prescribing clinicians) received an event report describing the event along with the biometric information around the timeframe of these events. Investigators reviewed the ECG data first and answered a questionnaire on whether they found the ECG rhythm or symptom information useful. Then they reviewed the biometrics trend data to answer a questionnaire to document whether the biometric data were useful in assisting or changing their ECG- or symptom-based diagnosis and recommending modification(s) to the subject's treatment plan, including changes to medication, a follow-up plan, and/or lifestyle. At the end of the device 100 use, investigations received an end-of-use report (e.g., a use period report) that contained an assessment of the subject's wellness in addition to the heart rhythm and biometric information summary. Investigators answered addition questionnaires to understand how they used wellness information provided in the end-of-use report.

The patient/subject selection consisted of adult patients 21 years or older with an indication for mobile cardiac telemetry (MCT) monitoring for the detection of non-lethal cardiac arrhythmias. Patients were excluded if they had an implantable cardiac device, wearable cardioverter defibrillator, Holter monitor, wearable event recorder, and/or another MCT device at the same time. Other exclusion criteria were a skin condition preventing the patient from wearing the device 100, being hospitalized at the time of screening, being non-ambulatory, being pregnant, or participating in another study. All subjects provided informed consent, and the protocol was approved by a central or local Institutional Review Board.

The primary endpoint for the study was the demonstrated clinical utility provided by adding biometric information to the ECG-only and/or symptom-only assessment. Clinical utility of biometric data was determined by analyzing the clinicians' responses to the questionnaire based on the event reports. These questionnaires captured how useful the clinicians found the biometric data in assisting or changing their ECG-alone or patient-reported symptom-alone based action plan, and in recommending modifications to the subject's treatment plan, including changes to medication, a follow-up plan, and/or lifestyle.

The secondary endpoint for the study was the overall usefulness of longitudinal wellness reporting, e.g., wellness reporting over a period of time, for better patient management decisions. The usefulness of the longitudinal biometrics data was determined by analyzing the physicians' responses to the questionnaire based on the end-of-use report, which contained the wellness information (e.g., biometric information for the use period). The answers to these questionnaires determined whether physicians utilized the longitudinal biometrics data to better understand the circumstances associated with the reported arrhythmia(s), symptom(s), or when neither arrhythmia(s) or symptom(s) were reported. In addition, these answers were utilized to determine the recommendations or changes to the subject's care plan based on the longitudinal biometrics data.

With respect to data analysis, event-, subject-, and physician-level analyses were performed. Demographic, clinical, follow-up, and biometric data were presented as means $\pm$ standard deviation (SD) for skewed distributions as median and interquartile range (IQR). Arrhythmias of interest that were analyzed during this study included ectopic beats consisting of premature atrial and premature ventricular contractions, atrial fibrillation, supra-ventricular tachycardia, intra-ventricular conduction disorders, ventricular tachycardia, pause, and 2nd/3rd degree atrioventricular blocks. Odds ratios were used to quantify the strength of the associations between the various arrhythmias of interest and the activity biometrics. Pairwise t-test with Holm-Bonferroni correction was used to compare heart rate and respiration rate between the various arrhythmias of interest. Identical analyses were performed for determining the associations between subject-reported symptoms and biometrics data. Physician responses to the event report and end-of-use report questionnaires were reported at an event-, subject-, and physician level. Changes to the subject's treatment plan (medication, follow-up plan, and/or subject's lifestyle), when an arrhythmic event or subject-reported symptom was reported, based on the biometrics data were analyzed for each of the biometrics (activity, body position, sleep, and respiration rate). All analyses were conducted using RStudio version 1.2.1335 (RStudio Inc, Boston, MA). $P < 0.05$ was considered significant.

Five hundred eighty-three patients were enrolled by twenty-seven physicians from eighteen U.S. cardiology practices. Information for these patients is shown below in Table 1. The mean age of these subjects was $63 \pm 16$ years, and 43% were male. The most common reason for device 100 prescription was palpitations (40%) followed by atrial fibrillation (21%). Based on the prescriptions, the median length of need was 30 days (IQR = 14-30 days). Five hundred thirty-seven (92%) of the subjects wore the device 100 for longer than one day. For these 537 subjects, the median number of days they used the device 100 was 21 days (IQR = 12-30 days), and the median average wear time per day was 21.5 hours (IQR = 19-23 hours). Of the 537 subjects who wore the device for longer than one day, end-of-use reports were published for 535 subjects. Insufficient trend information prevented the creation of end-of-use reports for the remaining two subjects.

Table 1:

| (n=583) | |
| --- | --- |
| Age, years | $63 \pm 16$ |
| Male, n | 249 (43%) |
| Reason for MCT monitoring | |
| Palpitations, n | 231 (40%) |
| Atrial Fibrillation, n | 121 (21%) |
| Syncope/Pre-syncope, n | 86 (15%) |
| SVT/Tachy/Brady, n | 60 (10%) |
| Other, n | 83 (14%) |
| BMI | $31 \pm 8$ |
| Height, inches | $66 \pm 4$ |
| Weight, lbs. | $195 \pm 52$ |
| EF, % (n=284) | $58 \pm 9$ |
| History, n | |
| Palpitations | 419 (72%) |
| Hypertension | 378 (65%) |
| Hyperlipidemia | 298 (51%) |
| Dizziness | 258 (44%) |
| Smoking | 202 (35%) |
| Syncope | 106 (18%) |

(continued)

| (n=583) | |
|---|---|
| **SVT** | 94 (16%) |
| **Bradycardia** | 88 (15%) |
| **Sleep Apnea** | 71 (12%) |
| **Heart Failure** | 65 (11%) |
| **CABG** | 62 (11%) |
| **Prior Ablation** | 61 (10%) |
| **Stroke** | 59 (10%) |
| **Myocardial Infarction** | 39 (7%) |
| **Chronic Kidney Disease** | 33 (6%) |

A total of 5,831 event reports were generated for the 535 subjects who wore the device 100 for more than one day. Arrhythmias of interest were detected in 365 (68%) subjects. Ectopic beats were detected in 53% of the subjects; atrial fibrillation, supra-ventricular tachycardia (SVT), and intra-ventricular conduction disorders (IVCD) were detected in 15%, 13%, and 11% of the subjects, respectively; and ventricular tachycardia (VT), pause, and 2nd/3rd degree atrioventricular blocks (AVB) were detected in 3%, 2.6%, and 1.9% of the subjects, respectively. Overall, 313 (59%) subjects reported at least one symptom. The commonly reported symptoms were heart racing (25%), light headedness (22%), shortness of breath (21%), fatigue (19%), skipped beat (18%), and chest discomfort (14%). Two percent of the patients reported to have fainted or fallen during the study.

Twenty-six physicians completed 2,685 event report questionnaires from 508 subjects. When presented with just the initial ECG and associated symptoms, physicians labelled 17% of the events as occurring while subjects were active, 5% of the events as occurring while the subjects were sleeping, and 3% of the events as occurring while the subjects were tachypneic. In contrast, when physicians reviewed the ECG along with the biometrics data, they were able to assess that 70% (P < 0.001) of the events occurred while the subjects were active, 55% (P < 0.001) of the events occurred while sleeping, and for 39% (P < 0.001) of the events, the subjects were tachypneic.

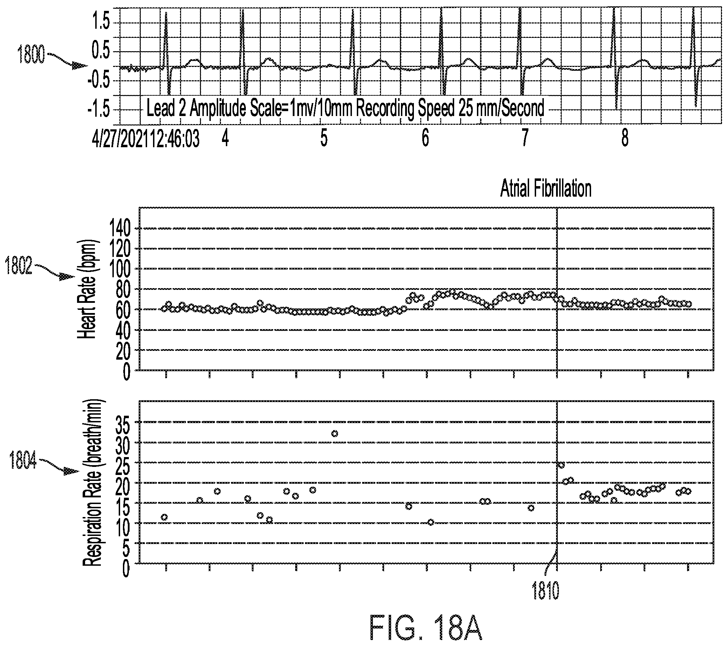

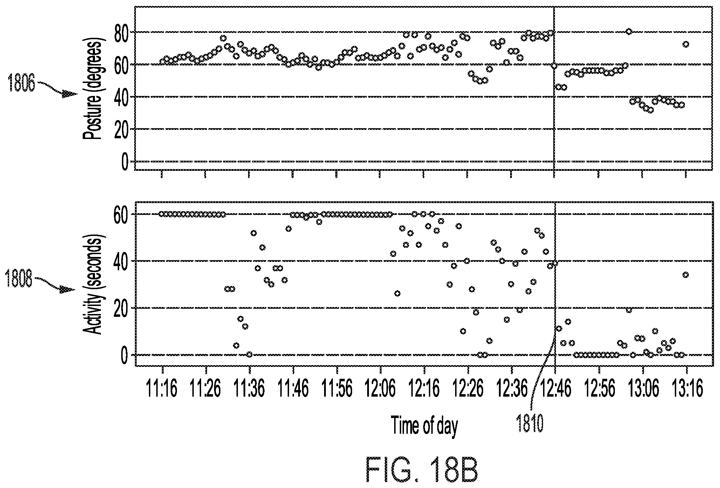

An example event report is shown in FIGS. 18A and 18B (e.g., with FIG. 18A showing a first part of the example event report and with FIG. 18B showing a second part of the example event report), where a first section 1800 illustrates a six-second ECG for the onset of atrial fibrillation with controlled ventricular response. Second through fifth sections 1802-1808, respectively, illustrate contextual biometric information for the episode of atrial fibrillation, with a vertical line 1810 showing the onset of the atrial fibrillation episode. More specifically, second section 1802 shows the patient's heart rate trend data, third section 1804 shows the patient's respiration rate trend data, fourth section 1806 shows the patient's posture trend data, and fifth section 1808 shows the patient's activity trend data in one-minute intervals 90 minutes before and 60 minutes after the atrial fibrillation onset. As illustrated in FIGS. 18A and 18B, minutes before the onset of atrial fibrillation, the subject showed a decreasing trend in upright activity. At the onset of the atrial fibrillation, the subject was inactive and was tachypneic (with a respiration rate of 25 breaths/min). The subject returned to upright activity after about 30 minutes of not being active. Based on these biometric trend data, the physician initiated changes to medication, follow-up plan, and subject's lifestyle.

Overall, with the assistance of the biometric data in addition to ECG data, 96% of the physicians changed an individual subject's treatment plan, including changes to medication, a follow-up plan, and/or subject's lifestyle. In combination, treatment plans were changed for a total of 64% of the subjects. Specifically, the biometric data assisted physicians in making changes to medication and follow-up plan in 17% of the subjects. When determining which biometric data physicians found useful in recommended lifestyle changes, activity was the most useful in 62% of subjects, respectively. From physician reporting, the change recommendations included activity/exercise, explanation of relationship of events to activity levels and body position, changing sleep position, changing sleep pattern, stress reduction, reduction of caffeine intake, and subject reassurance.

The analysis, at the event level, on how physicians utilized the biometrics data to change treatment plans is shown in Table 2 below. For example for VT events, the treatment plan (medication, follow-up plan, and/or lifestyle) was changed compared to ECG alone in 90% of the events, with activity and body position as the most useful for making changes to the treatment plan. Similarly, when the subject reported fainting or falls, physicians used biometrics data to change the treatment in 88% of the events compared to ECG alone. In these patients, the activity biometrics were deemed to be most helpful (86%).

# EP 4 258 984 B1

Table 2:

| Treatment Plan Change and Biometrics (n=2685 events) | | | | | | |
|---|---|---|---|---|---|---|
| | Total Events , n | Treatment Plan Changed, n (%) | Treatment Plan Change Using Activity (%) | Treatment Plan Change Using Body Position (%) | Treatment Plan Change Using Sleep (%) | Treatment Plan Change Using Respiration Rate (%) |
| Arrhythmia | | | | | | |
| Ectopic Beat | 583 | 297 (51%) | 98% | 74% | 55% | 42% |
| Atrial Fibrillation | 338 | 223 (66%) | 91% | 66% | 71% | 33% |
| SVT | 170 | 111 (66%) | 99% | 73% | 51% | 43% |
| 2nd/3rd Degree AV Block | 35 | 22 (63%) | 95% | 95% | 86% | 9% |
| Pause | 34 | 21 (62%) | 90% | 90% | 86% | 76% |
| Ventricular Tachycardi a | 21 | 19 (90%) | 89% | 84% | 42% | 21% |
| Symptom | | | | | | |
| Skipped Beat | 380 | 234 (62%) | 97% | 65% | 45% | 40% |
| Heart Racing | 281 | 168 (60%) | 99% | 72% | 62% | 44% |
| Shortness of Breath | 215 | 98 (46%) | 97% | 79% | 64% | 49% |
| Light Headedness | 214 | 118 (55%) | 96% | 78% | 51% | 44% |
| Chest Discomfort | 199 | 110 (55%) | 97% | 81% | 77% | 61% |
| Fatigue | 152 | 88% (58%) | 99% | 74% | 57% | 55% |
| Other | 106 | 58 (55%) | 98% | 74% | 64% | 67% |
| Fainted/Fell | 8 | 7 (88%) | 86% | 57% | 29% | 57% |

Longitudinal wellness information for the use period of the device 100 was provided for each of the 535 subjects in the form of an end-of-use summary, as described above. The information contained in the wellness report includes activity and sleep durations for each day along with arrhythmic events and patient-reported symptoms, as well as median heart rate and respiration trends (daytime and nocturnal) and respiration rates at the onsets of arrhythmia or symptoms. For instance, the end-of-use wellness report included a graph showing the median daytime heart rate for each day of the use period and the median nighttime heart rate for each day of the use period, and a graph showing the median daytime respiration rate for each day of the use period and the median nighttime respiration rate for each day of the use period.

Twenty-seven physicians completed end-of-use report questionnaires for 527 subjects. Ninety-six percent of the physicians found the end-of-use wellness information useful to better understand the circumstances associated with the arrhythmia diagnosis or subject-reported symptoms (in 52% of the subjects). With the addition of wellness information, 63% of the physicians changed the treatment plan (e.g., changes to medication, a follow-up plans, and/or subject's lifestyle) for at least one subject. These changes were made for 24% of the total subjects. Specifically, changes in medications and/or a follow-up plan were made in 11% of the subjects, and in 22% of the subjects the wellness summary was used to recommend changes to subject's lifestyle. For the 91 subjects who had no reported arrhythmias or symptoms, physicians reported the wellness report helpful to better understand the patient status in 38 subjects (42%).

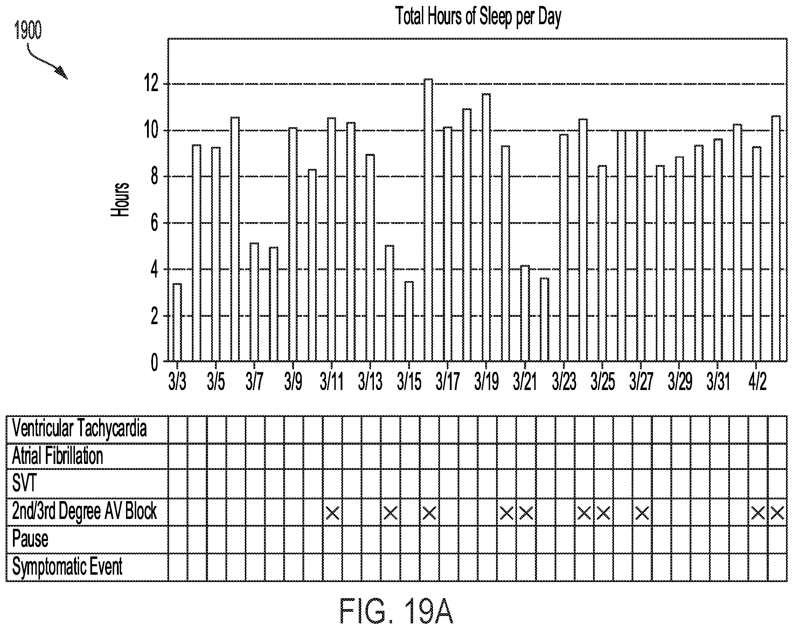

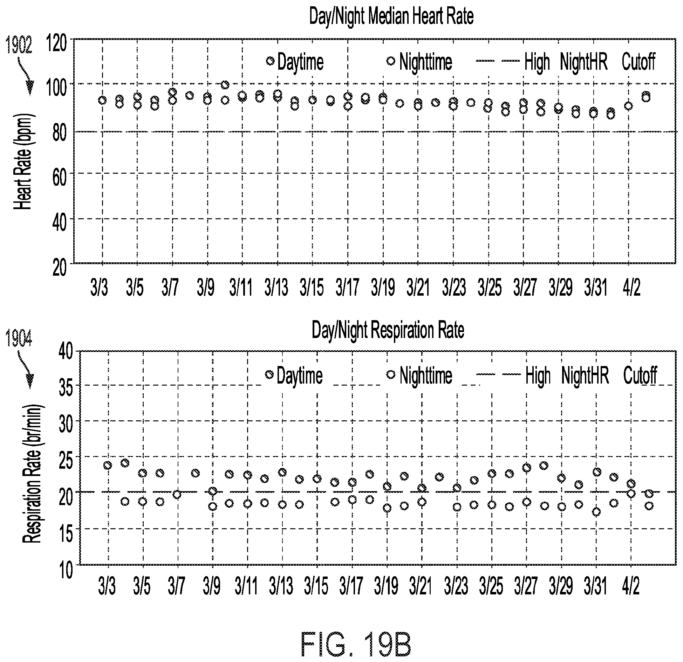

An example use-period wellness report is shown in FIGS. 19A and FIG. 19B (e.g., with FIG. 19A showing a first part of the example use-period wellness report and FIG. 19B showing a second part of the example use-period wellness report). A first section 1902 illustrates sleep duration for each day of the use period along with summaries of arrhythmias and symptomatic events. In the example first section 1902 shown in FIG 19, the subject experienced several 2nd/3rd AVB events when the subject was in a sleep state. A second section 1904 illustrates the subject's median heart rate for each day of the use period, and a second section 1906 illustrates the subject's median respiration rate for each day of the use period. The subject also experienced consistently elevated median heart rate of about 90 bmp during each night of the monitoring period, as shown in the second section 1904, and an increased median respiration rate of about 18 breaths per minute during the night, as shown in the third section 1906. Based on these data the physician reported to have changed the

45

medication, recommended lifestyle changes, and better understood the circumstances of the reported arrhythmias. Additionally, the physician shared these longitudinal wellness data with the patient and reported that they were "addressing sleep apnea as the potential cause."

Overall, this example study found that, when using biometrics data (both from event and end of use reports) compared to ECG alone, 96% of the physicians made changes to the subject's treatment plan. The biometrics-based treatment plan changes involved 63% of all patients (n=535) and included modifications to medications, follow-up plans, and lifestyle in 18%, 19%, and 62% of the subjects, respectively. Furthermore, for the subgroup of subjects with a reported arrhythmia or symptom (n=444), physicians reported that the biometrics data helped better understand the circumstances associated with the event in 69% of these subjects.

The study also found that among the various arrhythmias and symptoms, atrial fibrillation, shortness of breath, and light-headedness showed a significant likelihood of occurring during activity. In contrast, 2nd/3rd AVB, pauses, skipped beats, and heart racing events were more likely to occur when subjects were at rest (inactive and/or asleep). Providing objective physiologic data to clinicians reduces or eliminates the reliance on the subjective patient diaries and provides more in-depth contextual information around provocative factors and patient responses, enhancing the interpretation of both arrhythmic events and symptoms.

Additionally, elevated resting heart rate has been shown to be an independent risk factor for mortality and cardiovascular risk. Furthermore, studies have shown that heart rate reduction is associated with improved outcomes. As such, systems and methods herein facilitate the achievement of rate control that is helpful in not only improving cardiovascular outcomes but also improving quality of life. In examples, new or worsening symptoms during rate control therapy titration can prompt investigation with ambulatory cardiac monitoring. In such cases, providing clinicians with heart rate and biometrics trend data, as described above, before and after the onset of an arrhythmic or symptomatic event could aid the physician in better rate control therapy titration. One finding from the current study related to the onset of atrial fibrillation. The median heart rate of 96 bpm at atrial fibrillation onset was significantly higher than the heart rate at the onset of other arrhythmias. Based on this, physicians used the activity assessment at a higher frequency (91% of the events; Table 2) than other biometrics parameters, when modifying the treatment plan for patients with atrial fibrillation.

While most cardiac monitors are prescribed solely for diagnostic purposes, the ability to review daily biometrics and the availability of trending data allows for a broader range of usefulness. As described above, the wellness trend report, available at the end of the prescription use period, contains daytime and nighttime heart rate trending data. This was useful in assessing the response to medication titration and as a tool to review medication compliance.

Ambulatory daily respiration rates trends and elevated sleeping respiration rates have also been identified as important risk markers for heart failure and sleep apnea. However, no previous study has reported the relationships between arrhythmic or symptomatic events and respiration rate. In this example study, the respiration rates at the onset of SVT and shortness of breath events were significantly higher than the respiration rates of other arrhythmias and other reported symptoms. In contrast, the respiration rates at the onset of pause events were significantly lower than the respiration rates seen at the onset of other arrhythmias. Interestingly, physicians modified the treatment for 76% (Table 2) of pause events when they had respiration rate data available. These results exemplify the fidelity of the biometric data to capture the interactions of the cardiopulmonary system, which then allow physicians to modify their treatment plan accordingly.

Further, in the example study, 91 (17%) out the 535 subjects did not experience an arrhythmia of interest or report any symptoms. Typically, in these subjects the cardiac monitoring would be considered as inconclusive. In this study, however, physicians were able to use the longitudinal wellness information provided in the end of use reports to better understand the patient status. Physicians reported the longitudinal trend data was useful in 38 (42%) of these 91 subjects. Additionally, even in subjects with arrhythmic or symptomatic events, these longitudinal wellness data provided insights into patient's overall wellness and health status by reporting total daily activity time and sleep durations. In many cases these longitudinal biometric data were harbingers of other cardiovascular risk and comorbidities. For example, as seen in FIGS. 19A and 19B, the elevated nocturnal heart rate, where heart rate decreased less than 10% from the daytime heart rate, has been shown in previous studies as an important predictor of cardiovascular and non-cardiovascular risk.

In some implementations, a biometric context report may be formatted or formulated differently from the examples and embodiments provided above. As an example, arrhythmia information may be disassociated from biometric context information (e.g., with the ECG showing an arrhythmia provided on one page and biometric context information, such as 24-hour trends or nighttime biometric information, provided on a second page). As another example, a report may allow for further interactivity with a caregiver. For instance, a caregiver may be able to zoom into a portion of a report (e.g., ECG information and/or biometric context information) to see shorter trends, such as daily trends for the patient, and zoom out of the portion of the report to see longer trends, such as weekly or monthly trends. As a further illustration, a caregiver may be able to configure a report to view the same time period from multiple days. As a further illustration, a caregiver may be able to enter a specific time period (e.g., over a certain number of days, over a certain number of weeks, etc.) and a time of day and thereby view ECG information and/or biometric context information for each day of the specific time period and the specified time of day.

As another example, a report may illustrate where a medication change for the patient occurred along with ECG biometric context information for the patient to show whether medication changes are correlated with changes in arrhythmias and/or biometric information. As another example, a report may include trends of ECG information and/or arrhythmia information along with trends of other biometric context information, such as trends of posture, activity level, respiration rate, and/or sleep status over the same period of time (e.g., whether an arrhythmia occurred in a given week trended with average activity level per day for the given week). As another example, a report may illustrate sleep trends over time (e.g., how many hours of sleep per day the patient has gotten). As another example, a report may include three-dimensional (3D) biometric context information for ECG information and/or arrhythmia information, such as by mapping the ECG information and/or arrhythmia information against time and a type of biometric context information. To illustrate, arrhythmia information over time may be mapped in the same 3D graph against patient activity over time. As another example, a report may display trends from previous weeks (e.g., compressed trends) along with more granular ECG information and/or arrhythmia information and biometric context information for the present day, week, 10-day period, etc. As another example, a report may display ECG information and/or arrhythmia information and biometric context information in a heat map. As another example, a report may group types of biometric context information with ECG information and/or arrhythmia information. For instance, a report may show ECG information or atrial fibrillation episodes when the patient was at a body posture of greater than 90 degrees.

As another example, a report may include longer strips of ECG information (e.g., hour-long ECG strips, two-hour long ECG strips, three-hour long ECG strips, four-hour long ECG strips, five-hour long ECG strips, six-hour long ECG strips, twelve-hour long ECG strips, eighteen-hour long ECG strips, twenty-four-hour long ECG strips, etc.) with a running trend (e.g., from the past 3 days, past 5 days, past week, past two weeks, past month, etc.) above or underneath the strips of ECG information. As another example, a report may include longer strips of ECG information with markers that indicate changes in trends of the patient's biometric context information. To illustrate, a report may show when a patient's activity level rises above or falls below a predetermined level or changes by a predetermined percentage, rises above or falls below a predetermined level of average sleep per night or changes by a predetermined percentage, when a patient's wear time rises above or falls below a predetermined level or changes by a predetermined percentage, etc. As another illustration, a report may note changes in an ECG strip that correspond with a change in one or more biometric context information trends.

As another example, a report may include a wellness "score" that summarizes the arrhythmia information and biometric context information in the report. For instance, a wellness score may be based on a combination of sinus rhythm over the report period, average level of patient activity over the report period, and whether the patient was at or above a predetermined average sleep level over the report period. As another example, a report may include an arrhythmia risk score for the patient, for example, based on the trends of the biometric context information (e.g., decreasing heart rate variability). For instance, machine learning may be used to determine whether, based on various inputs such as the types of biometric context information, a predicted risk (e.g., from 0 to 100) of the patient developing an arrhythmia. As another example, a report may include a heart failure prediction (e.g., based on R-R intervals, based on heart rate, etc.) using, for instance, machine learning similar to how an arrhythmia risk score may be generated. As another example, a report may include suggested next steps to improve the patient's health (e.g., a pacemaker, increased physical activity, increased sleep, a medication change, etc.).

As another example, a report may include wear time for the cardiac monitoring device 104 over the biometric context time period and/or over a use period (e.g., determined from whether the cardiac monitoring device 104 is on, from whether the electrodes are sensing an ECG from the patient, from impedance sensors configured to transmit a fall-off signal into the patient, etc.). For instance, a report may include wear time overlaid with sleep status and activity level graphs. As another example, a report may include heart rate variability alone, overlaid with other biometric context information, overlaid with arrhythmic data, linked to an autonomic nervous system disorder, recurrence plots for heart rate variability to visualize patterns, etc.

As another example, a report may be configured to be presented to a patient. For instance, a report may include more streamlined information such that it is easier for a patient to parse through the information, such as by including more trends and less day-to-day information and/or including a shortened summary of the information in the report at the beginning of the report with less detailed patient information. As another illustration, a patient report may include a patient's recommendations for improving the patient's health. As another illustration, the patient may be provided with a patient report along with notifications, such as a notification to increase an activity level or increase the amount of time the patient sleeps if the patient trends below a predetermined level. Notifications may be sent, for example, to the patient via email, as a text or SMS message, as a phone call, etc. As another illustration, the patient report may include a wellness score, as described above.

Returning to the cardiac monitoring device 100, in some implementations, the cardiac monitoring device 100 may include a cardiac sensor 104 that further includes an adhesive patch 108 and a sensor unit 110. FIGS. 12A-12E show the cardiac sensor 104 discussed herein including the adhesive patch 108 and the sensor unit 110, as well as illustrations of attaching the cardiac sensor 104 to a patient's body. As shown in FIG. 12A, the patch 108 may include a patch frame 1230 (e.g., plastic frame) delineating the boundary of the region of the patch 108 that is configured for housing the sensor unit 110. The patch 108 may be disposable (e.g., single- or few-use patches), and may be made of biocompatible, non-woven material. In some embodiments, the sensor unit 110 may be designed for long-term usage. In such embodiments, the connection between the patch 108 and the sensor unit 110 may be configured to be reversible, e.g., the sensor unit 110 may be configured to be removably attached to the patch 108. For example, as shown in FIG. 12B, the sensor unit 110 may include components such as snap-in clips 1270 that are configured to secure the sensor unit 110 to the patch 108 (e.g., the patch frame 1230) upon attachment (and released the sensor unit 110 from the patch 108 when separation is desired), as further illustrated in FIG. 12C. The sensor unit 110 may also include positioning tabs 1260 that facilitate the attachment process between the sensor unit 110 and the patch 108. In some embodiments, the patch may be designed to maintain attachment to skin of a patient for several days (e.g., in the range from about 4 days to about 10 days, from about 3 days to 5 days, from about 5 days to about 7 days, from about 7 days to 10 days, from about 10 days to 14 days, from about 14 days to 30 days, etc.).

In some embodiments, the patch 108 may include additional components that facilitate or aid with the monitoring and/or recording or acquiring of physiological data by the sensor unit 110. For example, as discussed above, the patch 108 may include conductive elements such as one or more ECG electrodes 112 (e.g., a single lead, two leads, etc.) that can be used when recording ECG signals from the surface (e.g., skin contacted directly or through a covering) of a patient's body. The electrodes 112 may be coupled to the sensor unit 110 by dedicated wiring within the patch. In some embodiments, the ECG may have a sampling rate in then range from about 250 Hz to about 500 Hz, from about 300 Hz to about 450 Hz, from about 350 Hz to about 400 Hz, including values and subranges therebetween. In some embodiments, the ECG signal may be sampled after band-pass filtering by a 12 bit ADC. During normal operation, data may be transferred to the server "as-is" and can then be used by the remote server 102 for analysis. In some embodiments, an internal process allows for real-time evaluation of the ECG signal quality upon each attachment of the device to the patient ("attachment test"). Alternatively, as discussed above, in some embodiments, an internal process allows for processing of the ECG signals, such as by determining arrhythmia events that have occurred or are occurring in the patient based on the ECG signals.

Examples of locations on surface of a patient body at which a patch may be placed are shown in FIG. 1, as well as in FIGS. 12D and 12E, where a patch 108 housing the sensor unit 110 is shown as placed at on the side (below armpit, for example, as shown in FIG. 12D) and upper chest of the torso of a patient (along the left midclavicular line over the pectoris muscle to provide a lead-II equivalent electrode placement, for example, as shown in FIG. 12E). It is to be noted that the patch may be placed on any part of the surface of a patient's body that allows for efficient monitoring and recording of a physiological data (e.g., area of skin that allows for uniform attachment of the patch 108 to the skin). For example, one may place the patch 108 under an armpit at the nipple level for performing lung fluid level measurements. With respect to ECG measurements, the ECG signal at this location may be represented as the difference between standard V5 and V6 leads of an ECG.

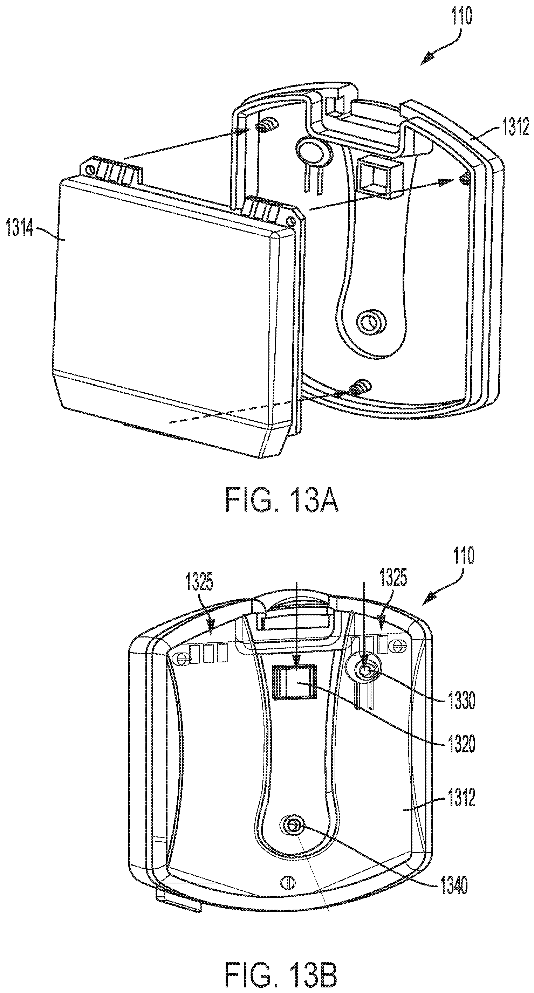

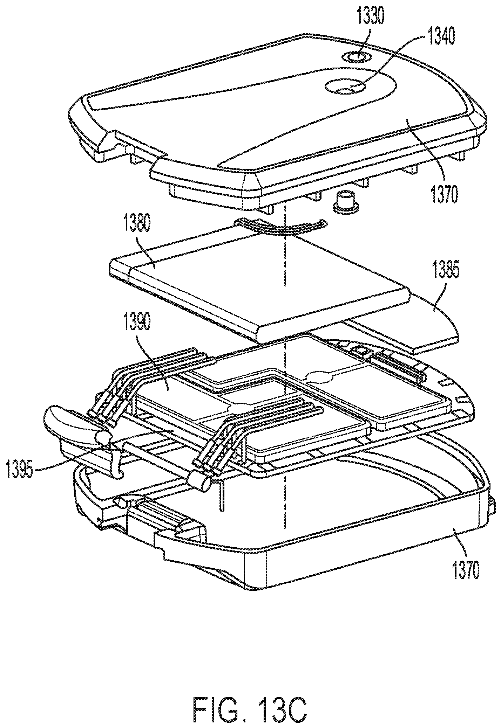

With reference to FIGS. 13A-C, in some embodiments, front, back and exploded views, respectively, of the sensor unit 110 disclosed herein are shown. FIG. 13A shows the front 1312 and back 1314 covers of the sensor unit 110 (labelled as top and bottom covers 1370 in FIG. 13C). In some embodiments, such covers may couple to each other to seal the electrical components of the sensor from the surrounding environment (e.g., electrical sealing). In such embodiments, as shown in FIG. 13B, metallic tabs 1325 may protrude outside the covers to provide electrical connection for situations such as performing ECG measurements, charging power source and/or the like.

FIG. 13B shows that the sensor unit 110 may include one or more indicators that identify the status of the sensor unit 110 to the user of the sensor unit 110. Examples of such indicators include but are not limited to a light indicator 1340 (e.g., a light emitting diode (LED) indicator) and sound indicators 1320. In some embodiments, the indicators 1320, 1340 provide feedback on the status of the sensor unit 110 and components thereof, such as the charging and/or power level of the power source of the sensor unit 110 (e.g., a battery), the attachment level of the sensor unit 110 to the patch 108, the attachment level of the patch 108 to the surface of the body to which the patch 108 is attached, etc. To illustrate, the light indicator 1340 may show, for example, when the sensor unit 110 is charging (e.g., with a solid orange light), fully charged (e.g., with a solid green light), or disconnected from the charger 107 (e.g., with no light). As another example, the sensor unit 110 may respond by blinking (e.g., via the light indicator 1340) or buzzing (e.g., via the sound indicator 1320) in response to an engagement by a patient to indicate possible symptoms.

In some embodiments, FIG. 13C provides an exploded view of the sensor unit 110 depicting at least some of the components of the sensor. For example, the sensor unit 110 may comprise a power source such as a battery 1380; a light indicator 1340; a button 1330 for facilitating the interaction of a patient, a healthcare provider, and/or a technician with the sensor unit 110; a wireless communications circuit 1385; a radio frequency shield 1390 (such as a metallic cover, e.g., to prevent interferences with the ECG processing and other digital circuitry); a digital circuitry board 1395; and/or the like. FIG. 13C shows a Bluetooth® unit as an example of a wireless communications circuit 1385, although in addition to or alternatively to the Bluetooth® unit, other modules facilitating other types of communications (examples of which including Wi-Fi, cellular, etc.) may be included in the sensor unit 110. In examples, the battery 1380 comprises a rechargeable lithium ion battery configured to supply power for at least one month of continuous ECG and biometric recording.

In some embodiments, the sensor unit 110 may also include input interfaces such as buttons for interfacing with a user. For example, the sensor may include a button 1330 that allows a patient or a health care professional to activate or deactivate the sensor unit 110. Such input interfaces may be configured to avoid or at least minimize unintended interactions with a user. For example, a button may be sized and shaped to avoid accidental activation (e.g., the button may be configured to require activation by being pushed in with an external object). This button may be used to reset the sensor as well as pair the sensor to the gateway device and initiate communication. As another example, an input interface of the sensor unit 110 may include a touchscreen configured to receive input from a user and/or provide information back to the user. In some embodiments, the input may allow the user to set the sensor in an "airplane mode," for example by deactivating any wireless communication (e.g., Wi-Fi, Bluetooth®, etc.) with external devices and/or servers. For example, the button can be implemented as a magnetic switch, e.g., an embedded magnetic switch, instead of a physical button. Such an implementation can be useful for designing the housing of the device and avoid exposing button components to the environment.

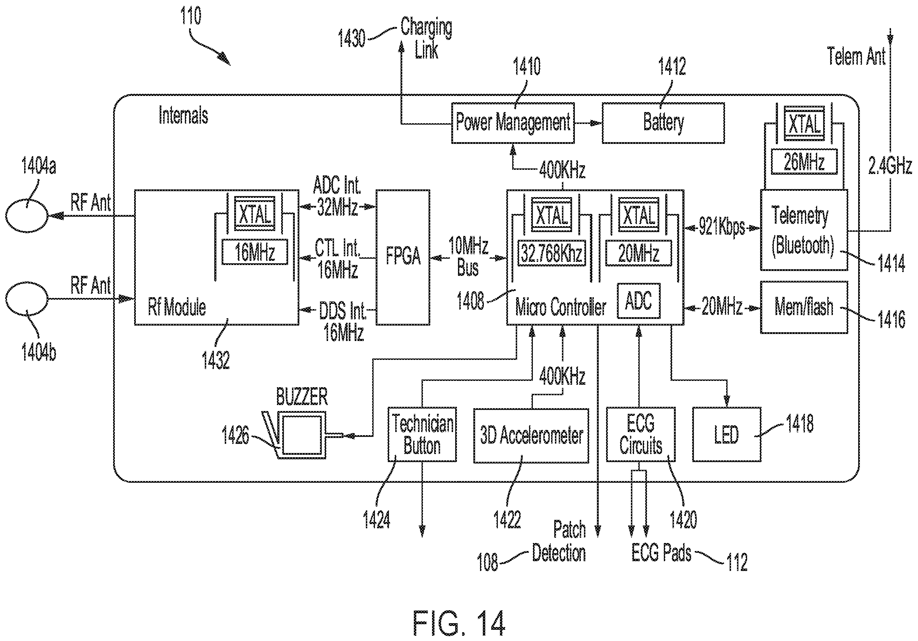

In some embodiments, as described above, the disclosed cardiac sensor 104 is configured to monitor and/or acquire data on physiological parameters including but not limited to ECG, heart rate, respiration rate, physical activity, posture, thoracic impedance, and/or the like. To that effect, the sensor unit 110 and/or the patch 108 housing the sensor unit 110 may include components that facilitate or undertake the monitoring and/or recording of at least some of these parameters. For example, as noted above, the patch 108 housing the sensor unit 110 may include ECG electrodes 112 coupled to the sensor unit 110 to facilitate the monitoring and/or acquiring of ECG data. As shown in FIG. 14, which shows an example embodiment of device electronics architecture for measurements and transmission of patient physiological data, the sensor unit 110 includes EGG processing circuitry configured to couple to the ECG electrodes 112 embedded in the patch 108 housing the sensor itself. The ECG processing circuitry is configured to, for example, perform filtering, amplification, and/or removal of noise, low frequency variations in the signal, and other signal artifacts.

With reference to FIG. 14, in some embodiments, the sensor unit 110 includes external interfaces, such as but not limited to, a button or switch 1424 for activating or deactivating the sensor unit 110, an LED 1418 and a buzzer 1426 for providing light and audio feedback to a user of the sensor unit 110, a battery charging link 1430 coupled to a power management module 1410 for charging an onboard power source such as a battery 1412, and ECG pads 1430 for recording synchronization signals. In some embodiments, the unit 110 may also include a wireless link (e.g., Bluetooth®) (not shown) to communicate, for example, with the portable gateway 106. In some embodiments, the sensor unit 110 may also include RF antennas (e.g., bi-static) 1404a, 1404b for transmitting and receiving RF signals, which may be used to determine thoracic impedance.

Internally, in some embodiments, the sensor unit 110 may include a microprocessor 1408 (which may be alternatively referred to as a micro-controller) that includes instructions thereon specifying how measurements (RF, ECG, accelerometer, etc.) are taken and the obtained data are transmitted, how to relay the status of the sensor unit 110, how/when the sensor unit 110 can enter a plurality of sleep levels, and/or the like. In some embodiments, the instructions may also specify the conditions for performing certain types of measurements. For example, the instructions may specify that an accelerometer of the sensor unit 110 may not commence measurements (e.g., for physical activity, patient posture, respiration rate, etc.) unless the user of the sensor unit 110 is at rest or maintaining a certain posture. As another example, the instructions may identify the conditions that may have to be fulfilled before ECG measurements can commence, such conditions including at least sufficient attachment level between the sensor and the surface on the body to which the sensor unit 110 is attached. In some embodiments, the microprocessor 1408 may have internal and external non-volatile memory banks that can be used for keeping measurement directory and data, scheduler information, and/or a log of actions and errors. This non-volatile memory allows saving power via a total power-down while retaining data and status information.

As discussed above, in various embodiments, the sensor unit 110 includes one or more motion sensors. In some implementations, the sensor unit 110 may include an accelerometer, and the accelerometer may be used to determine one or more of the physical activity, posture, respiration rate, etc. of a patient wearing the sensor unit 110, as discussed above. For example, a three-axis (3D) accelerometer 1422 may be used to acquire data on patient movements and posture as well as the respiration rate, and a processor (e.g., of the sensor unit 110, the portable gateway 106, and/or the remote server 102) receiving the acquired data may use the data (e.g., in conjunction with data obtained by the sensor such as ECG data) to determine biometric information of the patient. In some embodiments, the accelerometer 1422 may also contain an internal tap detector, which may be used for recording a patient-provided symptom input (e.g., using "double tap" feature). In some implementations, the sensor unit 110 may include more than one accelerometer, such as an accelerometer that measures patient posture and an accelerometer that measures patient respiration and activity. In some implementations, in addition to or in the alternative of the accelerometer 1422, the sensor unit 110 may include gyroscope(s), magnetometer(s), ballistocardiograph(s), and/or the like.

For example, in some implementations, the sensor unit 110 may include an RF module 1432 configured to transmit and receive RF signals via the RF antennas 1404a, 1404b. The RF module 1432 may communicate with a field-programmable gate array ("FPGA"), which may be configured to control a transceiver module, control analog-to-digital signal conversion, output samples, and the like. The RF signals may be processed to determine, for instance, lung fluid levels for the patient.

As discussed, FIG. 14 shows an example embodiment of a sensor unit 110 comprising a 3D accelerometer 1422, RF antennas 1404a, 1404b, ECG processing circuitry 1420 coupled to ECG electrodes 112, a microcontroller 1408 (which may be alternatively referred as microprocessor throughout this disclosure) and a telemetry (e.g., Bluetooth®) 1414. In such embodiments, for example, the micro-controller 1408 may receive data on patient respiration rate, movements, posture, ECG as well as RF-based measurements of the patient. The micro-controller 1408 may further process and/or transmit to an external processor, via the telemetry 1414, these measurements for further processing to determine a physiological parameter (e.g., ECG of the patient, biometric information) of the patient. As an example, the micro-controller 1408 of the sensor may cause the Bluetooth® telemetry 1414 to transmit the noted data and measurements to a remote server 102 which in turn analyzes the RF measurements, the ECG, posture, movement, and/or respiration rate data to determine biometric information for the patient, as discussed above. As another example, the remote server 102 may analyze ECG data to determine patient health conditions related to one or more of a heart rate, atrial fibrillation, flutter, supraventricular tachycardia, ventricular tachycardia, pause, AV block, ventricular fibrillation, bigeminy, trigemini, ventricular ectopic beats, supraventricular ectopic beats (SVEB), bradycardia, and tachycardia. Alternatively, as further discussed above, at least some of these determinations may be made at the cardiac sensor 104 (e.g., at the sensor unit 110) and/or the portable gateway 106, in some embodiments. Various signals acquired at the sensor unit 110 may also be stored (e.g., either short-term or long-term) in the sensor unit 110, such as within memory 1416. The micro-controller 1408 may also perform other functions, such as determining whether the sensor unit 110 has been correctly attached to the adhesive patch.

In some embodiments, the sensor unit 110 may also include other types of sensors, such as a temperature sensor, conductance sensor, a pressure sensor, a respiration sensor, SPO2 sensor, a light sensor, a humidity sensor, an altimeter, an anemometer, an air quality sensor, and/or a GPS sensor. For example, a respiration sensor can include an accelerometer configured to monitor the patient's chest movements, e.g., during certain portions of the day and/or night. For instance, a 3D multi-axis, multichannel accelerometer can be configured to, on a first channel, monitor for a patient movement and/or posture, and on a second, different channel, monitor the chest movements of the patient to determine respiration rate and other related data. Alternatively, a respiration accelerometer can be provided in the device that is separate from a posture sensing accelerometer. In some examples, the respiration rate measurement can be based on the operation of a tri-axis micro-electromechanical system (MEMS) accelerometer within the device mounted on the patient's torso. The accelerometer can measure projections of the gravity vector on its intrinsic axes. From these measurements, a respiration rate can be derived based on measured quasi-periodic changes of the projections that occur due to respiration movements of the patient's rib cage.

An alternative implementation of the cardiac monitoring device 100 is illustrated in FIG. 15. As shown in FIG. 15, in some implementations, the cardiac monitoring device 100 may include a cardiac sensor 104 that further includes individual electrodes 112 configured to be adhered to the patient's body. Each individual electrode 112 may be covered with a hydrogel for signal acquisition from the patient. The individual electrodes 112 may be in wired communication with the sensor unit 110 via cables 118, as shown. The sensor unit 110 may be worn, for example, on a belt of the patient via a belt clip attachment (not shown). In implementations, the sensor unit 110 may communicate with a portable gateway 106, which transmits signals from the sensor unit 110 to the remote server 102, similar to implementations discussed above. In implementations, the sensor unit 110 may communicate directly with the remote server 102, similar to implementations discussed above.

The individual electrodes 112 may be positioned on the patient in a configuration suited for acquiring ECG signals from the patient. For example, as illustrated in FIG. 15, the cardiac sensor 104 may include three electrodes 112, which two of the electrodes 112 positioned near either side of the patient's collarbone and the third electrode positioned lower on the patient's thorax. Additionally, in implementations, the cardiac sensor 104 may include one or more motion sensors in the sensor unit 110, similar to implementations of the cardiac sensor 104 including the adhesive patch 108 discussed above. In implementations, the cardiac sensor 104 may include one or more motion sensors connected to the individual electrodes 112. For instance, each electrode 112 may have a connected motion sensor, or a particular electrode 112 may have a connected motion sensor, such as the electrode 112 configured to be positioned lower on the patient's thorax in FIG. 15. In implementations, the cardiac sensor 104 may include one or more motion sensors connected to the cables 118.

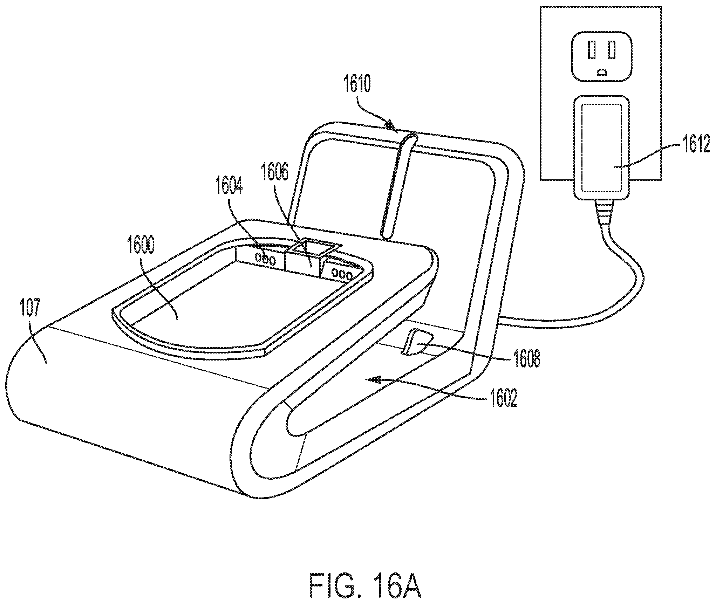

FIGS. 16A-16D illustrate a charger 107 that may be included as part of a cardiac monitoring device 100, according to some embodiments. For example, the charger 107 shown in FIGS. 16A-16D may be used in embodiments where the cardiac monitoring device 100 includes a sensor unit 110 and a portable gateway 106. FIG. 16A shows the charger 107 in an unused configuration. As shown in FIG. 16A, the charger 107 includes a power cord 1612 configured to plug into a standard outlet. For example, the charger 107 may be configured for an input of 100 to 240 VAC at 50/60 Hz. Once the charger 107 is properly connected to a power source via the power cord 1612, an indicator light 1610 may light up (e.g., may light blue when properly connected).

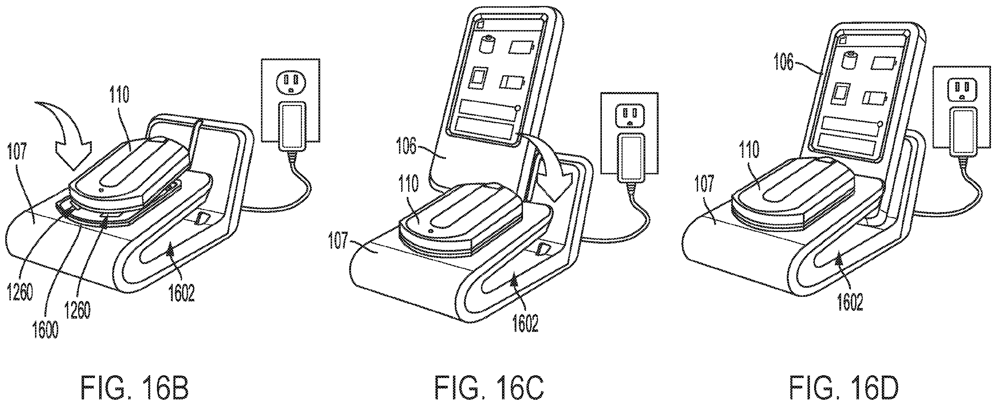

As illustrated, the charger 107 includes a sensor unit cradle 1600 configured to receive a sensor unit 110 (e.g., the sensor unit 110 shown in FIG. 12B). The sensor unit cradle 1600 may include metallic tabs 1604 configured to contact similar metallic tabs in the sensor unit 110 (e.g., metallic tabs 1325 shown in FIG. 13B) for the purpose of charging the sensor unit 110. FIG. 16B shows an illustration of inserting the sensor unit 110 into the sensor unit cradle 1600. As shown, the patient or other user inserting the sensor unit 110 tilts the lower part of the sensor unit 110 with the tabs (e.g., the positioning tabs 1260) on the bottom to help slide the sensor unit 110 into the sensor unit cradle 1600. The user then snaps the sensor unit 110 into place. For this reason, the sensor unit cradle 1600 may also include a clip receiver 1606 configured to receive a corresponding clip on the sensor unit 110 (e.g., clip 1270 shown in FIG. 12B). The clip receiver 1606 may help keep the sensor unit 110 in place in the sensor unit cradle 1600 by requiring the patient or other user to press down on the clip of the sensor unit 110 to remove the sensor unit 110 from the sensor unit cradle 1600. As discussed above, a light indicator on the sensor unit 110 (e.g., light indicator 1340) may show when the sensor unit 110 is properly positioned in the sensor unit cradle 1600 and charging, such as by turning an amber color to indicate charging. The light indicator may turn a second color when the sensor unit 110 is finished charging (e.g., the light indicator 1340 may turn green when the sensor unit 110 is fully charged, such as after about an hour of charging).

As shown in FIG. 16A, the charger 107 also includes a portable gateway cradle 1602 configured to receive the portable gateway 106. As such, the portable gateway cradle 1602 includes portable gateway supports 1608 configured to help hold the portable gateway 106 in place in the portable gateway cradle 1602. In implementations, the charger 107 includes a plug that the portable gateway 106 plugs into for charging. In implementations, the charger 107 is configured to wirelessly charge the portable gateway 106 (and, in some implementations, the sensor unit 110), such as via inductive charging. FIG. 16C shows an illustration of inserting the portable gateway 106 into the charger 107. The patient or other user may slide the portable gateway 106 into the portable gateway cradle 1602 as shown. As further shown in FIG. 16D, the portable gateway 106 and the sensor unit 110 may both charge in the charger 107 at the same time.

Although the subject matter contained herein has been described in detail for the purpose of illustration, such detail is solely for that purpose and that the present disclosure is not limited to the disclosed embodiments, but, on the contrary, is intended to cover modifications and equivalent arrangements that are within the scope of the appended claims. For example, it is to be understood that the present disclosure contemplates that, to the extent possible, one or more features of any embodiment can be combined with one or more features of any other embodiment.

certain functions described above can be implemented using software, hardware, firmware, hardwiring, or combinations of any of these. Features implementing functions can also be physically located at various positions, including being distributed such that portions of functions are implemented at different physical locations.

While various inventive embodiments have been described and illustrated herein, those of ordinary skill in the art will readily envision a variety of other means and/or structures for performing the function and/or obtaining the results and/or one or more of the advantages described herein. Those skilled in the art will readily appreciate that all parameters, dimensions, materials, and configurations described herein are meant to be an example and that the actual parameters, dimensions, materials, and/or configurations will depend upon the specific application or applications for which the inventive teachings is/are used.

Also, various inventive concepts may be embodied as one or more methods, of which an example has been provided. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

**Claims**

1. A cardiac monitoring system (100) for displaying contextual biometric information for arrhythmia events occurring in a patient, comprising:

   a cardiac sensor (104) configured to be coupled to the patient, the cardiac sensor comprising

   a plurality of ECG electrodes (112) configured to sense ECG signals of the patient; and
   a motion sensor configured to acquire motion signals associated with the patient,

   wherein the motion signals comprise motion information and/or posture information of the patient;
   wherein the cardiac sensor (104) is further configured to transmit the ECG signals and motion signals to a remote server (102); and
   the remote server in communication with the cardiac sensor (104) and further in communication with a user interface (114, 116), the remote server (102) comprising

   a database implemented in a non-transitory media; and
   a processor in communication with the database; the processor configured to

store the ECG signals and the motion signals in the database,
identify an arrhythmia event based on the received ECG signals wherein the processor is configured to determine the arrhythmia event based on the received ECG signals by determining an onset of the arrhythmia event based on the received ECG signals,
determine an arrhythmia contextual time period around the onset of the arrhythmia event,
determine, based on the motion signals, contextual biometric information of the patient for the arrhythmia event during the arrhythmia contextual time period, and
display, on the user interface (114, 116), the arrhythmia event overlaid on the contextual biometric information of the patient by displaying on the user interface

a graphical timeline comprising the arrhythmia contextual time period around the onset of the arrhythmia event;
a biometric graphical representation of the contextual biometric information of the patient during the arrhythmia contextual time period; and
an arrhythmia onset graphical indicator corresponding to the onset of the arrhythmia event, wherein the arrhythmia onset graphical indicator is overlaid on the biometric graphical representation.

2. The cardiac monitoring system (100) of claim 1, wherein the processor is further configured to

determine an offset of the arrhythmia event based on the received ECG signals; and
display the arrhythmia event overlaid on the contextual biometric information of the patient by further displaying on the user interface (114, 116)

an arrhythmia offset graphical indicator corresponding to the offset of the arrhythmia event,
wherein the arrhythmia offset graphical indicator is overlaid on the contextual biometric information.

3. The cardiac monitoring system (100) of claim 2, wherein the arrhythmia contextual time period comprises at least one of: a two hour time period during which the offset of the arrhythmia event occurred, an hour time period during which the offset of the arrhythmia event occurred, a 30 minute time period during which the offset of the arrhythmia event occurred, a 15 minute time period during which the offset of the arrhythmia event occurred, or a 10 minute time period during which the offset of the arrhythmia event occurred; and
wherein the arrhythmia contextual time period comprises at least one of: a two hour time period during which the onset of the arrhythmia event occurred, an hour time period during which the onset of the arrhythmia event occurred, a 30 minute time period during which the onset of the arrhythmia event occurred, a 15 minute time period during which the onset of the arrhythmia event occurred, or a 10 minute time period during which the onset of the arrhythmia event occurred.

4. The cardiac monitoring system (100) of claim 1, wherein the cardiac sensor (104) further comprises

a patch (108), the plurality of ECG electrodes (112) disposed on the patch; and
a sensor unit (110) configured to be removably attached to the patch and in electrical communication with the plurality of ECG electrodes, the sensor unit comprising the motion sensor.

5. The cardiac monitoring system (100) of claim 1, wherein the contextual biometric information is indicative of a posture of the patient: and
wherein the processor is configured to display the arrhythmia event overlaid on the contextual biometric information of the patient by displaying changes in the patient's posture.

6. The cardiac monitoring system (100) of claim 5, wherein the changes in the patient's posture comprise changes in a degree of the patient's posture; and/or

wherein the changes in the patient's posture comprise changes in a type of the patient's posture;
wherein the types of the patient's posture optionally comprise at least two of: supine, reclined, upright, standing, sitting, resting, lying on a right side, or lying on a left side.

7. The cardiac monitoring system (100) of claim 1, wherein the contextual biometric information comprises an activity level of the patient;
wherein the processor is configured to display the arrhythmia event overlaid on the contextual biometric information of the patient by displaying changes in the patient's activity level.

8. The cardiac monitoring system (100) of claim 7, wherein the changes in the patient's activity level comprise changes in a type of the patient's activity level; and, optionally, wherein the types of the patient's activity level comprise at least two of: active, non-active, walking, low activity, intermediate activity, or high activity.

9. The cardiac monitoring system (100) of claim 7, wherein the contextual biometric information is further indicative of heart rate recovery of the patient.

10. The cardiac monitoring system (100) of claim 1, wherein the contextual biometric information is indicative of respiration of the patient; and
   wherein the processor is configured to display the arrhythmia event overlaid on the contextual biometric information of the patient by displaying changes in the patient's respiration.

11. The cardiac monitoring system (100) of claim 10, wherein the changes in the patient's respiration comprise changes in a type of the patient's respiration; wherein the types of the patient's respiration comprise at least two of: regular breathing, rapid breathing, shallow breathing, rapid shallow breathing, deep breathing, wheezing, sleep disordered breathing, or Cheyne-Stokes respiration.

12. The cardiac monitoring system (100) of claim 10, wherein the processor is configured to display the arrhythmia event overlaid on the contextual biometric information of the patient by further displaying when the patient's respiration rate crosses a respiration rate threshold.

13. The cardiac monitoring system (100) of claim 1, wherein the contextual biometric information is indicative of a sleep status of the patient; and wherein the processor is configured to display the arrhythmia event overlaid on the contextual biometric information of the patient by displaying changes in the patient's sleep status.

14. The cardiac monitoring system (100) of claim 13, wherein the contextual biometric information further comprises a posture of the patient, an activity level of the patient, and respiration of the patient, and wherein the processor is further configured to
   determine the patient's sleep status based on a combination of at least two of: the posture of the patient, the activity level of the patient, or the respiration of the patient.

15. The cardiac monitoring system (100) of claim **1,** wherein the processor is further configured to

   determine an arrhythmia type for the arrhythmia event; and
   determine the arrhythmia contextual time period based on the arrhythmia type for the arrhythmia event; or
   determine a biometric status of the patient at the onset of the arrhythmia event; and
   determine the arrhythmia contextual time period based on the biometric status of the patient at the onset of the arrhythmia event.

**Patentansprüche**

1. Herzüberwachungssystem (100) zur Anzeige kontextbezogener biometrischer Informationen für Arrhythmieereignisse, die bei einem Patienten auftreten, umfassend:
   einen Herzsensor (104), der konfiguriert ist, um mit dem Patienten gekoppelt zu werden, der Herzsensor umfassend:

   eine Vielzahl von EKG-Elektroden (112), die konfiguriert sind, um EKG-Signale des Patienten zu erfassen; und
   einen Bewegungssensor, der konfiguriert ist, um mit dem Patienten verbundene Bewegungssignale zu erfassen, wobei die Bewegungssignale Bewegungsinformationen und/oder Haltungsinformationen des Patienten umfassen;
   wobei der Herzsensor (104) ferner konfiguriert ist, um die EKG-Signale und Bewegungssignale an einen Fernserver (102) zu übertragen; und
   wobei sich der Fernserver in Kommunikation mit dem Herzsensor (104) und ferner in Kommunikation mit einer Benutzerschnittstelle (114, 116) befindet, der Fernserver (102) umfassend
   eine Datenbank, die in einem nicht transitorischen Medium implementiert ist; und
   einen Prozessor, der mit der Datenbank kommuniziert; der Prozessor konfiguriert ist zum
   Speichern der EKG-Signale und der Bewegungssignale in der Datenbank,
   Identifizieren eines Arrhythmieereignisses basierend auf den empfangenen EKG-Signalen, wobei der Prozessor konfiguriert ist, um das Arrhythmieereignis basierend auf den empfangenen EKG-Signalen zu bestimmen, indem er einen Beginn des Arrhythmieereignisses basierend auf den empfangenen EKG-Signalen bestimmt, Bestimmen eines Arrhythmiekontextzeitraums um das Einsetzen des Arrhythmieereignisses herum, Bestimmen, basierend auf den Bewegungssignalen, kontextbezogener biometrischer Informationen des Patienten für das Arrhythmieereignis während des kontextbezogenen Zeitraums der Arrhythmie, und Anzeigen des Arrhythmieereignisses auf der Benutzerschnittstelle (114, 116), überlagert mit den kontextbezogenen biometrischen Informationen des Patienten durch Anzeigen auf der Benutzerschnittstelle einer grafischen Zeitleiste, die die kontextbezogene Zeitspanne um den Beginn des Arrhythmieereignisses umfasst; einer biometrischen grafischen Darstellung der kontextbezogenen biometrischen Informationen des Patienten während der kontextbezogenen Zeitspanne der Arrhythmie; und eines grafischen Arrhythmieereignisindikators, der dem Beginn des Arrhythmieereignisses entspricht, wobei der grafische Arrhythmieereignisindikator der biometrischen grafischen Darstellung überlagert ist.

2. Herzüberwachungssystem (100) nach Anspruch 1, wobei der Prozessor ferner konfiguriert ist zum Bestimmen eines Versatzes des Arrhythmieereignisses basierend auf den empfangenen EKG-Signalen; und

Anzeigen des Arrhythmieereignisses, überlagert mit den kontextbezogenen biometrischen Informationen des Patienten, durch weiteres Anzeigen auf der Benutzerschnittstelle (114, 116) eines grafischen Arrhythmieversatzindikators, der dem Versatz des Arrhythmieereignisses entspricht, wobei der grafische Arrhythmieversatzindikator den kontextbezogenen biometrischen Informationen überlagert wird.

3. Herzüberwachungssystem (100) nach Anspruch 2, wobei der kontextbezogene Arrhythmiezeitraum mindestens eines von Folgendem umfasst: einen zweistündigen Zeitraum, in dem der Versatz des Arrhythmieereignisses aufgetreten ist, einen einstündigen Zeitraum, in dem der Versatz des Arrhythmieereignisses aufgetreten ist, einen 30-minütigen Zeitraum, in dem der Versatz des Arrhythmieereignisses aufgetreten ist, einen 15-minütigen Zeitraum, in dem der Versatz des Arrhythmieereignisses aufgetreten ist, oder einen 10-minütigen Zeitraum, in dem der Versatz des Arrhythmieereignisses aufgetreten ist; und wobei der kontextbezogene Arrhythmiezeitraum mindestens eines von Folgendem umfasst: einen zweistündigen Zeitraum, in dem das Auftreten des Arrhythmieereignisses stattfand, einen einstündigen Zeitraum, in dem das Auftreten des Arrhythmieereignisses stattfand, einen 30-minütigen Zeitraum, in dem das Auftreten des Arrhythmieereignisses stattfand, einen 15-minütigen Zeitraum, in dem das Auftreten des Arrhythmieereignisses stattfand, oder einen 10-minütigen Zeitraum, in dem das Auftreten des Arrhythmieereignisses stattfand.

4. Herzüberwachungssystem (100) nach Anspruch 1,

wobei der Herzsensor (104) ferner Folgendes umfasst ein Pflaster (108), wobei die Vielzahl von EKG-Elektroden (112) auf dem Pflaster angeordnet ist; und eine Sensoreinheit (110), die konfiguriert ist, um abnehmbar an dem Pflaster befestigt zu werden und in elektrischer Verbindung mit der Vielzahl von EKG-Elektroden zu stehen, wobei die Sensoreinheit den Bewegungssensor umfasst.

5. Herzüberwachungssystem (100) nach Anspruch 1, wobei die kontextbezogene biometrische Information eine Haltung des Patienten anzeigt; und wobei der Prozessor konfiguriert ist, um das Arrhythmieereignis über die kontextuellen biometrischen Informationen des Patienten zu legen, indem er Veränderungen in der Haltung des Patienten anzeigt.

6. Herzüberwachungssystem (100) nach Anspruch 5, wobei die Veränderungen in der Körperhaltung des Patienten Veränderungen in einem Grad der Haltung des Patienten umfassen; und/oder

wobei die Veränderungen in der Haltung des Patienten Veränderungen in einem Typ der Körperhaltung des Patienten umfassen; wobei die Haltungen des Patienten optional mindestens zwei von Folgendem umfassen: Rückenlage, Liegeposition, aufrechte Haltung, Stehen, Sitzen, Ruhen, Liegen auf der rechten Seite oder Liegen auf der linken Seite.

7. Herzüberwachungssystem (100) nach Anspruch 1, wobei die kontextbezogene biometrische Information ein Aktivitätsniveau des Patienten umfasst; wobei der Prozessor konfiguriert ist, um das Arrhythmieereignis über die kontextbezogenen biometrischen Informationen des Patienten zu legen, indem er Veränderungen im Aktivitätsniveau des Patienten anzeigt.

8. Herzüberwachungssystem (100) nach Anspruch 7, wobei die Veränderungen des Aktivitätsniveaus des Patienten Veränderungen eines Typs des Aktivitätsniveaus des Patienten umfassen; und optional, wobei die Typen des Aktivitätsniveaus des Patienten mindestens zwei von Folgendem umfassen: aktiv, nicht aktiv, Gehen, geringe Aktivität, mittlere Aktivität oder hohe Aktivität.

9. Herzüberwachungssystem (100) nach Anspruch 7, wobei die kontextbezogenen biometrischen Informationen ferner auf die Herzfrequenzerholung des Patienten hinweisen.

10. Herzüberwachungssystem (100) nach Anspruch 1, wobei die kontextbezogene biometrische Information die Atmung des Patienten anzeigt; und
wobei der Prozessor konfiguriert ist, um das Arrhythmieereignis über die kontextuellen biometrischen Informationen des Patienten zu legen, indem er Änderungen in der Atmung des Patienten anzeigt.

11. Herzüberwachungssystem (100) nach Anspruch 10, wobei die Veränderungen in der Atmung des Patienten Veränderungen in einem Typ der Atmung des Patienten umfassen; wobei die Typen der Atmung des Patienten mindestens zwei von Folgendem umfassen: regelmäßige Atmung, schnelle Atmung, flache Atmung, schnelle flache Atmung, tiefe Atmung, Keuchen, schlafgestörte Atmung oder Cheyne-Stokes-Atmung.

12. Herzüberwachungssystem (100) nach Anspruch 10, wobei der Prozessor konfiguriert ist, um das Arrhythmieereignis über den kontextbezogenen biometrischen Informationen des Patienten anzuzeigen, indem ferner angezeigt wird, wenn die Atemfrequenz des Patienten einen Atemfrequenzschwellenwert überschreitet.

13. Herzüberwachungssystem (100) nach Anspruch 1, wobei die kontextbezogenen biometrischen Informationen einen Schlafstatus des Patienten anzeigen; und wobei der Prozessor konfiguriert ist, um das Arrhythmieereignis überlagert mit den kontextbezogenen biometrischen Informationen des Patienten anzuzeigen, indem Veränderungen im Schlafstatus des Patienten angezeigt werden.

14. Herzüberwachungssystem (100) nach Anspruch 13, wobei die kontextbezogenen biometrischen Informationen ferner eine Haltung des Patienten, ein Aktivitätsniveau des Patienten und die Atmung des Patienten umfassen, und wobei der Prozessor ferner konfiguriert ist zum
Bestimmen des Schlafstatus des Patienten basierend auf einer Kombination von mindestens zwei von Folgendem: der Haltung des Patienten, dem Aktivitätsniveau des Patienten oder der Atmung des Patienten.

15. Herzüberwachungssystem (100) nach Anspruch 1, wobei der Prozessor ferner konfiguriert ist zum:

Bestimmen eines Arrhythmietyps für das Arrhythmieereignis; und
Bestimmen des kontextbezogenen Arrhythmiezeitraums basierend auf dem Arrhythmietyp für das Arrhythmieereignis; oder
Bestimmen eines biometrischen Status des Patienten beim Auftreten des Arrhythmieereignisses; und
Bestimmen des kontextbezogenen Arrhythmiezeitraums basierend auf dem biometrischen Status des Patienten beim Auftreten des Arrhythmieereignisses.

**Revendications**

1. Système de surveillance cardiaque (100) permettant l'affichage d'informations biométriques contextuelles pour des événements d'arythmie survenant chez un patient, comprenant :

un capteur cardiaque (104) configuré pour être couplé au patient, le capteur cardiaque comprenant
une pluralité d'électrodes ECG (112) configurées pour détecter les signaux ECG du patient ; et
un capteur de mouvement configuré pour acquérir des signaux de mouvement associés au patient, dans lequel les signaux de mouvement comprennent des informations sur le mouvement et/ou des informations sur la posture du patient ;
dans lequel le capteur cardiaque (104) est en outre configuré pour transmettre les signaux ECG et les signaux de mouvement à un serveur distant (102) ; et
le serveur distant en communication avec le capteur cardiaque (104) et en outre en communication avec une interface utilisateur (114, 116), le serveur distant (102) comprenant

une base de données implémentée sur un support non transitoire ; et

un processeur en communication avec la base de données ; le processeur configuré pour

stocker les signaux ECG et les signaux de mouvement dans la base de données,

identifier un événement d'arythmie sur la base des signaux ECG reçus dans lequel le processeur est configuré pour déterminer l'événement d'arythmie sur la base des signaux ECG reçus en déterminant l'apparition de l'événement d'arythmie sur la base des signaux ECG reçus, déterminer une période de temps contextuelle de l'arythmie autour de l'apparition de l'événement d'arythmie,

déterminer, sur la base des signaux de mouvement, les informations biométriques contextuelles du patient pour l'événement d'arythmie pendant la période de temps contextuelle de l'arythmie, et

afficher, sur l'interface utilisateur (114, 116), l'événement d'arythmie superposé aux informations biométriques contextuelles du patient en affichant sur l'interface utilisateur

une chronologie graphique comprenant la période de temps contextuelle de l'arythmie autour de l'apparition de l'événement d'arythmie ;

une représentation graphique biométrique des informations biométriques contextuelles du patient pendant la période de temps contextuelle de l'arythmie ; et

un indicateur graphique d'apparition de l'arythmie correspondant à l'apparition de l'événement d'arythmie, dans lequel l'indicateur graphique d'apparition de l'arythmie est superposé à la représentation graphique biométrique.

2. Système de surveillance cardiaque (100) selon la revendication 1, dans lequel le processeur est en outre configuré pour

déterminer un décalage de l'événement d'arythmie sur la base des signaux ECG reçus ; et

afficher l'événement d'arythmie superposé aux informations biométriques contextuelles du patient en affichant en outre sur l'interface utilisateur (114, 116) un indicateur graphique de décalage de l'arythmie correspondant au décalage de l'événement d'arythmie, dans lequel l'indicateur graphique de décalage de l'arythmie est superposé aux informations biométriques contextuelles.

3. Système de surveillance cardiaque (100) selon la revendication 2, dans lequel la période de temps contextuelle de l'arythmie comprend au moins un élément parmi : une période de temps de deux heures pendant laquelle le décalage de l'événement d'arythmie s'est produit, une période de temps d'une heure pendant laquelle le décalage de l'événement d'arythmie s'est produit, une période de temps de 30 minutes pendant laquelle le décalage de l'événement d'arythmie s'est produit, une période de temps de 15 minutes pendant laquelle le décalage de l'événement d'arythmie s'est produit, ou une période de temps de 10 minutes pendant laquelle le décalage de l'événement d'arythmie s'est produit ; et

dans lequel la période de temps contextuelle de l'arythmie comprend au moins un élément parmi : une période de temps de deux heures pendant laquelle l'apparition de l'arythmie s'est produite, une période de temps d'une heure pendant laquelle l'apparition de l'arythmie s'est produite, une période de temps de 30 minutes pendant laquelle l'apparition de l'arythmie s'est produite, une période de temps de 15 minutes pendant laquelle l'apparition de l'arythmie s'est produite, ou une période de temps de 10 minutes pendant laquelle l'apparition de l'arythmie s'est produite.

4. Système de surveillance cardiaque (100) selon la revendication 1, dans lequel le capteur cardiaque (104) comprend en outre

un patch (108), la pluralité d'électrodes ECG (112) disposée sur le patch ; et

une unité de capteur (110) configurée pour être fixé de manière amovible au patch et en communication électrique avec la pluralité d'électrodes ECG, l'unité de capteur comprenant le détecteur de mouvement.

5. Système de surveillance cardiaque (100) selon la revendication 1, dans lequel les informations biométriques contextuelles sont indicatives d'une posture du patient ; et

dans lequel le processeur est configuré pour afficher l'événement d'arythmie superposé aux informations biométriques contextuelles du patient en affichant les changements dans la posture du patient.

6. Système de surveillance cardiaque (100) selon la revendication 5, dans lequel les changements dans la posture du patient comprennent des changements dans le degré de la posture du patient ; et/ou

dans lequel les changements dans la posture du patient comprennent des changements dans un type de posture du patient ;

dans lequel les types de posture du patient comprennent éventuellement au moins deux éléments parmi : allongé sur le dos, incliné, redressé, debout, assis, au repos, couché sur le côté droit ou couché sur le côté gauche.

7. Système de surveillance cardiaque (100) selon la revendication 1, dans lequel les informations biométriques contextuelles comprennent un niveau d'activité du patient ;

dans lequel le processeur est configuré pour afficher l'événement d'arythmie superposé aux informations biométriques contextuelles du patient en affichant les changements dans le niveau d'activité du patient.

8. Système de surveillance cardiaque (100) selon la revendication 7, dans lequel les changements dans le niveau d'activité du patient comprennent des changements dans un type de niveau d'activité du patient ; et, éventuellement, dans lequel les types de niveau d'activité du patient comprennent au moins deux éléments parmi : actif, non actif, marche, activité faible, activité intermédiaire ou activité élevée.

9. Système de surveillance cardiaque (100) selon la revendication 7, dans lequel les informations biométriques contextuelles sont en outre indicatives de la récupération de la fréquence cardiaque du patient.

10. Système de surveillance cardiaque (100) selon la revendication 1, dans lequel les informations biométriques contextuelles sont indicatives de la respiration du patient ; et

dans lequel le processeur est configuré pour afficher l'événement d'arythmie superposé aux informations biométriques contextuelles du patient en affichant les changements dans la respiration du patient.

11. Système de surveillance cardiaque (100) selon la revendication 10, dans lequel les changements dans la respiration du patient comprennent des changements dans un type de respiration du patient ; dans lequel les types de respiration du patient comprennent au moins deux éléments parmi : respiration régulière, respiration rapide, respiration superficielle, respiration superficielle rapide, respiration profonde, respiration sifflante, troubles respiratoires du sommeil ou respiration de Cheyne-Stokes.

12. Système de surveillance cardiaque (100) selon la revendication 10, dans lequel le processeur est configuré pour afficher l'événement d'arythmie superposé aux informations biométriques contextuelles du patient en affichant en outre le moment où la fréquence respiratoire du patient dépasse un seuil de fréquence respiratoire.

13. Système de surveillance cardiaque (100) selon la revendication 1, dans lequel les informations biométriques contextuelles sont indicatives d'un état de sommeil du patient ; et dans lequel le processeur est configuré pour afficher l'événement d'arythmie superposé aux informations biométriques contextuelles du patient en affichant les changements dans l'état de sommeil du patient.

14. Système de surveillance cardiaque (100) selon la revendication 13, dans lequel les informations biométriques contextuelles comprennent en outre une posture du patient, un niveau d'activité du patient et la respiration du patient, et dans lequel le processeur est en outre configuré pour

déterminer l'état de sommeil du patient sur la base d'une combinaison d'au moins deux éléments parmi : la posture du patient, le niveau d'activité du patient ou la respiration du patient.

15. Système de surveillance cardiaque (100) selon la revendication 1, dans lequel le processeur est en outre configuré pour

déterminer un type d'arythmie pour l'événement d'arythmie ; et
déterminer la période de temps contextuelle de l'arythmie sur la base du type d'arythmie pour l'événement d'arythmie ; ou
déterminer l'état biométrique du patient au moment de l'apparition de l'arythmie ; et
déterminer la période de temps contextuelle de l'arythmie sur la base de l'état biométrique du patient au moment de l'apparition de l'arythmie.

FIG. 1

*200*

Cardiac monitoring device —100

Remote server —102

**202**

Sense ECG signals

**204**

Acquire motion signals

**206**

Transmit the sensed ECG signals and acquired motion signals

**208**

Store the ECG signals and motion signals

**210**

Determine onset of arrhythmia event

**212**

Determine arrhythmia contextual time period

**214**

Determine contextual biometric information for arrhythmia contextual time period

**216**

Display arrhythmia report

FIG. 2A

FIG. 2B

212

Identify onset of arrhythmia event — 240

Identify period of time for arrhythmia contextual time period — 242

Use period of time to identify arrhythmia contextual time period — 244

FIG. 2C

214

250

Retrieve stored motion signals

252

254

Filter motion signals

| 250a | 254b | 254c | 254d |

| Determine posture information | Determine activity information | Determine respiration information | Determine sleep information | ... |

256

Determine biometric information over arrhythmia contextual time period

FIG. 2D

FIG. 2E

216

Display graphical timeline — 295

↓

Display biometric graphical representation — 297

↓

Display arrhythmia onset graphical indicator
overlaid on biometric graphical rep. — 299

FIG. 2F

FIG. 2G

*300*

Cardiac monitoring device — 100

Remote server — 102

302

Sense ECG signals

↓

304

Acquire motion signals

↓

306

Determine onset of arrhythmia event

↓

308

Determine biometric information

↓

310

Transmit the onset, ECG signals corresponding to arrhythmia event, and biometric information

312

Store the onset, ECG signals corresponding to arrhythmia event, and biometric information

↓

314

Determine arrhythmia contextual time period

↓

316

Determine contextual biometric information for arrhythmia contextual time period

↓

318

Display arrhythmia report

FIG. 3

400

402

Patient: Smith, Joe (M), 57
DOB: 12/01/1960 ID: 12345
Phone: (415) 555-1212

404

EVENT REPORT - 05/14/2020

| PHYSICIAN INFORMATION | | PRESCRIPTION SUMMARY | |
|---|---|---|---|
| **Ordering Physician:** Demo Prescriber | **Physician's Phone:** (412) 555-1214 | **Enrollment Date:** 05/07/2019 | **Reason for Order:** R00.2 |
| **Facility:** ABC Cardiology | **Physician's Address:** 121 Gamma Drive Pittsburgh, Pennsylvania | **Duration:** 30 Days **End Date:** 06/06/2019 | |

406

TRENDS

408

414

408a

410

FIG. 4A

EP 4 258 984 B1

FIG. 4B

420

422

**Patient:** Hernandez, Jose (M), 67
**DOB:** 05/10/1949 **ID:** 000635
**Phone:** (555) 555-5555

424

| DAILY REPORT - 06/06/2016 |
|---|

426

| PHYSICIAN INFORMATION | | PRESCRIPTION SUMMARY | |
|---|---|---|---|
| **Ordering Physician:** Dr. Mario Demo DemoPrescriber<br>**Facility:** ZOLL Lab Services | **Physician's Phone:** (555) 555-5555<br>**Physician's Address:**<br>121 Gamma Drive<br>Pittsburgh, Pennsylvania | **Enrollment Date:** 05/23/2016<br>**Duration:** Day 15 of 30<br>**End Date:** 06/21/2016 | **Reason for Order:** 148.91, R00.2<br>**Time Monitored:** 23 hr, 59 min |

| TRENDS |
|---|

428

428a

Night Time Heart Rate (bpm)

05/17   05/19   05/21   05/23   05/25   05/27   05/29   05/31   06/02   06/04   06/05

100

50

0

FIG. 4C

o Denotes median night time value, displayed for Respiration Rate, Sleep Posture, and Heart Rate
▲ Denotes maximum night time value
▼ Denotes minimum night time value
Activity is total hours active per day

FIG. 4D

EP 4 258 984 B1

FIG. 5

FIG. 6A  FIG. 6B  FIG. 6C

**FIG. 6A**
9:41 AM
600
602 — Sensor  100%
604 — Record Event
Gateway  100%

**FIG. 6B**
606 — How Did You Feel?  ✕
608 — September 29 at 05:38 PM
○ Light Headed
○ My Heart Racing
○ Fatigued
○ I Fainted/Fell
○ Chest Discomfort
○ Heart Skipped a Beat
○ Shortness of Breath
○ Other
610
612 — Next

**FIG. 6C**
614 — ← How Active Were You?  ✕
616 — September 29 at 05:38 PM
○ Resting
○ Slightly Active
○ Moderately Active
○ Vigorously Active
618
620 — Save

72

FIG. 7

700

Cardiac monitoring device — 100

702 — Sense ECG signals

704 — Acquire motion signals

706 — Receive patient-provided symptom input

708 — Determine biometric information

710 — Transmit the biometric information, symptom input, and ECG signals corresponding to the symptom input

Remote server — 102

712 — Store the biometric information, symptom input, and ECG signals corresponding to the symptom input

714 — Determine symptom contextual time period

716 — Determine contextual biometric information

718 — Display symptom report

EP 4 258 984 B1

73

**FIG. 8A**

Patient: Smith, Joe (M), 57
DOB: 12/01/1960 ID: 12345
Phone: (415) 555-1212

EVENT REPORT - 05/14/2020

| PHYSICIAN INFORMATION | | PRESCRIPTION SUMMARY | |
|---|---|---|---|
| Ordering Physician: Demo Prescriber | Physician's Phone: (412) 555-1214 | Enrollment Date: 05/07/2019 | Reason for Order: R00.2 |
| Facility: ABC Cardiology | Physician's Address: 121 Gamma Drive Pittsburgh, Pennsylvania | Duration: 30 Days End Date: 06/06/2019 | |

TRENDS

EP 4 258 984 B1

Respiration Rate
(Breaths per min)

808b

Body Posture
(Degrees)

808c

Activity
(Total Sec/Min Active)

808d

Time of Day

FIG. 8B

810

o denotes one minute average
Respiration Rate not reported during periods of activity

**Patient:** Smith, Joe (M), 57
822 **DOB:** 12/01/1960 **ID:** 12345
**Phone:** (415) 555-1212

824

| EVENT REPORT - 05/14/2020 |
|---|

| PHYSICIAN INFORMATION | | PRESCRIPTION SUMMARY | |
|---|---|---|---|
| **Ordering Physician:** Demo Prescriber<br>**Facility:** ABC Cardiology | **Physician's Phone:** (412) 555-1214<br>**Physician's Address:**<br>121 Gamma Drive<br>Pittsburgh, Pennsylvania | **Enrollment Date:** 05/07/2019<br>**Duration:** 30 Days<br>**End Date:** 06/06/2019 | **Reason for Order:** R00.2 |

826

828

| TRENDS |
|---|

9:31 am Patient-triggered event: Normal Sinus Rhythm with PAC

832

828a — Heart Rate (Bpm)

828b — Respiration Rate (Breaths per min)

**FIG. 8C**

830

o Denotes one minute average

* Respiration Rate not reported during periods of activity

** Upright Activity is derived from the device-determined parameters posture (≥35°) and activity (≥30 seconds per minute active).

*** Sleep is derived from the device-determined parameters posture (<35°) and activity (<12 seconds per minute active).

FIG. 8D

FIG. 9A

**900**

Cardiac monitoring device ─ 100

902 ─ Sense ECG signals

904 ─ Acquire motion signals

906 ─ Transmit the sensed ECG signals and acquired motion signals

Remote server ─ 102

Store the ECG signals, motion signals, and patient-provided symptom inputs ─ 908

Determine arrhythmia events ─ 910

Determine biometric context summary ─ 912

Present summary view of arrhythmia events and/or symptom inputs ─ 914

EP 4 258 984 B1

912

920 — Retrieve stored motion signals

922 — Filter motion signals

924

924a — Determine posture information

924b — Determine activity information

924c — Determine respiration information

924d — Determine sleep information

• • •

926 — Determine biometric context summary from biometric information

FIG. 9B

914

930 — Present information about each arrhythmia event and/or symptom input

932 — Present information about biometric context summary

FIG. 9C

FIG. 10

**Patient:** Smith, Joe (M), 57
**DOB:** 12/01/1960  **ID:** 12345
**Phone:** (415) 555-1212

1102

1104

1100

08

1106

### HEALTH & WELLNESS

**ACTIVITY**
  Avg 7 hours active per day / Max 9 hours / Min 1 hour
  Atrial Fibrillation (70), SVT (3), AVB (1) events detected
  during activity
  10 symptomatic events during activity: Fatigue (7), Shortness
  of breath (3)

**SLEEP**
  Avg 5.5 hours sleep per day / Max 7 hours / Min 1 hour
  Atrial Fibrillation (30), AVB (1) events detected during sleep
  3 symptomatic events during sleep: Palpitations (3)

**Heart Rate**
  Maximum                  178 bpm (at 11:22a on 8/20)
  Minimum                  48 bpm (at 2:01a on 8/19)

**Respiration Rate**
  Maximum                  26 breaths per min
  Minimum                  9 breaths per min

**Symptoms**
  Fatigue (10), Shortness of breath (3), Palpitations (3)

ACTIVITY — 1108a

Onsets

SLEEP — 1108b

Onsets

EP 4 258 984 B1

## FIG. 11A

*Upright activity is derived from the device-determined parameters posture (≥35°) and activity (≥30 seconds per minute active).

**Sleep is derived from the device-determined parameters posture (<35°) and activity (<12 seconds per minute active). Sleep time is between 9pm - 9am.

FIG. 11B

EP 4 258 984 B1

EP 4 258 984 B1

1100

1110

1102

**Patient:** Smith, Joe (M), 57
**DOB:** 12/01/1960  **ID:** 12345
**Phone:** (415) 555-1212

1110a

Activity — Total Hours of Activity per Day

Hours — 8, 6, 4, 2, 0

| | Ventricular Tachycardia | Atrial Fibrillation | SVT | Pause | 2nd/3rd Degree AVB | Symptomatic Event | PVCs | PACs, PJCs |
|---|---|---|---|---|---|---|---|---|

Dates: 8/20 8/21 8/22 8/23 8/24 8/25 8/26 8/27 8/28 8/29 8/30 8/31  9/1 9/2 9/3 9/4 9/5 9/6 9/7 9/8 9/9 9/10 9/11 9/12 9/13 9/14 9/15 9/16 9/17 9/18

*Each dot in the graph represents at least one episode onset

FIG. 11C

**Sleep** — Total Hours of Sleep per Night

1110b

Hours — 10, 8, 6, 4, 2, 0

| | 8/20 | 8/21 | 8/22 | 8/23 | 8/24 | 8/25 | 8/26 | 8/27 | 8/28 | 8/29 | 8/30 | 8/31 | 9/1 | 9/2 | 9/3 | 9/4 | 9/5 | 9/6 | 9/7 | 9/8 | 9/9 | 9/10 | 9/11 | 9/12 | 9/13 | 9/14 | 9/15 | 9/16 | 9/17 | 9/18 |
|---|---|---|---|---|---|---|---|---|---|---|---|---|---|---|---|---|---|---|---|---|---|---|---|---|---|---|---|---|---|---|
| Ventricular Tachycardia | | | | | | | | | | | | | | | | | | | | | | | | | | | | | | |
| Atrial Fibrillation | | | • | • | • | | | | | • | • | | • | | | | | | • | | • | • | | | • | • | | | | • |
| SVT | | | | | | | | | | | | | | | | | | | | | • | | | | | | | | | |
| Pause | | | | | | | | | | | | | | | | | | | | | | | | | | | | | | |
| 2nd/3rd Degree AVB | | | | | | | | | | | | • | | | | | | | | | | | | | | | | | | |
| Symptomatic Event | | | | | | | | | | | | • | | | | • | • | | | | | | | | | | | | | |
| PVCs | | | | | | • | • | • | • | • | • | | | | • | • | • | • | • | | | | | | | | | | | |
| PACs, PJCs | • | • | • | • | • | • | • | • | • | • | • | • | • | • | • | • | • | • | • | • | • | • | • | • | • | • | • | • | • | • |

*Each dot in the graph represents at least one episode onset

FIG. 11D

FIG. 11E

EP 4 258 984 B1

**EVENT SUMMARY**

| Day 15 - 1/18/2016 | Event Type | Duration | Symptom | Activity | Technician Notes |
|---|---|---|---|---|---|
| 02:12 | AF | 0 hr, 28 min, 37 sec | None Selected | None Selected | Normal Sinus Rhythm into Atrial Fibrillation |
| 13:02 | AF | 0 hr, 34 min, 58 sec | None Selected | None Selected | |
| 17:07 | AF | 0 hr, 9 min, 57 sec | None Selected | None Selected | Normal Sinus Rhythm into Atrial Fibrillation |
| 19:01 | AF | 1 hr, 21 min, 29 sec | None Selected | None Selected | Normal Sinus Rhythm into Atrial Fibrillation |
| **Day 16 - 1/19/2016** | **Event Type** | **Duration** | **Symptom** | **Activity** | **Technician Notes** |
| 00:39 | AF | 2 hr, 29 min, 12 sec | None Selected | None Selected | Atrial Fibrillation |
| 04:45 | AF | 0 hr, 49 min, 25 sec | None Selected | None Selected | Normal Sinus Rhythm into Atrial Fibrillation |
| 06:18 | AF | 0 hr, 4 min, 44 sec | None Selected | None Selected | Normal Sinus Rhythm into Atrial Fibrillation |
| 10:21 | AF | 2 hr, 51 min, 53 sec | None Selected | None Selected | Normal Sinus Rhythm into Atrial Fibrillation |
| **Day 17 - 1/20/2016** | **Event Type** | **Duration** | **Symptom** | **Activity** | **Technician Notes** |
| 03:25 | AF | 1 hr, 36 min, 27 sec | None Selected | None Selected | Normal Sinus Rhythm into Atrial Fibrillation |
| 04:19 | Tachycardia | 0 hr, 4 min, 14 sec | None Selected | None Selected | Atrial Fibrillation into Ventricular Tachycardia |
| 06:05 | AF | 0 hr, 4 min, 32 sec | None Selected | None Selected | Normal Sinus Rhythm into Atrial Fibrillation |
| 09:01 | AF | 0 hr, 6 min, 41 sec | None Selected | None Selected | Normal Sinus Rhythm into Atrial Fibrillation |
| 09:46 | AF | 0 hr, 0 min, 21 sec | None Selected | None Selected | Normal Sinus Rhythm into Atrial Fibrillation |
| 10:22 | AF | 0 hr, 6 min, 37 sec | None Selected | None Selected | Normal Sinus Rhythm into Atrial Fibrillation |
| 21:20 | AF | 0 hr, 19 min, 38 sec | None Selected | None Selected | Atrial Fibrillation into Normal Sinus Rhythm |

FIG. 11F

FIG. 12A

FIG. 12B

FIG. 12C

FIG. 12D

FIG. 12E

FIG. 13A

FIG. 13B

FIG. 13C

FIG. 14

FIG. 15

FIG. 16A

FIG. 16B

FIG. 16C

FIG. 16D

FIG. 17A

FIG. 17B

FIG. 18A

EP 4 258 984 B1

EP 4 258 984 B1

FIG. 18B

FIG. 19A

EP 4 258 984 B1

FIG. 19B

**REFERENCES CITED IN THE DESCRIPTION**

**Patent documents cited in the description**

- US 2017300653 A1
- US 2011245629 A1
- WO 2019030746 A1
- US 2019069794 A1
- US 04140218
- US 11020002 B
- US 08347220

**Non-patent literature cited in the description**

- **M. J. MATHIE ; B. G. CELLAR ; N. H. LOVELL ; A. C. F. COSTER**. Classification of basic daily movements using a triaxial accelerometer. *Med. Biol. Eng. Comput.*, 2004, vol. 42, 679-687
- **A. BATES ; M. J. LING ; J. MANN ; D. K. ARVIND**. Respiratory rate and flow waveform estimation from tri-axial accelerometer data. *Sensors*, 2016, vol. 16, 750-756